# EUROPEAN PATENT SPECIFICATION

(11) **EP 3 145 546 B1**
(45) Date of publication and mention of the grant of the patent: **09.12.2020**
(21) Application number: 15752115.4
(22) Date of filing: 19.02.2015
(51) Int. Cl.: A61K 39/42, C07K 16/10

(54) **MARBURG MONOCLONAL ANTIBODIES**
MONOKLONALE MARBURG-ANTIKÖRPER
ANTICORPS MONOCLONAUX DE MARBURG

(30) Priority: 19.02.2014 US 201461941807 P; 24.06.2014 US 201462016419 P
(43) Date of publication of application: 29.03.2017
(73) Proprietor: Emergent BioSolutions Canada Inc., Toronto, Ontario M5X 1A4 (CA); The Scripps Research Institute, La Jolla, CA 92037 (US)
(72) Inventor: Berry, Jody, Winnipeg, Manitoba R3T 5Y3 (CA); Saphire, Erica, La Jolla, California 92037 (US)
(74) Representative: MacLean, Martin Robert
(86) International application number: PCT/US2015/016708
(87) International publication number: WO 2015/127140

(56) References cited:
- WO-A1-2006/017173
- WO-A1-2010/078526
- US-A1- 2003 119 018
- US-A1- 2006 270 003
- US-A1- 2007 298 042
- US-A1- 2008 160 035
- US-A1- 2008 166 756
- US-A1- 2009 275 124
- US-A1- 2011 250 203
- US-A1- 2012 034 229
- US-A1- 2013 171 152
- US-A1- 2013 288 927
- HEVEY M ET AL: "Characterization of monoclonal antibodies to Marburg virus (strain Musoke) glycoprotein and identification of two protective epitopes", VIRO, ELSEVIER, AMSTERDAM, NL, vol. 314, no. 1, 15 September 2003 (2003-09-15), pages 350-357, XP004457953, ISSN: 0042-6822, DOI: 10.1016/S0042-6822(03)00416-1
- CALEB D. MARCEAU ET AL: "Novel neutralizing monoclonal antibodies protect rodents against lethal filovirus challenges", TRIALS IN VACCINOLOGY, vol. 3, 16 May 2014 (2014-05-16), pages 89-94, XP055405555, ISSN: 1879-4378, DOI: 10.1016/j.trivac.2014.04.002

## Description

### FIELD OF THE INVENTION

The present disclosure relates to monoclonal antibodies and antigen-binding portions of said antibodies that specifically bind to filoviruses. The disclosure encompasses nucleic acid molecules encoding such antibodies and antigen-binding portions and methods of using and making such antibodies and portions.

### BACKGROUND OF THE INVENTION

Filoviruses are enveloped, negative-strand RNA viruses that can cause lethal hemorrhagic fever in both humans and non-human primates. The filoviridae family consists of the major genera *Ebolavirus* and *Marburgvirus.* There are five known ebolaviruses (Ebola virus (EBOV), Sudan virus (SUDV), Reston virus (RESTV), Bundibugyo virus (BDBV) and Taï Forest virus (TAFV)) and one marburgvirus, the eponymously named Marburg virus (MARV). MARV can be further subdivided into different strains: Ci67, Musoke, Popp, Ravn, which emerged in 1987 and caused one lethal case, and Angola, which emerged in 2005 and caused a large outbreak with 88% lethality (Bukreyev et al., 1995. Arch Virol. 140(9):1589-1600; Johnson et al., 1996. Arch Virol. 11:101-114; International Society for Infectious Diseases. 2005. Marburg hemorrhagic fever - Angola, archive no. 20051108.3269. Brookline, Mass. [Online]). Of these, Ravn is the most divergent in its glycoprotein sequence from the other strains.

Filoviruses can cause severe disease in both humans and non-human primates with mortality rates up to 90%. The filovirus glycoprotein is responsible for attachment and entry of both ebola- and marburgviruses, and is the key target of the antibody response. Relatively few monoclonal antibodies (mAbs) exist for Marburg virus.

The filoviruses express a single glycoprotein protein (GP) on their surface. GP is responsible for attachment and entry of viruses into target cells. GP is expressed as a precursor that is cleaved by furin to yield two subunits: GP1, which contains the putative receptor-binding region as well as a large heavily glycosylated mucin domain, and GP2, which drives membrane fusion (Feldmann et al., 2001. J Gen Virol. 82(Pt2):2839-2848). After cell entry by macropinocytosis, GP is cleaved by host cathepsins.

No curative agent or vaccine is yet approved for use in humans against marburgvirus. As such, there still exists a need to develop monoclonal antibodies that recognize GPs of filoviruses (e.g, different strains of Marburg viruses and members of the *Ebolavirus* genus) and that may be used to prevent or treat filovirus infection.

Hevey M et al (2003), Virology, Volume 314, Issue 1, 15 September 2003, pages 350-357 describes the characterization of monoclonal antibodies to Marburg virus (strain Musoke) glycoprotein and identification of two protective epitopes. Caleb D. Marceau et al (2014), Trials in Vaccinology, Volume 3, 2014, Pages 89-94 describes that novel neutralizing monoclonal antibodies protect rodents against lethal filovirus challenges. WO 2010/078526 A1 describes anti-lymphotoxin antibodies.

### SUMMARY OF THE INVENTION

The invention is defined by the claims. The invention encompasses monoclonal antibodies (and antigen-binding portions
thereof) that bind to a filovirus as claimed. In some embodiments, a monoclonal antibody of the invention, or an antigen-binding portion thereof, binds specifically to a filovirus and comprises (i) an immunoglobulin light chain variable region comprising LCDR1, LCDR2, and LCDR3, and (ii) an immunoglobulin heavy chain variable region comprising HCDR1, HCDR2, and HCDR3, wherein
the LCDR1, LCDR2 and LCDR3 comprise the amino acid sequences set forth in SEQ ID NOs: 78, 79 and 80, respectively, and the HCDR1, HCDR2, and HCDR3 comprise the amino acid sequences set forth in SEQ ID NOs: 90, 91 and 92, respectively;
or an immunoglobulin light chain variable region comprising the amino acid sequence set forth in SEQ ID NO:2 and an immunoglobulin heavy chain variable region comprising the amino acid sequence set forth in SEQ ID NO:14; or an immunoglobulin light chain variable region comprising the amino acid sequence set forth in SEQ ID NO:26 and an immunoglobulin heavy chain variable region comprising the amino acid sequence set forth in SEQ ID NO:38.

In certain disclosures, the immunoglobulin light chain variable region of a monoclonal antibody or antigen-binding portion comprises an amino acid sequence selected from the group consisting of SEQ ID NO:2, SEQ ID NO:26, SEQ ID NO:50, SEQ ID NO:74, SEQ ID NO:98, SEQ ID NO:122, and SEQ ID NO:146. In some disclosures, the immunoglobulin heavy chain variable region of a monoclonal antibody or antigen-binding portion comprises an amino acid sequence selected from the group consisting of SEQ ID NO:14, SEQ ID NO:38, SEQ ID NO:62, SEQ ID NO:86, SEQ ID NO:110, SEQ ID NO:134, and SEQ ID NO:158.

The disclosure encompasses a monoclonal antibody or antigen-binding portion thereof wherein (a) the immunoglobulin light chain variable region comprises the amino acid sequence set forth in SEQ ID NO:2 and the immunoglobulin heavy chain variable region comprises the amino acid sequence set forth in SEQ ID NO:14; (b) the immunoglobulin light chain variable region comprises the amino acid sequence set forth in SEQ ID NO:26 and the immunoglobulin heavy chain variable region comprises the amino acid sequence set forth in SEQ ID NO:38; (c) the immunoglobulin light chain variable region comprises the amino acid sequence set forth in SEQ ID NO:50 and the immunoglobulin heavy chain variable region comprises the amino acid sequence set forth in SEQ ID NO:62; (d) the immunoglobulin light chain variable region comprises the amino acid sequence set forth in SEQ ID NO:74 and the immunoglobulin heavy chain variable region comprises the amino acid sequence set forth in SEQ ID NO:86; (e) the immunoglobulin light chain variable region comprises the amino acid sequence set forth in SEQ ID NO:98 and the immunoglobulin heavy chain variable region comprises the amino acid sequence set forth in SEQ ID NO:110; (f) the immunoglobulin light chain variable region comprises the amino acid sequence set forth in SEQ ID NO:122 and the immunoglobulin heavy chain variable region comprises the amino acid sequence set forth in SEQ ID NO:134; or (g) the immunoglobulin light chain variable region comprises the amino acid sequence set forth in SEQ ID NO:146 and the immunoglobulin heavy chain variable region comprises the amino acid sequence set forth in SEQ ID NO:158.

In some embodiments, a monoclonal antibody or antigen-binding portion of the invention may be (i) a whole immunoglobulin molecule; (b) an scFv; (c) a Fab fragment; (d) an F(ab')₂; and (e) a disulfide linked Fv. An antibody may comprise an immunoglobulin constant region selected from the group consisting of IgG1, IgG2, IgG3, IgG4, IgA1, IgA2, IgD, IgE and IgM.

The invention relates to a monoclonal antibody or antigen-binding portion as claimed that binds a filovirus that is a member of the *Marburgvirus* genus. A filovirus may be any strain of Marburg virus (e.g., Ravn, Angola, Musoke, or Popp Marburg virus strain). In some embodiments, a monoclonal antibody or antigen-binding portion binds to the Marburg virus glycoprotein. In certain embodiments, a monoclonal antibody or antigen-binding portion binds to the GP1 or the GP2 subunit of the Marburg virus glycoprotein.

A monoclonal antibody or antigen-binding portion may be cross-reactive to at least two filoviruses. At least one of these filoviruses may be a member of the *Ebolavirus* genus (e.g., Ebola virus, Sudan virus, Bundibugyo virus, Tai Forest virus or Reston virus). A monoclonal antibody or antigen-binding portion may bind to the Ebola virus glycoprotein.

In one aspect, the invention encompasses a pharmaceutical composition comprising at least one of the monoclonal antibodies or antigen-binding portions as claimed and at least one pharmaceutically acceptable adjuvant. In another aspect, the invention encompasses a pharmaceutical composition comprising at least one of the monoclonal antibodies or antigen-binding portions as claimed and at least one pharmaceutically acceptable carrier. A pharmaceutical composition may comprise a second agent (e.g., a monoclonal antibody or antigen-binding portion thereof). In some embodiments, a pharmaceutical composition comprises at least one Marburg virus-binding antibody or antigen-binding portion thereof, and at least one Ebola virus-binding antibody or antigen-binding portion thereof. A pharmaceutical composition may comprise at least two, at least three, or at least four Marburg virus-binding antibodies or antigen-binding portion thereof. A pharmaceutical composition may comprise at least two Marburg virus-binding antibodies or antigen-binding portion thereof, wherein each Marburg virus-binding antibody or antigen-binding portion bind different epitopes (e.g., overlapping or non-overlapping epitopes) of the Marburg virus glycoprotein.

The invention also encompasses a monoclonal antibody or antigen binding portion thereof as claimed for use in a method of ameliorating, preventing, or treating a filovirus (e.g., Marburg virus or Ebola virus) infection comprising administering to a subject in need thereof a therapeutically effective amount of any of the monoclonal antibodies or antigen-binding portions described herein. In some embodiments, the invention relates to a method of ameliorating, preventing, or treating a filovirus infection (e.g., Marburg virus or Ebola virus) comprising administering to a subject In need thereof a therapeutically effective amount of the monoclonal antibodies or antigen-binding portions. An antibody or antigen-binding portion disclosed herein for use in a method may comprise (i) an immunoglobulin light chain variable region comprising LCDR1, LCDR2, and LCDR3, and (ii) an immunoglobulin heavy chain variable region comprising HCDR1, HCDR2, and HCDR3, wherein (a) the LCDR1, LCDR2 and LCDR3 comprise the amino acid sequences set forth in SEQ ID NOs: 6, 7 and 8, respectively, and the HCDR1, HCDR2, and HCDR3 comprise the amino acid sequences set forth in SEQ 10 NOs: 18, 19 and 20, respectively; (b) the LCDR1, LCDR2 and LCDR3 comprise the amino acid sequences set forth in SEQ ID NOs: 30, 31 and 32, respectively, and the HCDR1, HCDR2, and HCDR3 comprise the amino acid sequences set forth in SEQ ID NOs: 42, 43 and 44, respectively; or (c) the LCDR1, LCDR2 and LCDR3 comprise the amino acid sequences set forth in SEQ ID NOs: 54, 55 and 56, respectively, and the HCDR1, HCDR2, and HCDR3 comprise the amino acid sequences set forth in SEQ ID NOs: 66, 67 and 68, respectively, (d) the LCDR1, LCDR2 and LCDR3 comprise the amino acid sequences set forth in SEQ ID NOs: 78, 79 and 80, respectively, and the HCDR1, HCDR2, and HCDR3 comprise the amino acid sequences set forth in SEQ ID NOs: 90, 91 and 92, respectively, (e) the LCDR1, LCDR2 and LCDR3 comprise the amino acid sequences set forth in SEQ ID NOs: 102, 103 and 104, respectively, and the HCDR1, HCDR2, and HCDR3 comprise the amino acid sequences set forth in SEQ ID NOs: 114, 115 and 116, respectively, (f) the LCDR1, LCDR2 and LCDR3 comprise the amino acid sequences set forth in SEQ ID NOs: 126, 127 and 128, respectively, and the HCDR1, HCDR2, and HCDR3 comprise the amino acid sequences set forth in SEQ ID NOs: 138, 139 and 140, respectively, or (g) the LCDR1, LCDR2 and LCDR3 comprise the amino acid sequences set forth in SEQ ID NOs: 150, 151 and 152, respectively, and the HCDR1, HCDR2, and HCDR3 comprise the amino acid sequences set forth in SEQ ID NOs: 162, 163 and 164, respectively. The invention encompasses a monoclonal antibody or antigen-binding portion thereof as claimed for use in ameliorating, preventing or treating a filovirus (e.g., Marburg virus or Ebola virus) infection in a subject in need thereof. The invention further encompasses such monoclonal antibodies or antigen-binding portions thereof for use in ameliorating, preventing or treating a filovirus (e.g., Marburg virus or Ebola virus) infection in a subject in need thereof.

In one aspect, the invention encompasses an isolated nucleic acid molecule encoding any of the anti-filovirus monoclonal antibodies or antigen-binding portions as claimed. An isolated nucleic acid molecule may encode the immunoglobulin light chain and heavy chain variable regions of any of the monoclonal antibodies or antigen-binding portions as claimed. In some disclosures, a nucleic acid molecule comprises one or more nucleotide sequence selected from the group consisting of SEQ ID NO:1, SEQ ID NO:3, SEQ ID NO:4, SEQ ID NO:5, SEQ ID NO:13, SEQ ID NO:15, SEQ ID NO:16, SEQ ID NO:17, SEQ ID NO:25, SEQ ID NO:27, SEQ ID NO:28, SEQ ID NO:29, SEQ ID NO:37, SEQ ID NO:39, SEQ ID NO:40, SEQ ID NO:41, SEQ ID NO:49, SEQ ID NO:51, SEQ ID NO:52, SEQ ID NO:53, SEQ ID NO:61, SEQ ID NO:63, SEQ ID NO:64, SEQ ID NO:65, SEQ ID NO:73, SEQ ID NO:75, SEQ ID NO:76, SEQ ID NO:77, SEQ ID NO:85, SEQ ID NO:87, SEQ ID NO:88, SEQ ID NO:89, SEQ ID NO:97, SEQ ID NO:99, SEQ ID NO:100, SEQ ID NO:101, SEQ ID NO:109, SEQ ID NO:111, SEQ ID NO:112, SEQ ID NO:113, SEQ ID NO:121, SEQ ID NO:123, SEQ ID NO:124, SEQ ID NO:125, SEQ ID NO:133, SEQ ID NO:135, SEQ ID NO:136, SEQ ID NO:137, SEQ ID NO:145, SEQ ID NO:147, SEQ ID NO:148, SEQ ID NO:149, SEQ ID NO:157, SEQ ID NO:159, SEQ ID NO:160, and SEQ ID NO:161.

The invention also relates to an expression vector comprising a nucleic acid segment encoding the immunoglobulin light and heavy chain variable regions of any of the anti-filovirus monoclonal antibodies or antigen-binding portions as claimed, wherein the nucleic acid segment is operatively linked to regulatory sequences suitable for expression of the nucleic acid segment in a host cell. In some disclosures, a nucleic acid segment in an expression vector may comprise one or more nucleotide sequence selected from the group consisting of SEQ ID NO:1, SEQ ID NO:3, SEQ ID NO:4, SEQ ID NO:5, SEQ ID NO:13, SEQ ID NO:15, SEQ ID NO:16, SEQ ID NO:17, SEQ ID NO:25, SEQ ID NO:27, SEQ ID NO:28, SEQ ID NO:29, SEQ ID NO:37, SEQ ID NO:39, SEQ ID NO:40, SEQ ID NO:41, SEQ ID NO:49, SEQ ID NO:51, SEQ ID NO:52, SEQ ID NO:53, SEQ ID NO:61, SEQ ID NO:63, SEQ ID NO:64, SEQ ID NO:65, SEQ ID NO:73, SEQ ID NO:75, SEQ ID NO:76, SEQ ID NO:77, SEQ ID NO:85, SEQ ID NO:87, SEQ ID NO:88, SEQ ID NO:89, SEQ ID NO:97, SEQ ID NO:99, SEQ ID NO:100, SEQ ID NO:101, SEQ ID NO:109, SEQ ID NO:111, SEQ ID NO:112, SEQ ID NO:113, SEQ ID NO:121, SEQ ID NO:123, SEQ ID NO:124, SEQ ID NO:125, SEQ ID NO:133, SEQ ID NO:135, SEQ ID NO:136, SEQ ID NO:137, SEQ ID NO:145, SEQ ID NO:147, SEQ ID NO:148, SEQ ID NO:149, SEQ ID NO:157, SEQ ID NO:159, SEQ ID NO:160, and SEQ ID NO:161. In a further aspect, the invention relates to a recombinant host cell comprising an expression vector as claimed. A recombinant host cell may be a bacterial, eukaryotic or mammalian cell (e.g., a COS-1, COS-7, HEK293, BHK21, CHO, BSC-1, HepG2, SP2/0, HeLa, myeloma or lymphoma cell). The invention also relates to a method for producing a filovirus-binding monoclonal antibody or antigen-binding portion thereof, the method comprising culturing a recombinant host cell comprising an expression vector as claimed under conditions whereby the nucleic acid segment is expressed, thereby producing the filovirus-binding monoclonal antibody or antigen-binding portion, and, optionally, recovering the filovirus-binding monoclonal antibody or antigen-binding portion.

The invention encompasses a method for detecting a filovirus glycoprotein (e.g., a Marburg virus, Ebola virus, Sudan virus, Bundibugyo virus, Tai Forest virus or Reston virus glycoprotein) in a sample, the method comprising contacting the sample with any of the monoclonal antibodies or antigen-binding portions as claimed. A sample may be a cell, tissue, or biological fluid from a subject suspected of having or at risk of a filovirus infection.

These and other embodiments and/or other aspects of the invention will become evident upon reference to the following detailed description of the invention and the attached drawings.

### BRIEF DESCRIPTION OF THE DRAWINGS

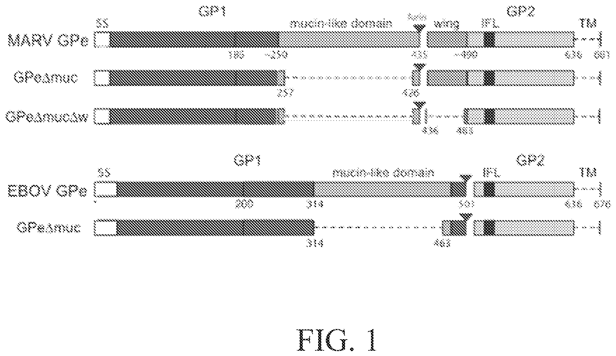

**Figure 1** is a schematic showing the domains of the *marburgvirus* and *ebolavirus* glycoprotein (GP) and construct design for the antigens described in Example 1. Marburg (MARV) GP and mucin-deleted constructs are shown at the top; Ebola (EBOV) GP and mucin-deleted constructs are shown at the bottom. Dashed lines represent deleted regions. SS, signal sequence; IFL, internal fusion loop; TM, transmembrane. GP ectodomain constructs (GPe) lack the transmembrane (TM) domain and consist of residues 1-637. GP ectodomain mucin-deleted constructs (GPeΔmuc) also lack the mucin-like domain: Δ257-425 for all MARV strains, Δ314-463 for EBOV (Ebola virus), SUDV (Sudan virus), BDBV (Bundibugyo virus) and Δ316-470 for RESTV (Reston virus). As a control for epitope-mapping experiments, an additional MARV GP construct was purified from S2 cells lacking both the GP1 mucin domain (Δ257-425) and the GP2-wing (Δ436-483), termed GPeΔmucΔw.

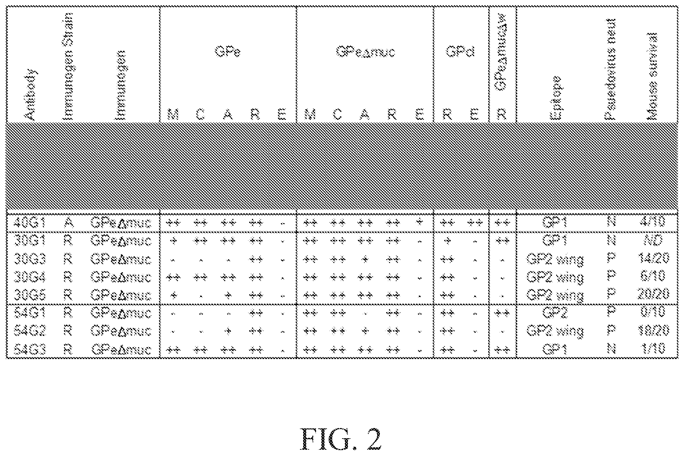

**Figure 2** is a table showing the results of characterization studies of the CAN30, CAN54 and CAN40 series mAbs. In some of the studies, these antibodies were tested for binding to different strains of MARV and EBOV engineered GPs (Glycoprotein ectodomain, GPe; glycoprotein ectodomain and mucin domain deleted, GPeΔmuc; glycoprotein enzymatically cleaved to resemble the GP core after endosomal cleavage, GPcl). Filovirus species are listed as: M, Musoke; C, Ci67; A, Angola R, Ravn; E, EBOV. Positive binding with the ELISA assay is represented by (+) when mAb concentrations at 10 µg/ml demonstrated OD450 >1.0 units above background, and (++) represents stronger binding if values were achieved at 0.5 µg/ml or below. Antibodies considered to have neutralized pseudovirus by a reduction of infectivity <60% of control are marked with a P for partial.

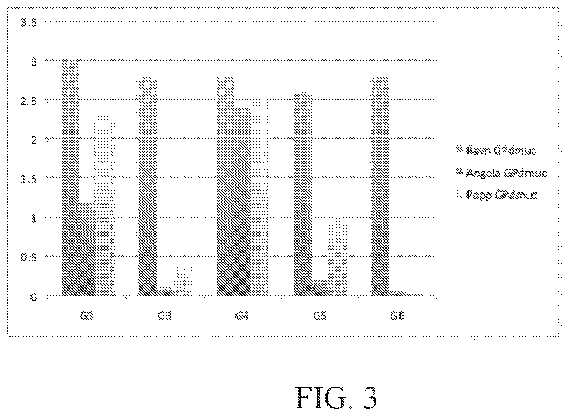

**Figure 3** is a bar graph showing the results of ELISA experiments measuring binding of mAbs CAN30G1 (G1), CAN30G3 (G3), CAN30G4 (G4), CAN30G5 (G5) and CAN30G6 (G6) to GPΔmucΔtm (GPdmuc) of MARV-Ravn (left bar in each set), MARV-Angola (center bar in each set), and MARV-Popp (right bar in each set).

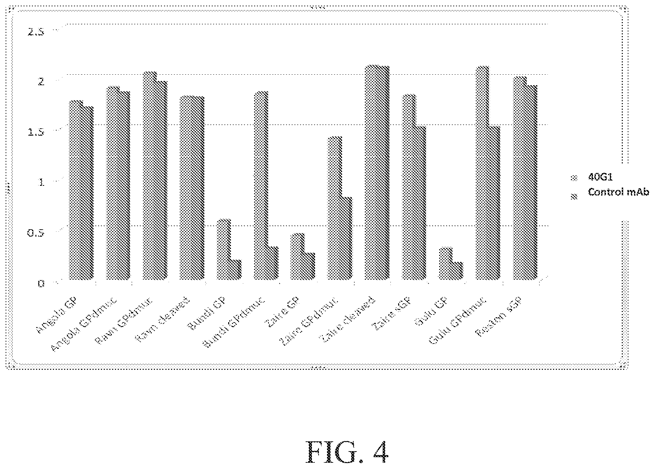

**Figure 4** is a bar graph showing the results of ELISA experiments measuring binding of mAb CAN40G1 (40G1) to various MARV strains. The control mAb was anti-MARV Musoke.

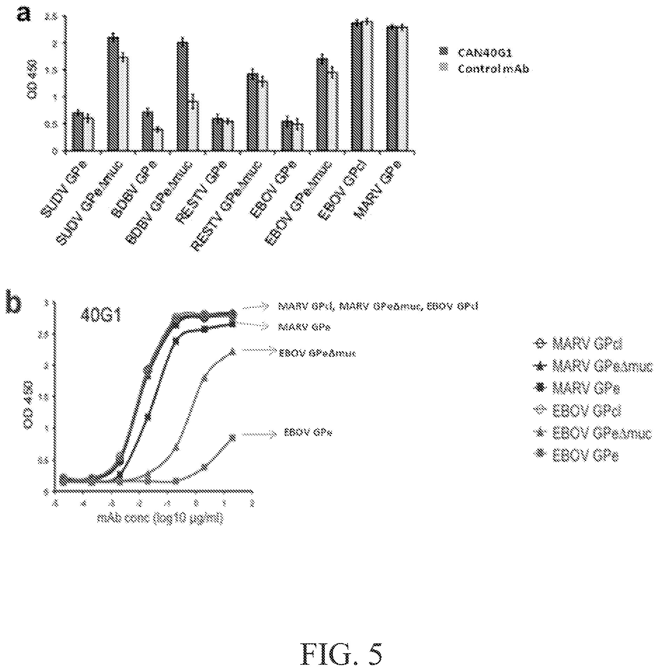

**Figure 5A** is a bar graph showing the results of ELISA experiments measuring binding of mAb CAN40G1 (40G1) to various MARV strain and ebolavirus antigens. ELISA plates were coated with each of the ten antigens shown on the x-axis, and immunoreactivity against CAN40G1 or positive control anti-MARV musoke mAb (control mAb) bound at 5 µg/ml. Experiments were performed in triplicate, and standard deviations are displayed..
**Figure 5B** shows ELISA binding curves determined by binding CAN40G1 (40G1) or positive control anti-MARV musoke mAb (control mAb) to the indicated antigens at a starting concentration of 25 µg/ml, then diluting down by ten to a concentration of 2.5x10⁻⁵ µg/ml. Note that antibody binding affinity for MARV GPeΔmuc, MARV GPcl, and EBOV GPcl is similar causing the curves to overlay.

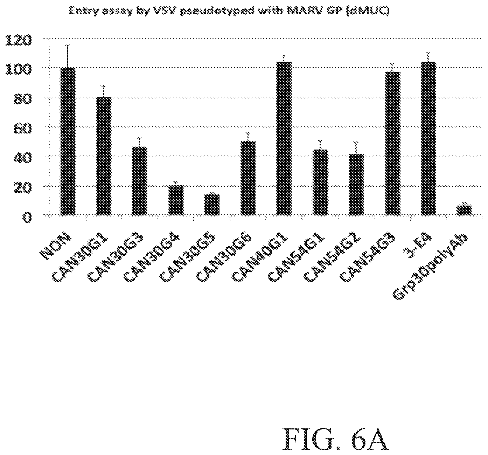

**Figure 6A** is a bar graph showing the results of pseudovirus neutralization assays with mucin-deleted MARV GP after treatment with anti-MARV mAbs (as indicated on the x-axis). Vero cell infectivity of mucin-deleted MARV GP-pseudotyped VSV at an MOI of 0.1 is shown after treatment with 50 µg/ml mAb. Percent infectivity is shown on the y-axis. Grp30polyAb is pooled polyclonal sera from immunized mice. NON is negative control; no antibody added.

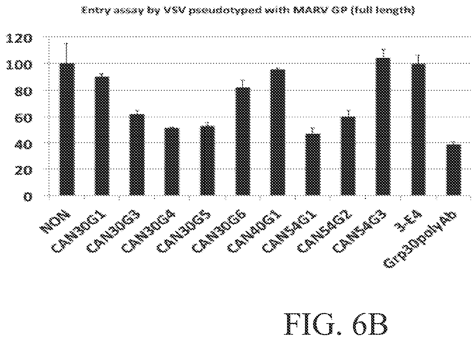

**Figure 6B** is a bar graph showing the results of pseudovirus neutralization assays with full-length (mucin-containing) MARV GP after treatment with anti-MARV mAbs (as indicated on the x-axis). Vero cell infectivity of mucin-containing MARV GP-pseudotyped VSV at an MOI of 1.0 is shown after treatment with 50 µg/ml mAb. Percent infectivity is shown on the y-axis. Grp30polyAb is pooled polyclonal sera from immunized mice. NON is negative control; no antibody added.

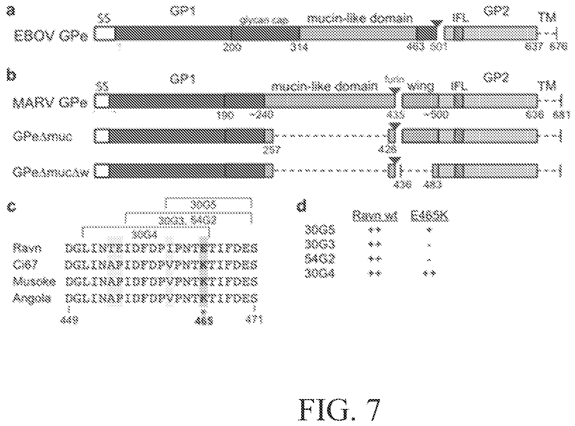

**Figure 7** shows filovirus GP schematics and sequence alignment of mAb epitopes. SS, signal sequence; IFL, internal fusion loop; TM, transmembrane. The furin cleavage site is indicated with an arrowhead where indicated. **Figure 7A** shows the Ebola virus GP schematic and construct design. Dashed lines represent deleted regions. GPeΔmuc constructs remove 314-463 from EBOV, BDBV, SUDV and 316-470 from RESTV. **Figure 7B** shows the Marburg virus GP schematic. **Figure 7C** shows the pepscan defined epitopes for anti-GP2 wing mAbs. This region has four residues unique to strain Ravn. The epitope sequences in this figure are assigned the following SEQ ID NOs: Ravn (SEQ ID NO:198); Ci67 (SEQ ID NO:199); Musoke (SEQ ID NO:200); Angola (SEQ ID NO:201). **Figure 7D** shows the results of an experiment where GP2 wing mAb reactivity to Ravn GPeΔmuc wt or E465K was evaluated by ELISA. Positive binding with the ELISA assay is represented by (+) when mAb concentrations at 10 µg/ml demonstrated OD450>1.0 units above background, and (++) represents stronger binding if values were achieved at 0.5 µg/ml or below.

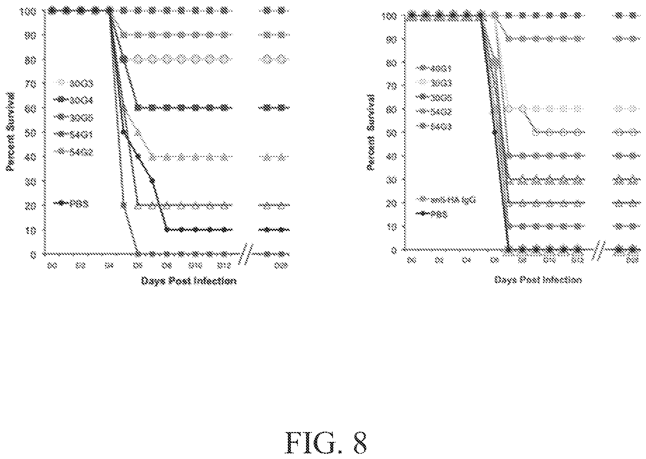

**Figure 8** is a Kaplan Meier plot showing the results of assays examining *in vivo* protection using mice challenged with a lethal dose of mouse-adapted MARV Ravn. Mice were treated one hour post-exposure with anti-GP antibody; 30G3 (CAN30G3), 30G4 (CAN30G4), 30G5 (CAN30G5), 54G1 (CAN54G1), 54G2 (CAN54G2), 40G1 (CAN40G1), 54G3 (CAN54G3) or PBS alone. Percent survival is shown on the y-axis.

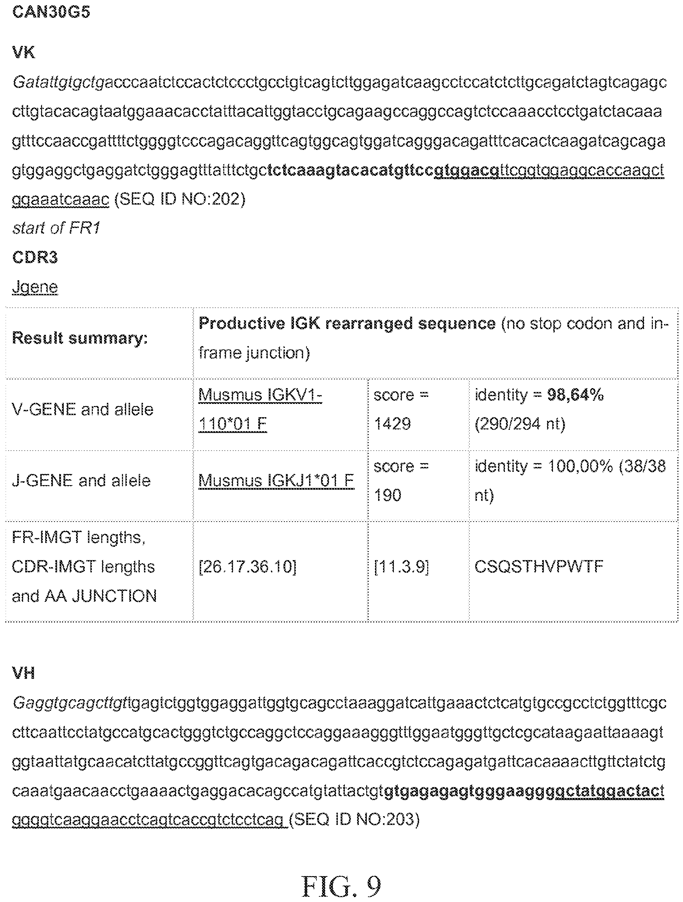

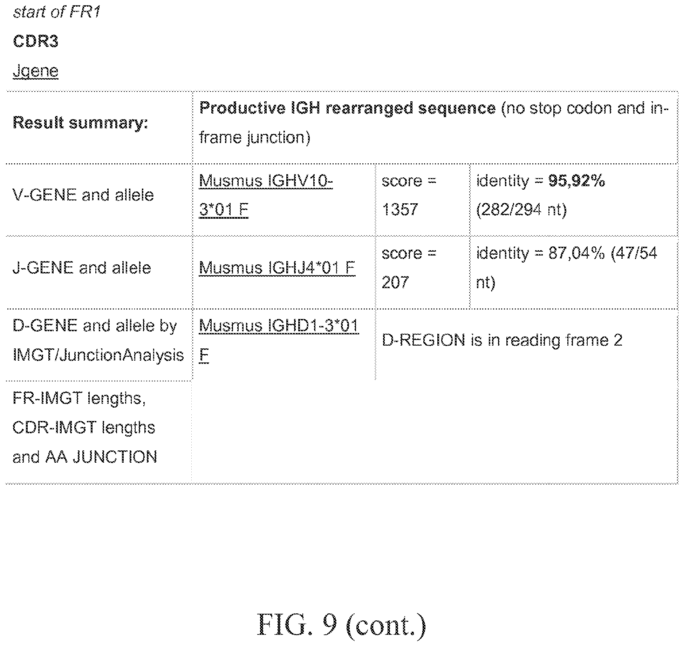

**Figure 9** shows variable (V) gene sequencing results for murine CAN30G5 that include VH and VL sequences from the murine CAN30G5 parental clone.

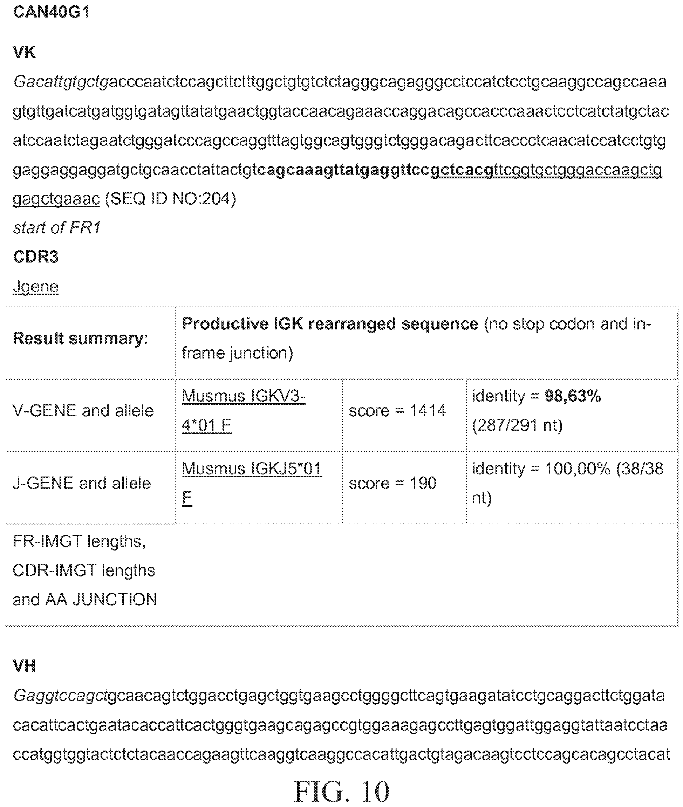

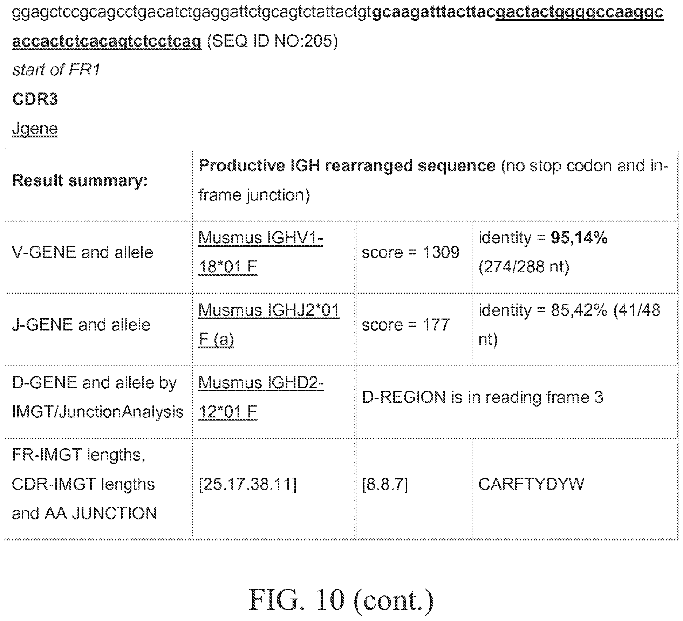

**Figure 10** shows variable (V) gene sequencing results for murine CAN40G1 that includes VH and VL sequences from the murine CAN40G1 parental clone.

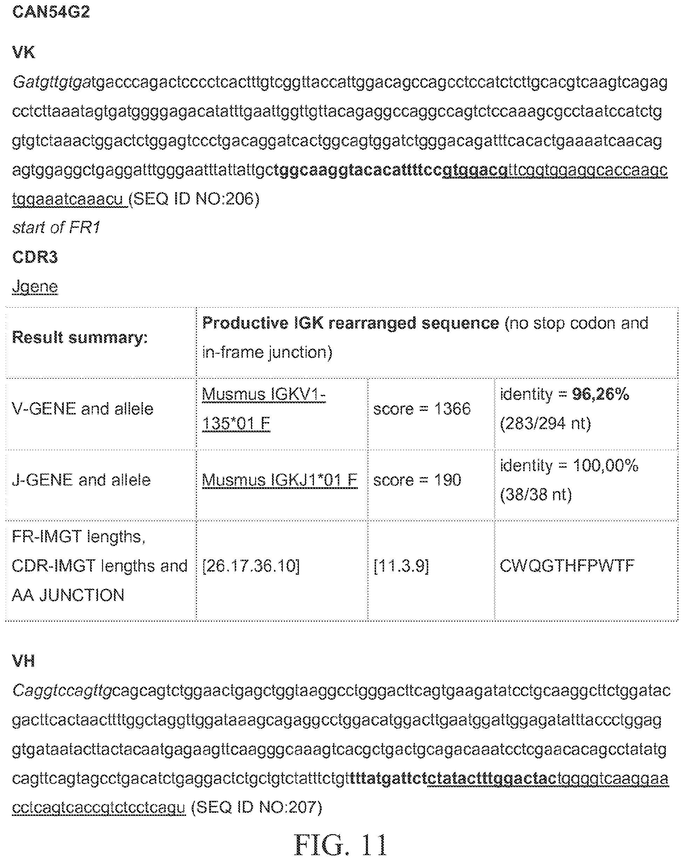

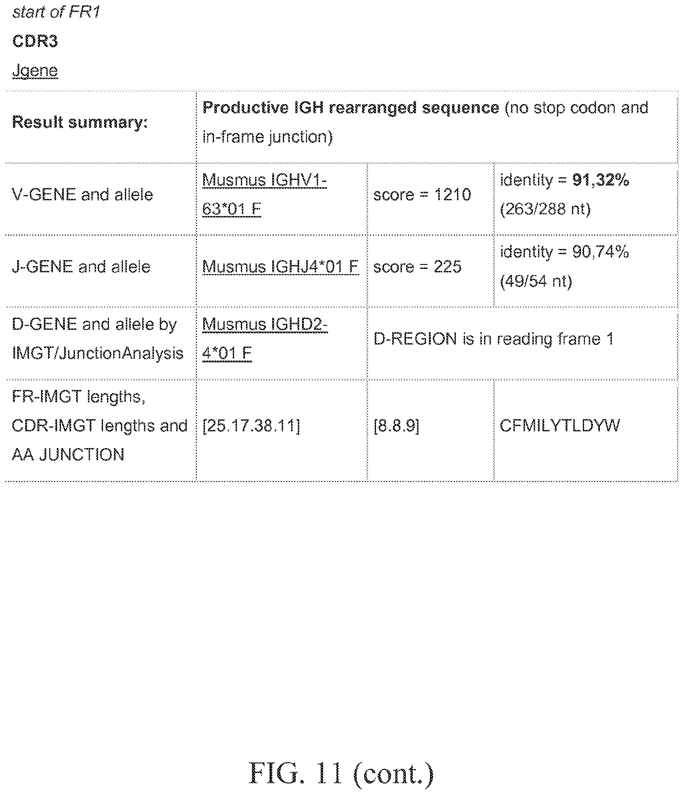

**Figure 11** shows variable (V) gene sequencing results for murine CAN54G2 that includes VH and VL sequences from the murine CAN54G2 parental clone.

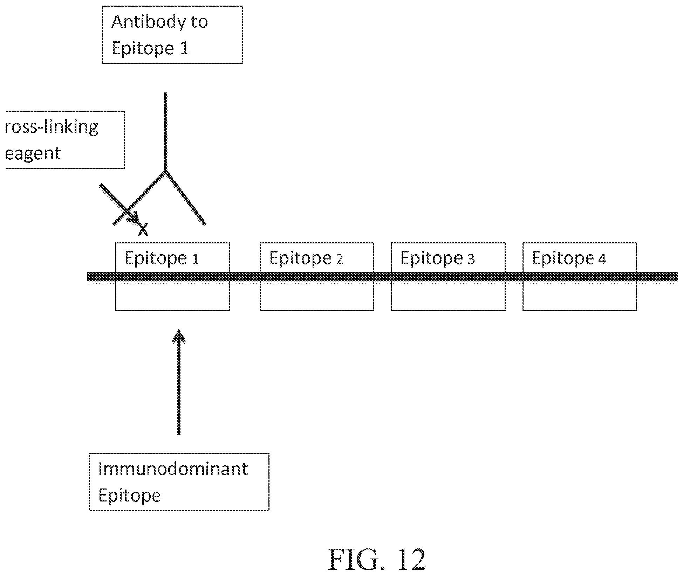

**Figure 12** is a diagram of an antigen-antibody complex that may be used for immunization to mask an immunodominant epitope (see, e.g., Example 2).

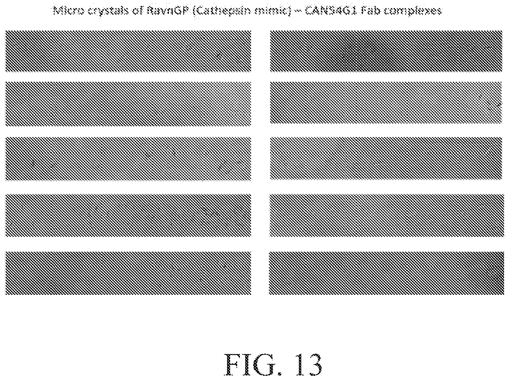

**Figure 13** shows microcrystals of a complex between Ravn Marburg virus GP and CAN54G1 Fab obtained by microfluidic free interface diffusion.

### DETAILED DESCRIPTION OF THE INVENTION

The invention relates to monoclonal antibodies and antigen binding portions thereof as claimed that specifically bind to at least one filovirus (e.g. at least one filovirus glycoprotein (GP)). The invention also provides pharmaceutical compositions and antibodies and antigen binding portions thereof for use in methods for the prevention or treatment of a filovirus infection as claimed. The filovirus family (*Filoviridae*) includes two accepted genera, *Ebolavirus* and *Marburgvirus.* The *Ebolavirus* genus includes EBOV (Ebola virus), SUDV (Sudan virus), BDBV (Bundibugyo virus), TAFV (Tai Forest virus) and RESTV (Reston virus). The *Marburgvirus* genus includes MARV (Marburg virus). All strains of MARV are contemplated for use in this invention (e.g., Ravn, Angola, Musoke, Popp, and Ci67). Examples of GP amino acid sequences are provided in Table 1 (SEQ ID NOs:173-183).

In the present description, any concentration range, percentage range, ratio range, or integer range is to be understood to include the value of any integer within the recited range and, when appropriate, fractions thereof (such as one tenth and one hundredth of an integer), unless otherwise indicated. As used herein, "about" means ± 20% of the indicated range, value, or structure, unless otherwise indicated or apparent from context. It should be understood that the terms "a" and "an" as used herein refer to "one or more" of the enumerated components unless otherwise indicated. The use of the alternative (e.g., "or") should be understood to mean either one, both, or any combination thereof of the alternatives. As used herein, the terms "include" and "comprise" are used synonymously. In addition, it should be understood that the polypeptides comprising the various combinations of the components (e.g., domains or regions) and substituents described herein, are disclosed by the present application to the same extent as if each polypeptide was set forth individually. Thus, selection of particular components of individual polypeptides is within the scope of the present disclosure.

In one aspect, the invention relates to a monoclonal antibody or an antigen-binding portion thereof that specifically binds to a filovirus as claimed. A binding domain or protein (e.g., antibody or antigen-binding portion) "specifically binds" a target if it binds the target with an affinity or Kₐ (*i.e.,* an equilibrium association constant of a particular binding interaction with units of 1/M) equal to or greater than 10⁵ M⁻¹, while not significantly binding other components present in a test sample. Binding domains can be classified as "high affinity" binding domains and "low affinity" binding domains. "High affinity" binding domains refer to those binding domains with a Kₐ of at least about 10⁷ M⁻¹, at least about 10⁸ M⁻¹, at least about 10⁹ M⁻¹, at least about 10¹⁰ M⁻¹, at least about 10¹¹ M⁻¹, at least about 10¹² M⁻¹, or at least about 10¹³ M⁻¹. "Low affinity" binding domains refer to those binding domains with a Kₐ of up to about 10⁷ M⁻¹, up to about 10⁶ M⁻¹, up to about 10⁵ M⁻¹. Alternatively, affinity can be defined as an equilibrium dissociation constant (K_{d}) of a particular binding interaction with units of M (*e.g.,* about 10⁻⁵ M to about 10⁻¹³ M). Affinities of binding domain polypeptides and single chain polypeptides according to the present disclosure can be readily determined using conventional techniques (*see, e.g.,* Scatchard et al. (1949) Ann. N.Y. Acad. Sci. 51:660; and U.S. Patent Nos. 5,283,173, 5,468,614, or the equivalent),

In some embodiments, an antibody or antigen-binding portion specifically binds at least one epitope on the GP of Marburg virus (MARV). An antibody or antigen-binding portion may also bind at least one epitope on the GP of a member of the *Ebolavirus* genus (e.g., Ebola virus (EBOV)). Immunogenic screening constructs were produced for multiple strains of MARV GP and EBOV GP: GPe, GPeΔmuc, GPeΔmucΔw, GPcl. Table 2 and Figure 1 illustrate the nature of the constructs; (i) the MARV GP and EBOV GP ectodomain constructs (GPe) lack the transmembrane (TM) domain and consist of residues 1-637; (ii) the MARV GP ectodomain mucin-deleted constructs (GPeΔmuc) also lack the mucine-like domain: Δ257-425 for all MARV strains; (iii) the EBOV GP ectodomain mucin-deleted constructs (GPeΔmuc) also lack the mucine-like domain: Δ314-463 for EBOV, SUDV, BDBV and Δ316-470 for RESTV, the MARV GP construct GPeΔmucΔw lacks both the GP1 mucin domain (Δ257-425) and the GP2-wing (Δ436-483); or, (iv) the cleaved MARV GP (GPcl) construct was enzymatically treated to mimic endosomal protease cleavage. The epitope may correspond to the GP1 or GP2 region of the MARV GP. An epitope bound by an antibody or antigen-binding portion of the invention may be linear or conformational. The antibodies may bind to more than one strain of Marburg virus and may also bind to more than one type of filovirus. The present compositions may be used for passive or active immunotherapy of a filovirus infection such as Marburg virus or used as prophylactic vaccines in populations at risk of infection from Marburg virus or other types of filoviruses.

The present antibodies, or antigen-binding portions, include, but are not limited to, monoclonal antibodies, chimeric antibodies, humanized antibodies, polyclonal antibodies, recombinant antibodies, as well as antigen-binding portions of the foregoing, e.g., Fab, F(ab')2, Fab', F(ab)', Fv, and single chain Fv (scFv). An antigen-binding portion of an antibody may include a portion of an antibody that specifically binds to an epitope on the GP of MARV as described herein. As used herein, "antibodies" includes fusion proteins comprising an antigen-binding portion.

The antibodies of the present disclosure include murine antibodies produced by hybridoma series CAN30, CAN40 and CAN54 (see Table 1). Also encompassed by the present disclosure are antibodies or antigen-binding portions that include an antigen-binding portion of an antibody produced by hybridoma series, CAN30, CAN40 and CAN54. CAN30G1, CAN30G2, CAN30G3, CAN30G4, CAN30G5, CAN54G1, CAN54G2, CAN54G3 or CAN40G1 refer to the hybridoma clone or monoclonal antibodies generated by the corresponding hybridoma clone. The amino acid and nucleotide sequences of various domains of these antibodies are provided in Table 1. For example, 30G5, 54G2 and 40G1 refer to hybridoma clones or monoclonal antibodies produced by CAN30G5, CAN54G2 and CAN40G1, respectively.

The CDRs of the present antibodies or antigen-binding portions thereof can be from a non-human or human source. The framework of the present antibodies or antigen-binding portions thereof can be human, humanized, non-human (e.g., a murine framework modified to decrease antigenicity in humans), or a synthetic framework (e.g., a consensus sequence).

In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. These particular regions have been described, for example, by Kabat et al., U.S. Dept. of Health and Human Services, "Sequences of Proteins of Immunological Interest" (1983) and by Chothia et al., J. Mol. Biol. 196:901-917 (1987).

The Kabat and Chothia definitions include overlapping or subsets of amino acids when compared against each other. Nevertheless, application of either definition (or other definitions known to those of ordinary skill in the art) to refer to a CDR of an antibody or variant thereof is intended to be within the scope of the term as defined and used herein, unless otherwise indicated. The appropriate amino acids which encompass the CDRs as defined by each of the above cited references are set forth in the table below as a comparison. The exact amino acid numbers which encompass a particular CDR will vary depending on the sequence and size of the CDR. Those skilled in the art can routinely determine which amino acids comprise a particular CDR given the variable region amino acid sequence of the antibody.

**CDR Definitions"**

| | **Kabat** | **Chothia** |
|---|---|---|
| **VH CDR1** | 31-35 | 26-32 |
| **VH CDR2** | 50-65 | 52-58 |
| **VH CDR2** | 95-102 | 95-102 |
| **VL CDR1** | 24-34 | 26-32 |
| **VL CDR2** | 50-56 | 50-52 |
| **VL CDR3** | 89-97 | 91-96 |

| | | |
|---|---|---|
| *Numbering of all CDR definitions In the table above is according to the numbering conventions set forth by Kabat et al. (see below), | | |

CDRs can also be determined using IMGT® (the international ImMunoGeneTics information system®) numbering. H: heavy chain; K: kappa or L: light chain. Kabat et al. also defined a numbering system for variable domain sequences that is applicable to any antibody. One of ordinary skill in the art can unambiguously assign this system of "Kabat numbering" to any variable domain sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless use of the Kabat numbering system is explicitly noted, however, consecutive numbering is used for all amino acid sequences in this disclosure.

In one embodiment, the present antibodies, or antigen-binding portions thereof, contain at least one heavy chain variable region and at least one light chain variable region as claimed. The heavy chain variable region (or light chain variable region) contains three CDRs and four framework regions (FRs), arranged from amino-terminus to carboxyl-terminus in the following order: FR1, CDR1, FR2, CDR2, FR3, CDR3, FR4. Kabat, E. A., et al. Sequences of Proteins of immunological Interest, Fifth Edition, U.S. Department of Health and Human Services, NIH Publication No. 91-3242, 1991. Chothia, C. et al, J. Mol. Biol. 196:901-917, 1987.

The present antibodies or antigen-binding portions can specifically bind to a conformational or non-conformational epitope (or both) of MARV glycoprotein with a dissociation constant (K_{D}) of less than about 10⁻⁷ M, less than about 10⁻⁸ M, less than about 10⁻⁹ M, less than about 10⁻¹⁰ M, less than about 10⁻¹¹ M, or less than about 10⁻¹² M.

The present antibodies or antigen-binding portions can bind specifically to a conformational or non-conformational epitope (or both) of the EBOV glycoprotein with a dissociation constant (K_{D}) of less than about 10⁻⁷ M, less than about 10⁻⁸ M, less than about 10⁻⁹ M, less than about 10⁻¹⁰ M, less than about 10⁻¹¹ M, or less than about 10⁻¹² M.

As used herein, the term "sequence identity" refers to a relationship between two or more polynucleotide sequences or between two or more polypeptide sequences. When a position in one sequence is occupied by the same nucleic acid base or amino acid residue in the corresponding position of the comparator sequence, the sequences are said to be "identical" at that position. The percentage "sequence identity" is calculated by determining the number of positions at which the identical nucleic acid base or amino acid residue occurs in both sequences to yield the number of "identical" positions. The number of "Identical" positions is then divided by the total number of positions In the comparison window and multiplied by 100 to yield the percentage of "sequence identity." Percentage of "sequence identity" is determined by comparing two optimally aligned sequences over a comparison window. The comparison window for nucleic acid sequences can be, for instance, at least about 20, 30, 40, 50, 60, 70, 80, 90, 100, 110, 120, 130, 140, 150, 160, 170, 180, 190, 200, 300,400, 500, 600, 700, 800, 900 or 1000 or more nucleic acids in length. The comparison window for polypeptide sequences can be, for instance, at least about 20, 30, 40, 50, 60, 70, 80, 90, 100, 110, 120, 130, 140, 150, 160, 170, 180, 190, 200, 300 or more amino acids in length. In order to optimally align sequences for comparison, the portion of a polynucleotide or polypeptide sequence in the comparison window can comprise additions or deletions termed gaps while the reference sequence is kept constant. An optimal alignment is that alignment which, even with gaps, produces the greatest possible number of "identical" positions between the reference and comparator sequences. Percentage "sequence identity" between two sequences can be determined using the version of the program "BLAST 2 Sequences" which was available from the National Center for Biotechnology Information as of September 1, 2004, which program incorporates the programs BLASTN (for nucleotide sequence comparison) and BLASTP (for polypeptide sequence comparison), which programs are based on the algorithm of Karlin and Altschul (Proc. Natl. Acad. Sci. USA 90(12):5873-5877, 1993). When utilizing "BLAST 2 Sequences," parameters that were default parameters as of September 1, 2004, can be used for word size (3), open gap penalty (11), extension gap penalty (1), gap dropoff (50), expect value (10) and any other required parameter including but not limited to matrix option. Two nucleotide or amino acid sequences are considered to have "substantially similar sequence identity" or "substantial sequence identity" if the two sequences have at least about 80%, at least about 85%, at least about 90%, at least about 95%, at least about 96%, at least about 97%, at least about 98%, or at least about 99% sequence identity relative to each other.

The disclosure includes antibodies and antibody fragments comprising a variable heavy chain region (HCVR or V_{H}) and a variable light chain region (LCVR or V_{L}) at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70% or more, about 75% or more, about 80% or more, about 81% or more, about 82% or more, about 85%, about 90%, about 95%, or about 100% identity to the variable heavy chain region and variable light chain region of the antibody produced by clone CAN30G1, CAN30G2, CAN30G3, CAN30G4, CAN30G5, CAN54G1, CAN54G2, CAN54G3 or CAN40G1 (see Table 1) and which are capable of binding to an epitope of MARV GP.

In related disclosures, antibodies (or the antigen-binding portions) against an epitope of MARV GP comprise, for example, the CDRs of variable heavy chain regions and/or variable light chain regions of CAN30G1, CAN30G2, CAN30G3, CAN30G4, CAN30G5, CAN54G1, CAN54G2, CAN54G3 or CAN40G1.

The CDRs of the variable regions from CAN30G5, CAN30G2, CAN30G1, CAN30G3, CAN30G4, CAN54G2 and CAN40G1 are shown in Table 1. Table 1 also includes the amino acid and nucleotide sequences of the various regions of the murine CAN30G5, CAN30G2, CAN30G1, CAN30G3, CAN30G4, CAN54G2 and CAN40G1 monoclonal antibodies.

In certain disclosures, antibodies or antigen-binding portions include a variable heavy chain region comprising an amino acid sequence having at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 81% or more, about 82% or more, about 85%, about 90%, about 95%, or about 100% identity to a variable heavy chain region amino acid sequence selected from the group consisting of SEQ ID NO:14, SEQ ID NO:38, SEQ ID NO:62, SEQ ID NO:86, SEQ ID NO:110, SEQ ID NO:134, and SEQ ID NO:158 and are capable of binding an epitope of MARV GP.

In certain disclosures, antibodies or antigen-binding portions include a variable heavy chain region comprising an amino acid sequence selected from the group consisting of SEQ ID NO:14, SEQ ID NO:38, SEQ ID NO:62, SEQ ID NO:86, SEQ ID NO:110, SEQ ID NO:134, and SEQ ID NO:158 and are capable of binding an epitope of MARV GP.

Antibodies or antigen-binding portions of the disclosure can include a variable light chain regior comprising an amino acid sequence having at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 85%, about 90%, about 95%, or about 100% identity to a variable light chain region amino acid sequence selected from the group consisting of SEQ ID NO:2, SEQ ID NO:26, SEQ ID NO:50, SEQ ID NO:74, SEQ ID NO:98, SEQ ID NO:122, and SEQ ID NO:146 and are capable of binding an epitope of MARV GP.

In certain disclosures, antibodies or antigen-binding portions include a variable light chain region comprising an amino acid sequence selected from the group consisting of SEQ ID NO:2, SEQ ID NO:26. SEQ ID NO:50, SEQ ID NO:74, SEQ ID NO:98, SEQ ID NO:122, and SEQ ID NO:146 and are capable of binding an epitope of MARV GP.

In certain disclosures, the antibodies or antigen-binding portions include both a variable heavy chain region comprising an amino acid sequence having at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 85%, about 90%, about 95%, or about 100% identity to a variable heavy chain region amino acid sequence selected from the group consisting of SEQ ID NO:14, SEQ ID NO:38, SEQ ID NO:62, SEQ ID NO:86, SEQ ID NO:110, SEQ ID NO:134, and SEQ ID NO:158, and a variable light chain region including an amino acid sequence having at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 85%, about 90%, about 95%, or about 100% identity to a variable light chain amino acid sequence selected from the group consisting of SEQ ID NO:2, SEQ ID NO:26, SEQ ID NO:50, SEQ ID NO:74, SEQ ID NO:98, SEQ ID NO:122, and SEQ ID NO:146 and are capable of binding an epitope of MARV GP.

Antibodies or antigen-binding portions may comprise a variable light chain region and a variable heavy chain region wherein (a) the immunoglobulin light chain variable region comprises the amino acid sequence set forth in SEQ ID NO:2 and the immunoglobulin heavy chain variable region comprises the amino acid sequence set forth in SEQ ID NO:14; (b) the immunoglobulin light chain variable region comprises the amino acid sequence set forth in SEQ ID NO:26 and the immunoglobulin heavy chain variable region comprises the amino acid sequence set forth in SEQ ID NO:38; or
(c) the immunoglobulin light chain variable region comprises the amino acid sequence set forth in SEQ ID NO:74 and the immunoglobulin heavy chain variable region comprises the amino acid sequence set forth in SEQ ID NO:86.

Antibodies or antigen-binding portions can specifically bind to an epitope that overlaps with, or comprises at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 85%, about 90%, about 95%, or about 100% identity to an epitope bound by an antibody produced by clone CAN30G5, CAN54G2 or CAN40G1 and/or compete for binding to an epitope of MARV GP with an antibody produced by clone CAN30G5, CAN54G2 or CAN40G1.

Antibodies or antigen-binding portions described herein can specifically bind to an epitope that overlaps with an epitope bound by an antibody produced by clone CAN30G5, CAN54G2 or CAN40G1 and/or compete for binding to an epitope of MARV GP with an antibody produced by clone CAN30G5, CAN54G2 or CAN40G1.

In some embodiments, an antibody or an antigen-binding portion thereof binds specifically to a filovirus and comprises (i) an immunoglobulin light chain variable region comprising LCDR1, LCDR2, and LCDR3, and (ii) an immunoglobulin heavy chain variable region comprising HCDR1, HCDR2, and HCDR3, wherein
the LCDR1, LCDR2 and LCDR3 comprise the amino acid sequences set forth in SEQ ID NOs: 78, 79 and 80, respectively, and the HCDR1, HCDR2, and HCDR3 comprise the amino acid sequences set forth in SEQ ID NOs: 90, 91 and 92, respectively.

A variable heavy chain region of the antibodies or antigen-bindng portions described herein can comprise one, two, or three complementarity determining regions (CDRs) that comprise one, two, or three CDRs of the antibody produced by clone CAN30G5, CAN30G2, CAN30G1, CAN30G3, CAN30G4, CAN54G2 or CAN40G1 and may be capable of binding an epitope of MARV GP. A variable heavy chain CDR3 (HCDR3) of the antibodies or antigen-binding portions described herein can comprise the HCDR3 of the antibody produced by clone CAN30G5, CAN30G2, CAN30G1, CAN30G3, CAN30G4, CAN54G2 or CAN40G1 and may be capable of binding an epitope of MARV GP.

A variable light chain region of the antibodies or antigen-bindng portions described herein can comprise one, two, or three CDRs of a variable light chain region of the antibody produced by clone CAN30G5, CAN30G2, CAN30G1, CAN30G3, CAN30G4, CAN54G2 or CAN40G1 and may be capable of binding an epitope of MARV GP.

An antibody may be a humanized antibody. A humanized antibody of the present invention is an antibody from a non-human species where the amino acid sequence in the non-antigen binding regions (and/or the antigen-binding regions) has been altered so that the antibody more closely resembles a human antibody, and still retains its antigen binding ability.

Humanized antibodies can be generated by replacing sequences of the variable region that are not directly involved in antigen binding with equivalent sequences from human variable regions. Those methods include isolating, manipulating, and expressing the nucleic acid sequences that encode all or part of variable regions from at least one of a heavy or light chain. Sources of such nucleic acid are well known to those skilled in the art and, for example, may be obtained from a hybridoma producing an antibody against an epitope of MARV GP. The recombinant DNA encoding the humanized antibody, or fragment thereof, can then be cloned into an appropriate expression vector.

An antibody light or heavy chain variable region consists of a framework region interrupted by three hypervariable regions, referred to as complementarity determining regions (CDRs). Humanized antibodies are antibody molecules from non-human species can have one, two or three (all) CDRs from the non-human species as well as a framework region from a human immunoglobulin molecule.

The humanized antibodies of the present invention can be produced by methods known in the art. For example, once non-human (e.g., murine) antibodies are obtained, variable regions can be sequenced, and the location of the CDRs and framework residues determined. Kabat, E. A., et al. (1991) Sequences of Proteins of Immunological Interest, Fifth Edition, U.S. Department of Health and Human Services, NIH Publication No. 91-3242. Chothia, C. et al. (1987) J. Mol. Biol., 196:901-917. The light and heavy chain variable regions can, optionally, be ligated to corresponding constant regions. CDR-grafted antibody molecules can be produced by CDR-grafting or CDR substitution. One, two, or all CDRs of an immunoglobulin chain can be replaced. For example, all of the CDRs of a particular antibody may be from at least a portion of a non-human animal (e.g., mouse such as CDRs shown in Table 1) or only some of the CDRs may be replaced. It is only necessary to keep the CDRs required for binding of the antibody to a predetermined antigen (e.g., a conformational epitope of oligomeric Aβ). Morrison, S. L., 1985, Science, 229:1202-1207. Oi et al., 1986, BioTechniques, 4:214. U.S. Patent Nos. 5,585,089; 5,225,539; 5,693,761 and 5,693,762. EP 519596. Jones et al., 1986, Nature, 321:552-525. Verhoeyan et al., 1988, Science, 239:1534. Beidler et al., 1988, J. Immunol., 141:4053-4060.

Also encompassed by the present disclosure are antibodies or antigen-binding portions containing one, two, or all CDRs as disclosed herein, together with the other regions replaced by sequences from at least one different species including, but not limited to, human, rabbits, sheep, dogs, cats, cows, horses, goats, pigs, monkeys, apes, gorillas, chimpanzees, ducks, geese, chickens, amphibians, reptiles and other animals.

A chimeric antibody is a molecule in which different portions are derived from different animal species. For example, an antibody may contain a variable region derived from a murine antibody and a human immunoglobulin constant region. Chimeric antibodies can be produced by recombinant DNA techniques. Morrison, et al., Proc Natl Acad Sci, 81:6851-6855 (1984). For example, a gene encoding a murine (or other species) monoclonal antibody molecule is digested with restriction enzymes to remove the region encoding the murine Fc, and the equivalent portion of a gene encoding a human Fc constant region is substituted. Chimeric antibodies can also be created by recombinant DNA techniques where DNA encoding murine V regions can be ligated to DNA encoding the human constant regions. Better et al., Science, 1988, 240:1041-1043, Liu et al. PNAS, 1987 84:3439-3443. Liu et al., J. Immunol., 1987, 139:3521-3526. Sun et al. PNAS, 1987,84:214-218. Nishimura et al., Canc. Res., 1987,47:999-1005. Wood et al. Nature, 1985, 314:446-449. Shaw et al., J. Natl. Cancer Inst., 1988, 80:1553-1559. International Patent Publication Nos. WO1987002671 and WO 86/01533. European Patent Application Nos. 184, 187; 171,496; 125,023; and 173,494. U.S. Patent No. 4,816,567.

The antibodies of the disclosure can be full-length antibodies or can include a fragment (or fragments) of the antibody having an antigen-binding portion, including, but not limited to, Fab, F(ab')2, Fab', F(ab)', Fv, single chain Fv (scFv), bivalent polypeptides, multivalent polypeptides, antibody fusion proteins and conjugates, disulfide linked Fv, Fc, Fd, dAb fragment (e.g., Ward et al., Nature, 341:544-546 (1989)), an isolated CDR, diabodies, triabodies, tetrabodies, linear antibodies, single-chain, bi-specific, multispecific antibodies formed from antibody fragments, or variants and/or mixtures thereof. Single chain antibodies produced by joining antibody fragments using recombinant methods, or a synthetic linker, are also encompassed by the present invention. Bird et al. Science, 1988, 242:423-426. Huston et al., Proc. Natl. Acad. Sci. USA, 1988, 85:5879-5883.

The antibodies or antigen-binding portions may be monospecific, bi-specific or multispecific. Multispecific or bispecific antibodies or fragments may be specific for different epitopes of one target polypeptide (e.g., an epitope of MARV GP) or may contain antigen-binding domains specific for more than one target polypeptides (e.g., antigen-binding domains specific for different epitopes of MARV GP; antigen-binding domains specific for a conformational epitope of MARV GP and other antigen of MARV GP; antigen-binding domains specific for an epitope of MARV GP and an eptiope of EBOV GP or antigen-binding domains specific for an epitope of MARV GP and other kind of antigen such as a protein, a peptide, etc.). In one embodiment, a multispecific antibody or antigen-binding portion comprises at least two different variable domains, wherein each variable domain is capable of specifically binding to a separate antigen or to a different epitope on the same antigen.

In some embodiments, an antibody or antigen-binding portion binds to the GP of more than one MARV strain. In certain embodiments, an antibody or antigen-binding portion binds to the GP of MARV and the GP of at least one member of the *Ebolavirus* genus. One example of a panfilovirus antibody is antibody CAN40G1. A panfilovirus antibody may neutralize or prevent an infection by multiple filoviruses.

The present antibodies can be linked to or co-expressed with another functional molecule, e.g., another peptide or protein. For example, an antibody or fragment thereof can be functionally linked (e.g., by chemical coupling, genetic fusion, noncovalent association or otherwise) to one or more other molecular entities, such as another antibody or antibody fragment to produce a bi-specific or a multispecific antibody with a second binding specificity. For example, the present invention includes bi-specific antibodies wherein one arm of an immunoglobulin is specific for an epitope of MARV GP, and the other arm of the immunoglobulin is specific for a second therapeutic target or is conjugated to a therapeutic moiety, In another embodiment, the present application encompasses bi-specific antibodies that are specific both for an epitope of MARV GP and a ligand for capture, detection, removal for therapeutic purposes.

All antibody isotypes are encompassed by the present invention, including IgG (e.g., IgG1, IgG2, IgG3, IgG4), IgM, IgA (IgA1, IgA2), IgD or IgE. The antibodies or antigen-binding portions thereof may be mammalian (e.g., mouse, human) antibodies or antigen-binding portions thereof. The light chains of the antibody may be of kappa or lambda type.

Chemically and/or enzymatically treated antibodies or antigen-binding portion are also included in the present application, such as deglycosylation, pegylation, etc. The antibodies or antigen-binding portion can be molecularly manipulated, such as silencing of glycosylation sites (e.g. aglycosylated), etc.

The antibodies or antigen-binding portions can be formed from peptides. The peptides may also include variants, analogs, orthologs, homologs and derivatives of peptides, that exhibit a biological activity, e.g., binding of an antigen. The peptides may also contain one or more analogs of an amino acid (including, for example, non-naturally occurring amino acids, amino acids which only occur naturally in an unrelated biological system, modified amino acids from mammalian systems etc.), peptides with substituted linkages, as well as other modifications known in the art. In certain instances, specific amino acids may be substituted, deleted, silenced or added.

The present application encompasses glycosylation variants of the antibodies or antigen-binding portion described herein. These alternations do not have a substantial effect on the peptide's biological properties such as binding activity, but may improve half-life and/or bioavailability. For example, antibodies may have amino acid substitutions in the framework region, such as to improve binding to the antigen. In another example, a selected, small number of acceptor framework residues can be replaced by the corresponding donor amino acids. The donor framework can be a mature or germline human antibody framework sequence or a consensus sequence. Guidance concerning how to make phenotypically silent amino acid substitutions is provided in Bowie et al., Science, 247: 1306-1310 (1990). Cunningham et al., Science, 244: 1081-1085 (1989). Ausubel (ed.), Current Protocols in Molecular Biology, John Wiley and Sons, Inc. (1994). T. Maniatis, E. F. Fritsch and J. Sambrook, Molecular Cloning: A Laboratory Manual, Cold Spring Harbor laboratory, Cold Spring Harbor, N.Y. (1989). Pearson, Methods Mol. Biol. 243:307-31 (1994). Gonnet et al., Science 256:1443-45 (1992).

An antibody or antigen-binding portion can be derivatized or linked to another functional molecule. For example, an antibody can be functionally linked (by chemical coupling, genetic fusion, noncovalent interaction, etc.) to one or more other molecular entities, such as another antibody, a detectable agent, a cytotoxic agent, a pharmaceutical agent, a protein or peptide that can mediate association with another molecule (such as a streptavidin core region or a polyhistidine tag), or facilitate uptake across blood brain barrier (e.g. fusion to cholera toxin A subunits), block or interact with receptors, amino acid linkers, signal sequences, immunogenic carriers, or ligands useful in protein purification, such as glutathione-S-transferase, histidine tag, and staphylococcal protein A. One type of derivatized protein is produced by crosslinking two or more proteins (of the same type or of different types). Suitable crosslinkers include those that are heterobifunctional, having two distinct reactive groups separated by an appropriate spacer (e.g., m-maleimidobenzoyl-N-hydroxysuccinimide ester) or homobifunctional (e.g., disuccinimidyl suberate). Such linkers are available from Pierce Chemical Company, Rockford, III. Useful detectable agents with which a protein can be derivatized (or labeled) include fluorescent compounds, various enzymes, prosthetic groups, luminescent materials, bioluminescent materials, and radioactive materials. Non-limiting, exemplary fluorescent detectable agents include fluorescein, fluorescein isothiocyanate, rhodamine, and, phycoerythrin. A protein or antibody can also be derivatized with detectable enzymes, such as alkaline phosphatase, horseradish peroxidase, beta-galactosidase, acetylcholinesterase, glucose oxidase and the like. A protein can also be derivatized with a prosthetic group (e.g., streptavidin/biotin and avidin/biotin).

The present polypeptides may be the functionally active variant of antibodies of antigen-binding portions thereof disclosed herein, e.g., with less than about 30%, about 25%, about 20%, about 15%, about 10%, about 5% or about 1% amino acid residues substituted or deleted but retain essentially the same immunological properties including, but not limited to, binding to an epitope of MARV GP.

The invention also encompasses a nucleic acid encoding the claimed antibody or antigen-binding portion thereof that specifically binds to an epitope of MARV GP. The nucleic acids comprise, for example, polynucleotides that encode all or part of the claimed antibody, for example, one or both chains of the antibody, or a fragment, derivative, mutant, or variant thereof. The nucleic acids can comprise one or more additional sequences, for example, regulatory sequences, and/or be part of a larger nucleic acid, for example, a vector. The nucleic acid may be expressed in a cell to produce the present antibody or antigen-binding portion thereof. An isolated nucleic acid described herein may comprise a sequence encoding a peptide comprising at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 70%, about 75%, about 80%, about 85%, about 90%, about 95%, or about 100% identity to SEQ ID NO:2, SEQ ID NO:26, SEQ ID NO:50, SEQ ID NO:74, SEQ ID NO:98, SEQ ID NO:122, and SEQ ID NO:146. An isolated nucleic acid described herein may comprise a sequence encoding a peptide comprising at least about 70%, at least about 75%, at least about 80%, at least about 85%, at least about 90%, at least about 95%, at least about 99%, about 70% to about 100%, about 80% to about 100%, about 90% to about 100%, about 95% to about 100%, about 70%, about 75%, about 80%, about 70%, about 75%, about 80%, about 85%, about 90%, about 95%, or about 100% identity to SEQ ID NO: 14, SEQ ID NO:38, SEQ ID NO:62, SEQ ID NO:86, SEQ ID NO:110, SEQ ID NO:134, and SEQ ID NO:158.

In some disclosures, a nucleic acid molecule comprises one or more nucleotide sequences selected from the group consisting of SEQ ID NO:1, SEQ ID NO:3, SEQ ID NO:4, SEQ ID NO:5, SEQ ID NO:13, SEQ ID NO:15, SEQ ID NO:16, SEQ ID NO:17, SEQ ID NO:25, SEQ ID NO:27, SEQ ID NO:28, SEQ ID NO:29, SEQ ID NO:37, SEQ ID NO:39, SEQ ID NO:40, SEQ ID NO:41, SEQ ID NO:49, SEQ ID NO:51, SEQ ID NO:52, SEQ ID NO:53, SEQ ID NO:61, SEQ ID NO:63, SEQ ID NO:64, SEQ ID NO:65, SEQ ID NO:73, SEQ ID NO:75, SEQ ID NO:76, SEQ ID NO:77, SEQ ID NO:85, SEQ ID NO:87, SEQ ID NO:88, SEQ ID NO:89, SEQ ID NO:97, SEQ ID NO:99, SEQ ID NO:100, SEQ ID NO:101, SEQ ID NO:109, SEQ ID NO:111, SEQ ID NO:112, SEQ ID NO:113, SEQ ID NO:121, SEQ ID NO:123, SEQ ID NO:124, SEQ ID NO:125, SEQ ID NO:133, SEQ ID NO:135, SEQ ID NO:136, SEQ ID NO:137, SEQ ID NO:145, SEQ ID NO:147, SEQ ID NO:148, SEQ ID NO:149, SEQ ID NO:157, SEQ ID NO:159, SEQ ID NO:160, and SEQ ID NO:161.

In another aspect, the present invention provides vectors comprising a nucleic acid encoding the claimed antibody or a portion thereof. The invention also encompasses expression vectors comprising any of nucleic acids molecules or nucleotide sequences as claimed. Examples of vectors include, but are not limited to, plasmids, viral vectors, non-episomal mammalian vectors and expression vectors, for example, recombinant expression vectors.

The recombinant expression vectors as claimed can comprise a nucleic acid of the invention in a form suitable for expression of the nucleic acid in a host cell. The recombinant expression vectors include one or more regulatory sequences, selected on the basis of the host cells to be used for expression, which is operably linked to the nucleic acid sequence to be expressed. The regulatory sequence can, for example, exert its effects directly on the regulated nucleic acid, or through the action of one or more other molecules (e.g., polypeptides that bind to the regulatory sequence and/or the nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology 185, Academic Press, San Diego, Calif. and Baron et al., 1995, Nucleic Acids Res. 23:3605-06. Regulatory sequences include those that direct constitutive expression of a nucleotide sequence in many types of host cells (e.g., SV40 early gene enhancer, Rous sarcoma virus promoter, cytomegalovirus promoter, etc.), those that direct expression of the nucleotide sequence only in certain host cells (e.g., tissue-specific regulatory sequences, see Voss et al., 1986, Trends Biochem. Sci. 11:287, Maniatis et al., 1987, Science 236:1237), and those that direct inducible expression of a nucleotide sequence in response to particular treatment or condition (e.g., the metallothionin promoter in mammalian cells, the tet-responsive and/or streptomycin responsive promoter in both prokaryotic and eukaryotic systems, etc.). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

Changes can be introduced by mutation into a nucleic acid, thereby leading to changes in the amino acid sequence of a polypeptide (e.g., the antibody or fragment thereof) that it encodes. Mutations can be introduced using any technique known in the art. In one disclosure, one or more particular amino acid residues are changed using, for example, a site-directed mutagenesis protocol. In another disclosure, one or more randomly selected residues is changed using, for example, a random mutagenesis protocol. However it is made, a mutant polypeptide can be expressed and screened for a desired property (e.g., binding to MARV GP).

Nucleic acid molecules encoding a functionally active variant of an antibody or antigen-binding portion thereof of the present disclosure are also described. These nucleic acid molecules may hybridize with a nucleic acid encoding an antibody or antigen-binding portion thereof of the present disclosure under medium stringency, high stringency, or very high stringency conditions. Guidance for performing hybridization reactions can be found in Current Protocols in Molecular Biology, John Wiley & Sons, N.Y. 6.3.1-6.3.6, 1989.

Specific hybridization conditions referred to herein are as follows: 1) medium stringency hybridization conditions: 6XSSC at about 45°C, followed by one or more washes in 0.2XSSC, 0.1% SDS at 60°C; 2) high stringency hybridization conditions: 6XSSC at about 45°C, followed by one or more washes in 0.2XSSC, 0.1% SDS at 65°C; and 3) very high stringency hybridization conditions: 0.5 M sodium phosphate, 7% SDS at 65°C, followed by one or more washes at 0.2XSSC, 1% SDS at 65°C.

A nucleic acid encoding the claimed antibody or antigen-binding portion thereof may be introduced into an expression vector that can be expressed in a suitable expression system, followed by isolation or purification of the expressed antibody or antigen-binding portion thereof. Optionally, a nucleic acid encoding the present antibody or antigen-binding portion thereof can be translated in a cell-free translation system. U.S. Patent No. 4,816,567. Queen et al., Proc Natl Acad Sci USA, 86:10029-10033 (1989).

The claimed antibodies or portions thereof can be produced by host cells transformed with DNA encoding light and heavy chains (or portions thereof) of a desired antibody. Antibodies can be isolated and purified from these culture supernatants and/or cells using standard techniques. For example, a host cell may be transformed with DNA encoding the light chain, the heavy chain, or both, of an antibody. Recombinant DNA technology may also be used to remove some or all of the DNA encoding either or both of the light and heavy chains that is not necessary for binding, e.g., the constant region.

The claimed nucleic acids can be expressed in various suitable cells, including prokaryotic and eukaryotic cells, e.g., bacterial cells, (e.g., *E*. coli), yeast cells, plant cells, insect cells, and mammalian cells. A number of mammalian cell lines are known in the art and include immortalized cell lines available from the American Type Culture Collection (ATCC). Non-limiting examples of the cells include all cell lines of mammalian origin or mammalian-like characteristics, including but not limited to, parental cells, derivatives and/or engineered variants of monkey kidney cells (COS, e.g., COS-1, COS-7), HEK293, baby hamster kidney (BHK, e.g., BHK21), Chinese hamster ovary (CHO), NS0, PerC6, BSC-1, human hepatocellular carcinoma cells (e.g., Hep G2), SP2/0, HeLa, Madin-Darby bovine kidney (MDBK), myeloma and lymphoma cells. The engineered variants include, e.g., glycan profile modified and/or site-specific integration site derivatives.

The present invention also provides for cells comprising the nucleic acids as claimed.

The cells may be a hybridoma or transfectant. The types of the cells are discussed above.

The present antibody or antigen-binding portion can be expressed in various cells. The types of the cells are discussed above.

Alternatively, the present antibody or antigen-binding portion can be synthesized by solid phase procedures well known in the art. Solid Phase Peptide Synthesis: A Practical Approach by E. Atherton and R. C. Sheppard, published by IRL at Oxford University Press (1989). Methods in Molecular Biology, Vol. 35: Peptide Synthesis Protocols (ed. M. W.Pennington and B. M. Dunn), chapter 7. Solid Phase Peptide Synthesis, 2nd Ed., Pierce Chemical Co., Rockford, IL (1984). G. Barany and R. B. Merrifield, The Peptides: Analysis, Synthesis, Biology, editors E. Gross and J. Meienhofer, Vol. 1 and Vol. 2, Academic Press, New York, (1980), pp. 3-254. M. Bodansky, Principles of Peptide Synthesis, Springer-Verlag, Berlin (1984).

Conventional methods can be used to prepare the present antigens and constructs to be used as but not limited to immunogens and screening peptides.

In the present invention, marburgvirus (MARV) and ebolavirus (EBOV) glycoproteins (GP) antigens were produced by stable cell line expression in Drosophila S2 and Spodoptera Sf9, or by transient transfection in Gnt-/- HEK293 cells. Proteins were engineered with either a strep or HA tag at the C terminus to facilitate purification using Qiagen straptactin or Roche anti-HA affinity resin, respectively.

Immunogenic screening constructs were produced for multiple strains of MARV GP and EBOV GP: GPe, GPeΔmuc, GPeΔmucΔw, GPcl. Table 2 and Figure 1 illustrate the nature of the constructs; (i) the MARV GP and EBOV GP ectodomain constructs (GPe) lack the transmembrane (TM) domain and consist of residues 1-637; (ii) the MARV GP ectodomain mucin-deieted constructs (GPeΔmuc) also lack the mucine-like domain: Δ257-425 for all MARV strains; (iii) the EBOV GP ectodomain mucin-deleted constructs (GPeΔmuc) also lack the mucine-like domain: Δ314-463 for EBOV, SUDV, BDBV and Δ316-470 for RESTV the MARV GP construct GPeΔmucΔw lacks both the GP1 mucin domain (Δ257-425) and the GP2-wing (Δ436-483); the cleaved MARV GP (GPcl) construct was produced to mimic endosomal protease cleavage. The construct was produced by incubation of 1 mg MARV Ravn GPeΔmuc with 0.01mg trypsin at 37°C for 1 hour. Cleaved EBOV GP was produced by treatment with thermolysin. GPe proteins were further purified by Superose 6 and all other GP proteins were purified by Superdex 200 size exclusion chromatography.

Conventional methods can be used to prepare the present antibodies including polyclonal antisera or monoclonal antibodies.

To produce polyclonal antibodies, a mammal (e.g. a mouse, hamster, or rabbit) can be immunized with an immunogenic form of the epitope which elicits an antibody response in the mammal. Following immunization, antisera can be obtained and, if desired, polyclonal antibodies isolated from the sera.

The present disclosure provides for a method for making a hybridoma that expresses an antibody that specifically binds to an epitope of MARV GP. The method contains the following steps: immunizing an animal with a composition that includes an epitope of MARV GP (e.g., a mucin deleted MARV GP or other peptides as disclosed herein); isolating splenocytes from the animal; generating hybridomas from the splenocytes; and selecting a hybridoma that produces an antibody that specifically binds to an epitope of MARV GP. Kohler and Milstein, Nature, 256: 495, 1975. Harlow, E. and Lane, D. Antibodies: A Laboratory Manual, Cold Spring Harbor Laboratory Press, Cold Spring Harbor, N.Y., 1988.

Mice can be immunized subcutaneously, intraperitoneally or intravenously with the antigen. After immunization, one or more boosts may or may not be given. The titers of the antibodies in the plasma can be monitored by, e.g., ELISA (enzyme-linked immunosorbent assay), other immunoassay procedures, or flow cytometry. Mice with sufficient titers of the antibodies against an epitope of MARV GP are used for fusions. Mice may or may not be boosted with the antigen 3 days before sacrifice and removal of the spleen. Mice may or may not be boosted with a different antigen before sacrifice and removal of the spleen. The mouse splenocytes are isolated and fused with PEG to a mouse myeloma cell line. The resulting hybridomas are then screened for the production of antigen-specific antibodies. Cells are plated, and then incubated in selective medium. Supernatants from individual wells are then screened by ELISA for monoclonal antibodies against the antigen. The antibody secreting hybridomas are re-plated, screened again, and if still positive for anti-antigen monoclonal antibodies, can be subcloned by limiting dilution.

Adjuvants that may be used to increase the immunogenicity of an antigen, e.g., an epitope of MARV GP, include any compound or compounds that act to increase an immune response to peptides or combination of peptides. Non-limiting examples of adjuvants include alum, aluminum phosphate, aluminum hydroxide, MF59 (4.3% w/v squalene, 0.5% w/v polysorbate 80 (Tween 80), 0.5% w/v sorbitan trioleate (Span 85)), CpG-containing nucleic acid, QS21 (saponin adjuvant), MPL (Monophosphoryl Lipid A), 3DMPL (3-O-deacylated MPL), extracts from Aquilla, ISCOMS (see, e.g., Sjolander et al. (1998) J. Leukocyte Biol. 64:713; WO90/03184; WO96/11711; WO 00/48630; WO98/36772; WO00/41720; WO06/134423 and WO07/026190), LT/CT mutants, poly(D,L-lactide-co-glycolide) (PLG) microparticles, Quil A, interleukins, Freund's, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dip-almitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion.

The immunized animal can be any animal that is capable of producing recoverable antibodies when administered an immunogen, such as, but not limited to, mice, rabbits, rats, hamsters, goats, horses, monkeys, baboons and humans. In one aspect, the host is transgenic and produces human antibodies, e.g., a mouse expressing the human immunoglobulin gene segments. U.S. Patent No. 8,236,311; 7,625,559 and 5,770,429.

Lonberg et al., Nature 368(6474): 856-859, 1994. Lonberg, N., Handbook of Experimental Pharmacology 113:49-101, 1994. Lonberg, N. and Huszar, D., Intern. Rev. Immunol., 13: 65-93, 1995. Harding, F. and Lonberg, N., Ann. N.Y. Acad. Sci., 764:536-546, 1995.

After the host is immunized and the antibodies are produced, the antibodies are assayed to confirm that they are specific for the antigen of interest and to determine whether they exhibit any cross reactivity with other antigens. One method of conducting such assays is a sera screen assay as described in U.S. Patent Publication No. 2004/0126829. Antibodies against an epitope of MARV GP can be characterized for binding to the antigen by a variety of known techniques.

For example, in an ELISA, microtiter plates are coated with the antigen in PBS, and then blocked with irrelevant proteins such as bovine serum albumin (BSA) diluted in PBS. Dilutions of plasma from antigen-immunized mice are added to each well and incubated. The plates are washed and then incubated with a secondary antibody conjugated to an enzyme (e.g., alkaline phosphatase). After washing, the plates are developed with the enzyme's substrate (e.g., ABTS), and analyzed at a specific OD. In other embodiments, to determine if the selected monoclonal antibodies bind to unique epitopes, the antibody can be biotinylated which can then be detected with a streptavidin labeled probe. Anti-antigen antibodies can be tested for reactivity with the antigen by Western blotting.

Antibodies may also be assayed by *in vitro* multiplex bead-based immunoassays. Multiplex bead-based immunoassays, such as the Luminex® xMAP® technology, allow the measurement of one analyte or simultaneous measurement of multiple analytes using a library of antigen-containing (or epitope-containing) peptides (or proteins) coupled to color-coded beads. Each bead is identified by the unique wavelength it emits when excited by a laser. Quantitation is accomplished by a sandwich assay using a fluorescently labeled detection antibody with affinity to the specific analyte captured by the bead-coupled antibody beads. Excitation by a second laser reads the quantity of bound detection antibody. Houser, Brett, Using Bead-Based Multiplexing Immunoassays to Explore Cellular Response to Drugs, Drug Discover and Development, May 9, 2011. The beads that can be used in the Luminex© xMAP® immunoassays include MagPlex®, MicroPlex®, LumAvidin®, SeroMAP™ microspheres, etc. MagPlex® microspheres are superparamagnetic microspheres which are internally labeled with fluorescent dyes and contain surface carboxyl groups for covalent attachment of ligands (or biomolecules). Baker et al., Conversion of a Capture ELISA to a Luminex© xMAP® Assay using a Multiplex Antibody Screening Method, J. Vis. Exp., (65), e4084 10.3791/4084, DOI: 10.3791/4084 (2012). Fulton et al., Advanced multiplexed analysis with the FlowMetrix system. Clinical Chemistry, 43, 1749-1756 (1997). Carson et al., Simultaneous quantitation of 15 cytokines using a multiplexed flow cytometric assay, J. Immunol. Methods, 227, 41-52 (1999).

In one embodiment, to detect the presence of antibodies to the epitope of MARV GP in a sample, the GPs are coupled to MagPlex® microspheres based on the multiplexing xMAP® platform and analyzed on the MagPix® instrument (Luminex Corporation, Austin, Texas). The sample is then contacted with GP-coupled microspheres, an immunoassay is used to detect and quantify antibodies specific to the GP.

Different peptides, e.g., Ravn GPeΔmuc, Angola GPeΔmuc, Musoke GPeΔmuc, Ci67 GPeΔmuc, Ravn GPe, Angola GPe, EBOV GPe, SUDV GPe, BDBV GPe, RESTV GPe, EBOV GPeΔmuc, SUDV GPeΔmuc, BDBV GPeΔmuc, RESTV GPeΔmuc, Ravn GPeΔmucΔw peptides as disclosed herein, may be coupled to different sets or regions of MagPlex® microspheres. Because each of these regions has a unique internal fluorescent dye, the immunoassay is able to discriminate between the antibodies specific to the different peptides.

Biacore™ assay can be used to characterize antibodies by measuring protein-protein interaction and binding affinity based on surface plasmon resonance (SPR). Karlsson et al., Analysis of active antibody concentration, Journal of Immunological Methods, (1993) 166, 75-84. Markey F., Measuring concentration, Biajournal, 1999, 2: 8 - 11. Antibody titers can also be measured by radioimmunoassay.

Hybridomas that produce antibodies that bind, preferably with high affinity, to the epitope of MARV GP can than be subcloned and further characterized. One clone from each hybridoma, which retains the reactivity of the parent cells (by ELISA), can then be chosen for making a cell bank, and for antibody purification.

To purify the anti-antigen antibodies, supernatants from the cultured hybridomas can be filtered and concentrated before affinity chromatography with protein A-Sepharose (Pharmacia, Piscataway, N.J.).

The present antibodies or antigen-binding portions have *in vitro* and *in vivo* therapeutic and/or prophylactic utilities. For example, these antibodies can be administered to cells in culture, e.g., *in vitro* or *ex vivo,* or to a subject, e.g., *in vivo,* to prevent a filovirus infection such as a MARV infection, inhibit or delay the onset of the infection, or slow progression of the infection. The invention encompasses a method for detecting a filovirus glycoprotein (e.g., a Marburg virus, Ebola virus, Sudan virus, Bundibugyo virus, Tai Forest virus or Reston virus glycoprotein) in a sample, the method comprising contacting the sample with any of the monoclonal antibodies or antigen-binding portions as claimed. A sample may be a cell, tissue, or biological fluid from a subject suspected of having or at risk of a filovirus infection.

Filovirus infections encompassed by the present application include, but are not limited to, *marburgvirus* and *ebolavirus.*

Infected subjects receiving the present compositions can be in the early, middle or late stages of infection, with mild, moderate or severe symptoms. In other cases, individuals suspected of being exposed to the virus may also receive such treatment, so the spread of the virus may be halted or their risk of developing symptoms may be diminished or eliminated. In other words, the anti-MARV treatment can be for use in a method of preventing MARV infection, other filovirus infections, or inhibiting the onset of fatal symptoms of the infection.

The claimed compositions, antibodies and antigen-binding portions may be for use in amelioration, prophylaxis and/or treatment of a filovirus infection. Ameliorating, or reducing, or reduction of infection or disease, as used herein, can include but is not limited to delaying the onset of the infection, attenuating the symptoms of the infection, shortening the duration of the infection, reducing the viral titer in a patient (eg. in the blood), or slowing the progression of the infection. Filovirus infections encompassed by the present application include, but are not limited to, marburgvirus and ebolavirus. One or more antibodies or portions may be administered to a subject. The one or more antibodies or antigen-binding portions that are administered may comprise (i) an immunoglobulin light chain variable region comprising LCDR1, LCDR2, and LCDR3, and (ii) an immunoglobulin heavy chain variable region comprising HCDR1, HCDR2, and HCDR3, wherein
the LCDR1, LCDR2 and LCDR3 comprise the amino acid sequences set forth in SEQ ID NOs: 78, 79 and 80, respectively, and the HCDR1, HCDR2, and HCDR3 comprise the amino acid sequences set forth in SEQ ID NOs: 90, 91 and 92, respectively;
or an immunoglobulin light chain variable region comprising the amino acid sequence set forth in SEQ ID NO:2 and an immunoglobulin heavy chain variable region comprising the amino acid sequence set forth in SEQ ID NO:14; or an immunoglobulin light chain variable region comprising the amino acid sequence set forth in SEQ ID NO:26 and an immunoglobulin heavy chain variable region comprising the amino acid sequence set forth in SEQ ID NO:38.

The invention includes administering a pharmaceutically effective amount of a pharmaceutical composition to an individual diagnosed with a filovirus infection or showing symptoms of a filovirus infection. A pharmaceutically effective amount of the present pharmaceutical composition can be administered to an individual at risk of being exposed to and contracting a filovirus infection. A pharmaceutically effective amount to be administered to the subject can be determined by a physician or other health care professional with consideration of individual differences in age, weight, disease severity, dose and frequency of administration, and individual response to the therapy. An exemplary, non-limiting range for a therapeutically or prophylactically effective amount of an antibody or antigen-binding portion of the invention is from about 0.001 to about 60 mg/kg body weight, about 0.01 to about 30 mg/kg body weight, about 0.01 to about 25 mg/kg body weight, about 0.5 to about 25 mg/kg body weight, about 0.1 to about 20 mg/kg body weight, about 10 to about 20 mg/kg body weight, about 0.75 to about 10 mg/kg body weight, about 1 to about 10 mg/kg body weight, about 2 to about 9 mg/kg body weight, about 1 to about 2 mg/kg body weight, about 3 to about 8 mg/kg body weight, about 4 to about 7 mg/kg body weight, about 5 to about 6 mg/kg body weight, about 8 to about 13 mg/kg body weight, about 8.3 to about 12.5 mg/kg body weight, about 4 to about 6 mg/kg body weight, about 4.2 to about 6.3 mg/kg body weight, about 1.6 to about 2.5 mg/kg body weight, about 2 to about 3 mg/kg body weight, or about 10 mg/kg body weight.

The antibody or antigen-binding portion thereof can be administered alone or in combination together with another therapeutic agent, e.g., a second monoclonal or polyclonal antibody or antigen-binding portion, or other therapeutic agents to treat filovirus infections (e.g. antiviral agents). The second therapeutic agent may be another anti-filovirus antibody or antigen-binding portion, and may be any of the antibodies or portions described herein. In one embodiment, a pharmaceutical composition comprises at least one Marburg virus-binding antibody or antigen-binding portion thereof, and at least one Ebola virus-binding antibody or antigen-binding portion thereof. A pharmaceutical composition may comprise at least two, at least three, or at least four Marburg virus-binding antibodies or antigen-binding portion thereof. A pharmaceutical composition may comprise at least two Marburg virus-binding antibodies or antigen-binding portion thereof, and wherein each Marburg virus-binding antibody or antigen-binding portion binds different epitopes of the Marburg virus glycoprotein. As used herein, "different epitopes" may be overlapping or non-overlapping.

The pharmaceutical compositions, therapeutic compositions, or immunogenic compositions may contain the antibodies together with one or more other active agents. Alternatively, the present compositions may be administered consecutively, simultaneously or in combination with one or more other active agents. Non-limiting examples of the active agents that may be used in combination with the present compositions include an anti-EBOV monoclonal antibody. The other active agents that can be used in combination with the present composition include those that are useful for treating marburgvirus infections or related filovirus infections. For example, the antibody or antigen-binding portion thereof of the present disclosure may be co-formulated and/or coadministered with one or more additional antibodies that bind other targets. An additional antibody may be another anti-filovirus antibody or antigen-binding portion, and may be any of the antibodies or portions described herein. In one embodiment, an antibody or portion of the invention is co-formulated or coadministered with at least one Ebola virus-binding antibody or antigen-binding portion thereof. An antibody or portion of the invention may be co-formulated or coadministered with at least one at least two, at least three, or at least four other Marburg virus-binding antibodies or antigen-binding portion thereof. An antibody or portion of the invention may be co-formulated or coadministered with at least one other Marburg virus-binding antibody or antigen-binding portion thereof, wherein each Marburg virus-binding antibody or antigen-binding portion binds different epitopes of the Marburg virus glycoprotein. As used herein, "different epitopes" may be overlapping or non-overlapping.

The present disclosure further encompasses vaccines and immunogen-containing compositions. The vaccines or immunogen-containing compositions may comprise one or more epitopes recognized and/or bound by one or more of the antibodies or antigen-binding portion thereof of the disclosure. In one disclosure, the vaccines or immunogen-containing compositions comprises one or more epitope recognized and/or bound by one or more of CAN30G1, CAN30G2, CAN30G3, CAN30G4, CAN30G5, CAN54G1, CAN54G2, CAN54G3 or CAN40G1, or antigen-binding portion of any of these antibodies. The vaccines or immunogen-containing compositions may contain the epitope, or may contain a peptide or protein having the epitope. In one disclosure, the epitope-containing portions, fragments, or peptides are derived from MARV GP. The epitope-containing portions, fragments, or peptides may also be chemically or recombinantly synthesized.

The present disclosure also provides compositions containing an antibody or antigen-binding portion thereof described herein. The composition may contain an isolated nucleic acid encoding the present antibody or antigen-binding portion thereof. The present compositions may also contain a pharmaceutically acceptable carrier. Pharmaceutically acceptable carriers include any and all solvents, dispersion media, isotonic and absorption delaying agents, and the like that are physiologically compatible. The pharmaceutical composition may be used for preventing the onset or reducing the severity or duration of a filovirus infection. As discussed above, the antibodies or antigen-binding portions thereof are specific to MARV GP.

The invention also features a pharmaceutical composition containing at least one antibody or antigen-binding portion thereof as claimed for use in methods of ameliorating, treating or preventing a filovirus infection in a subject by administering to the subject the pharmaceutical composition in an amount effective to treat or prevent a filovirus infection.

In certain embodiments, the present composition can be administered to a subject at a dose ranging from about 1 µg to 1 mg/kg body weight, about 10 µg to 800 µg/kg body weight, about 20 µg to 600 µg/kg body weight, about 30 µg to 500 µg/kg body weight, about 10 µg to 400 µg/kg body weight, about 20 µg to 400 µg/kg body weight, about 60 µg to 100 µg/kg body weight, about 10 µg to 200 µg per kg body weight, about 100 µg to 200 µg/kg body weight, about 50 µg/kg body weight, or about 100 µg/kg body weight. The dose may also range from about 10 mg/kg of body weight to about 5 g/kg body weight, about 5 mg/kg of body weight to about 2 g/kg body weight, about 50 mg/kg of body weight to about 4 g/kg body weight, about 100 mg/kg of body weight to about 3 g/kg body weight, about 0.1 g/kg body weight to about 1 g/kg body weight, about 0.2 g/kg body weight to about 0.8 g/kg body weight, about 0.2 g/kg of body weight to about 4 g/kg body weight, about 10 mg/kg of body weight to about 50 mg/kg body weight, about 0.2 g/kg body weight, about 0.4 g/kg body weight, about 0.8 g/kg body weight, about 5 mg/kg body weight to about 500 mg/kg body weight, at least about 10 mg/kg body weight, at least about 15 mg/ kg body weight, at least about 20 mg/kg body weight, at least about 25 mg/kg body weight, at least about 30 mg/kg body weight or at least 50 mg/kg body weight, up to about 100 mg/kg body weight, up to about 150 mg/kg body weight, up to about 200 mg/kg body weight, up to about 250 mg/kg body weight, up to about 300 mg/kg body weight, or up to about 400 mg/kg body weight. In other embodiments, the doses of the immunoglobulin can be greater or less.

Using a mass/volume unit, the present composition can be administered to a subject at a dose ranging from about 0.1 mg/ml to about 2000 mg/ml, or any amount there between, for example 0.1, 0.5, 1, 2, 5, 10, 15, 20, 25, 30, 35, 40, 10 50, 60, 70, 80, 90, 100, 120, 140, 160 180, 200, 250, 500, 750, 1000, 1500, 2000 mg/ml, or any amount therebetween; or from about 1 mg/ml to about 2000 mg/ml, or any amount there between, for example, 1.0, 2.0, 5.0, 10.0, 15.0, 20.0, 25.0, 30.0, 35.0, 40.0, 50.0 60.0, 70.0, 80.0, 90.0, 100, 120, 140, 160 180, 200, 250, 500, 750, 1000, 1500, 2000, mg/ml or any amount there between; or from about 10mg/ml to about 1000mg/ml or any amount 15 there between, for example, 10.0, 15.0, 20.0, 25.0, 30.0, 35.0, 40.0, 50.0 60.0, 70.0, 80.0, 90.0, 100, 120, 140, 160 180, 200, 250, 500, 750, 1000 mg/ml, or any amount there between; or from about 30mg/ml to about 1000mg/ml or any amount there between, for example 30.0, 35.0, 40.0, 50.0 60.0, 70.0, 80.0, 90.0, 100, 120, 140, 160 180, 200, 250, 500, 750, 1000 mg/ml.

The data obtained from the cell culture assays and animal studies can be used in formulating a range of dosage for use in humans or other animal species. In one embodiment, the dosage of such compounds lies within a range of circulating concentrations that include the ED50 with little or no toxicity. The dosage can vary within this range depending upon the dosage form employed and the route of administration utilized. In another embodiment, the therapeutically effective dose can be estimated initially from cell culture assays. A dose can be formulated in animal models to achieve a circulating plasma concentration range that includes the IC50 (i.e., the concentration of the test compound which achieves a half-maximal inhibition of symptoms) as determined in cell culture. Sonderstrup, Springer, Sem. Immunopathol. 25: 35-45, 2003. Nikula et al., Inhal. Toxicol. 4(12): 123-53, 2000.

The composition is formulated to contain an effective amount of the claimed antibody or antigen-binding portion thereof, wherein the amount depends on the subject to be treated and the condition to be treated. In one embodiment, the claimed antibody or antigen-binding portion thereof is administered at a dose ranging from about 0.01 mg to about 10 g, from about 0.1 mg to about 9 g, from about 1 mg to about 8 g, from about 1 mg to about 7 g, from about 5 mg to about 6 g, from about 10 mg to about 5 g, from about 20 mg to about 1 g, from about 50 mg to about 800 mg, from about 100 mg to about 500 mg, from about 0.01mg to about 10 g, from about 0.05 µg to about 1.5 mg, from about 10 µg to about 1 mg protein, from about 30 µg to about 500 µg, from about 40 pg to about 300 pg, from about 0.1 µg to about 200 mg, from about 0.1 µg to about 5 µg, from about 5 µg to about 10 µg, from about 10 µg to about 25 µg, from about 25 µg to about 50 µg, from about 50 µg to about 100 µg, from about 100 µg to about 500 µg, from about 500 µg to about 1 mg, from about 1 mg to about 2 mg. The specific dose level for any particular subject depends upon a variety of factors including the activity of the specific peptide, the age, body weight, general health, sex, diet, time of administration, route of administration, and rate of excretion, drug combination and the severity of the particular disease undergoing therapy.

The duration of administration can vary: it may range from about 10 minutes to about 1 day, from about 30 minutes to about 20 hours, from about 1 hour to about 15 hours, from about 2 hours to about 10 hours, from about 3 hours to about 8 hours, from about 4 hours to about 6 hours, from about 1 day to about 1 week, from about 2 weeks to about 4 weeks, from about 1 month to about 2 months, from about 2 months to about 4 months, from about 4 months to about 6 months, from about 6 months to about 8 months, from about 8 months to about 1 year, from about 1 year to about 2 years, or from about 2 years to about 4 years, or more. The duration of administration may be about 1 month, about 3 months, about 6 months, about 1 year, about 18 months, about 2 years, about 5 years, or about 10 years. In one embodiment, the treatment may last the remainder of a subject's natural life.

Compositions can be administered in a single dose treatment or in multiple dose treatments on a schedule and over a time period appropriate to the age, weight and condition of the subject, the particular composition used, and the route of administration. In one embodiment, a single dose of the composition according to the invention is administered. In other embodiments, multiple doses are administered. The frequency of administration can vary depending on any of a variety of factors, e.g., severity of the symptoms, degree of immunoprotection desired, whether the composition is used for prophylactic or curative purposes, etc. For example, the composition according to the invention is administered about once per day, once per month, twice per month, three times per month, about once every other month, every other week (qow), once per week (qw), twice per week (biw), three times per week (tiw), four times per week, five times per week, six times per week, every other day (qod), daily (qd), twice a day (qid), three times a day (tid), about once every 6 months, about once a year, about once every 2 years, or about once every 5 years, etc. The composition may also be administered in one or more doses per day.

Effectiveness of the treatment may be assessed during the entire course of administration after a certain time period, e.g., about every 3 months, about every 6 months, about every 9 years, about every year, etc. The administration schedule (dose and frequency) may be adjusted accordingly for any subsequent administrations. U.S. Patent Nos. 8,066,993 and 7,968,293.

Compositions or nucleic acids, polypeptides, or antibodies of the invention can be delivered alone or as pharmaceutical compositions by any routes known in the art, e.g., systemically, regionally, or locally; by intra-arterial, intrathecal (IT), intramuscular, intravenous (IV), parenteral, intra-pleural cavity, topical, oral, enteral, intranasal, intrapulmonary or inhalational, subcutaneous, intra-tracheal, transdermal, or transmucosal. Actual methods for preparing parenterally administrable compositions will be known or apparent to those skilled in the art and are described in detail. Bai, J. Neuroimmunol. 80: 65-75, 1997. Warren, J. Neurol. Sci. 152: 31-38, 1997. Tonegawa, J. Exp. Med. 186: 507-515, 1997. When administering the compositions by injection, the administration may be by continuous infusion or by single or multiple bolus injections.

In certain embodiments, an antibody of the present invention or fragment thereof may be linked to a half-life extending vehicle known in the art. Such vehicles include, but are not limited to, the Fc domain, polyethylene glycol (PEG), and dextran. Such vehicles are described, e.g., in U.S. Application No. 09/428,082 and WO 99/25044.

The present antibody or fragment thereof may be glyco-modified (e.g., deglycosylated, etc.) with improved effector function profile.

Pharmaceutical techniques may also be employed to control the duration of action of the compositions/preparations of the present invention. Control release preparations may be prepared through the use of polymers to complex, encapsulate, or absorb the antibodies. WO1999040939. In certain embodiments, the composition takes the form of, for example, implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. See, e.g., Sustained and Controlled Release Drug Delivery Systems, J. R. Robinson, ed., Marcel Dekker, Inc., New York, 1978.

The pharmaceutical compositions of the present invention may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. The liquid formulation may be administered directly. The lyophilized powder formulation may be reconstituted in a physiologically compatible medium before administration.

The compositions of the present invention can be prepared as injectables, either as liquid solutions or suspensions, or as solid forms which are suitable for solution or suspension in liquid vehicles prior to injection. The composition can also be prepared in solid form, emulsified or the active ingredient encapsulated in liposome vehicles or other particulate carriers used for sustained delivery. For example, the composition can be in the form of an oil emulsion, water-in-oil emulsion, water-in-oil-in-water emulsion, site-specific emulsion, long-residence emulsion, stickyemulsion, microemulsion, nanoemulsion, liposome, microparticle, microsphere, nanosphere, nanoparticle and various natural or synthetic polymers, such as nonresorbable impermeable polymers such as ethylenevinyl acetate copolymers and Hytrel® copolymers, swellable polymers such as hydrogels, or resorbable polymers such as collagen and certain polyacids or polyesters such as those used to make resorbable sutures, that allow for sustained release of the vaccine.

By way of example, intravenously injectable immunoglobulin preparations may contain an immununoglobulin distributed in a physiologically compatible medium. Suitable medium for the present compositions may be sterile water for injection (WFI) with or without isotonic amounts of sodium chloride. For example, diluents include sterile WFI, sodium chloride solution (see Gahart, B.L. & Nazareno, A.R., intravenous Medications: a handbook for nurses and allied health professionals, p. 516-521, Mosby, 1997).

The immunoglobulin concentration in the pharmaceutical composition may range from about 0.1% (w/w) to about 30% (w/w), from about 0.5% (w/w) to about 20% (w/w), from about 1% (w/w) to about 15% (w/w), from about 2% (w/w) to about 3% (w/w), or from about 5% (w/w) to about 10% (w/w).

In certain embodiments, the present composition may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation. Compositions for oral administration via tablet, capsule or suspension are prepared using adjuvants including sugars, such as lactose, glucose and sucrose; starches such as corn starch and potato starch; cellulose and derivatives thereof, including sodium carboxymethylcellulose, ethylcellulose and cellulose acetates; powdered tragancanth; malt; gelatin; talc; stearic acids; magnesium stearate; calcium sulfate; vegetable oils, such as peanut oils, cotton seed oil, sesame oil, olive oil and corn oil; polyols such as propylene glycol, glycerine, sorbital, mannitol and polyethylene glycol; agar; alginic acids; water; isotonic saline and phosphate buffer solutions. Wetting agents, lubricants such as sodium lauryl sulfate, stabilizers, tableting agents, anti-oxidants, preservatives, coloring agents and flavoring agents may also be present.

Creams, lotions and ointments may be prepared for topical application using an appropriate base such as a triglyceride base. Such creams, lotions and ointments may also contain a surface active agent. Aerosol formulations, for example, for nasal delivery, may also be prepared in which suitable propellant adjuvants are used. Other adjuvants may also be added to the composition regardless of how it is to be administered, for example, anti-microbial agents may be added to the composition to prevent microbial growth over prolonged storage periods. Therapeutic compositions typically must be sterile and stable under conditions of manufacture and storage.

The present antibodies or antigen-binding portions thereof are formulated into compositions for delivery to a mammalian subject. The composition is administered alone, and/or mixed with a pharmaceutically acceptable vehicle or exciplent. Suitable vehicles are, for example, water, saline, dextrose, glycerol, ethanol, or the like, and combinations thereof. In addition, the vehicle can contain minor amounts of auxiliary substances such as wetting or emulsifying agents, pH buffering agents, or adjuvants. The compositions of the present invention can also include ancillary substances, such as pharmacological agents, cytokines, or other biological response modifiers. Methods of preparing the formulations are known, or will be apparent, to those skilled in the art. See, e.g., Remington's Pharmaceutical Sciences, Mack Publishing Company, Easton, Pennsylvania, 21st edition.

The present antibodies or antigen-binding portion thereof can be combined with a pharmaceutically acceptable carrier. Pharmaceutically acceptable carriers can contain a physiologically acceptable compound that acts to, e.g., stabilize, or increase or decrease the absorption or clearance rates of the present antibodies or antigen-binding portion thereof. Physiologically acceptable compounds can include, e.g., carbohydrates, such as glucose, sucrose, or dextrans, antioxidants, such as ascorbic acid or glutathione, chelating agents, low molecular weight proteins, detergents, liposomal carriers, or excipients or other stabilizers and/or buffers. Other physiologically acceptable compounds include wetting agents, emulsifying agents, dispersing agents or preservatives. See e.g., Remington's Pharmaceutical Sciences, Mack Publishing Company, Easton, Pennsylvania, 21st edition.

In one aspect, the claimed antibodies or antigen-binding portion thereof are dissolved in a pharmaceutically acceptable carrier, e.g., an aqueous carrier. Examples of aqueous solutions include, e.g., water, saline, phosphate buffered saline, Hank's solution, Ringer's solution, dextrose/saline, glucose solutions and the like. The formulations can contain pharmaceutically acceptable auxiliary substances as required to approximate physiological conditions, such as buffering agents, tonicity adjusting agents, wetting agents, detergents and the like. Additives can also include additional active ingredients such as bactericidal agents, or stabilizers. For example, the solution can contain sodium acetate, sodium lactate, sodium chloride, potassium chloride, calcium chloride, sorbitan monolaurate or triethanolamine oleate.

Solid formulations can be used in the present invention. They can be formulated as, e.g., pills, tablets, powders or capsules. For solid compositions, conventional solid carriers can be used which include, e.g., mannitol, lactose, starch, magnesium stearate, sodium saccharin, talcum, cellulose, glucose, sucrose, magnesium carbonate, and the like. Suitable pharmaceutical excipients include e.g., starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol.

For transmucosal or transdermal administration, penetrants appropriate to the barrier to be permeated can be used in the formulation. Such penetrants are generally known in the art, and include, e.g., for transmucosal administration, bile salts and fusidic acid derivatives. In addition, detergents can be used to facilitate permeation. Transmucosal administration can be through nasal sprays or using suppositories. Sayani, Crit. Rev. Ther. Drug Carrier Syst. 13: 85-184, 1996. For topical, transdermal administration, the agents are formulated into ointments, creams, salves, powders and gels. Transdermal delivery systems can also include, e.g., patches.

For inhalation, the present compositions can be delivered using any system known in the art, including dry powder aerosols, liquids delivery systems, air jet nebulizers, propellant systems, and the like. Patton, Biotechniques 16: 141-143, 1998. Also can be used in the present invention are product and inhalation delivery systems for polypeptide macromolecules by, e.g., Dura Pharmaceuticals (San Diego, Calif.), Aradigrn (Hayward, Calif.), Aerogen (Santa Clara, Calif.), Inhale Therapeutic Systems (San Carlos, Calif.), and the like. For example, the pharmaceutical formulation can be administered in the form of an aerosol or mist. For aerosol administration, the formulation can be supplied in finely divided form along with a surfactant and propellant. In another aspect, the device for delivering the formulation to respiratory tissue is an inhaler in which the formulation vaporizes. Other liquid delivery systems include, e.g., air jet nebulizers.

The present compositions can also be administered in sustained delivery or sustained release mechanisms. For example, biodegradeable microspheres or capsules or other biodegradeable polymer configurations capable of sustained delivery of a peptide can be included in the formulations of the invention (see, e.g., Putney, Nat. Biotechnol. 16: 153-157, 1998).

In one aspect, the compositions are prepared with carriers that will protect the peptide against rapid elimination from the body, such as a controlled release formulation, including implants and microencapsulated delivery systems. Biodegradable, biocompatible polymers can be used, such as ethylene vinyl acetate, polyanhydrides, polygiycolic acid, collagen, polyorthoesters, and polylactic acid. Methods for preparation of such formulations will be apparent to those skilled in the art. Liposomal suspensions can also be used as pharmaceutically acceptable carriers. U.S. Patent No. 4,522,811.

In one aspect, the pharmaceutical formulations comprising nucleic acids, polypeptides, or antibodies of the invention are incorporated in lipid monolayers or bilayers, e.g., liposomes. U.S. Patent Nos. 6,110,490; 6,096,716; 5,283,185 and 5,279,833. Aspects of the invention also provide formulations in which nucleic acids, peptides or polypeptides of the invention have been attached to the surface of the monolayer or bilayer. For example, peptides can be attached to hydrazide-PEG-(distearoylphosphatidyl) ethanolamine-containing liposomes (see, e.g., Zalipsky, Bioconjug. Chem. 6: 705-708, 1995). Liposomes or any form of lipid membrane, such as planar lipid membranes or the cell membrane of an intact cell, e.g., a red blood cell, can be used. Liposomal formulations can be by any means, including administration intravenously, transdermally (see, e.g., Vutla, J. Pharm. Sci. 85: 5-8, 1996), transmucosally, or orally. The invention also provides pharmaceutical preparations in which the nucleic acid, peptides and/or polypeptides of the invention are incorporated within micelles and/or liposomes (see, e.g., Suntres, J. Pharm. Pharmacol. 46: 23-28, 1994; Woodle, Pharm. Res. 9: 260-265, 1992). Liposomes and liposomal formulations can be prepared according to standard methods and are also well known in the art. Akimaru, Cytokines Mol. Ther. 1: 197-210,1995. Alving, Immunol. Rev. 145: 5-31, 1995. Szoka, Ann. Rev. Biophys. Bioeng. 9: 467, 1980. U.S. Patent Nos. 4, 235,871; 4,501,728 and 4,837,028.

It is advantageous to formulate parenteral or oral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Also encompassed by the present disclosure is an article of manufacture comprising packaging material and a pharmaceutical composition. The composition comprises a pharmaceutically acceptable carrier and a pharmaceutically effective amount of the antibody or antigen-binding portion thereof as described above. The packaging material may be labeled to indicate that the composition is useful to treat or prevent a filovirus infection. The packaging material may be any suitable material generally used to package pharmaceutical agents including, for example, glass, plastic, foil and cardboard.

Additional components of the kits may include one or more of the following: instructions for use; another therapeutic agent, an agent useful for coupling an antibody to a label or therapeutic agent, other reagents, or other materials for preparing the antibody for administration; pharmaceutically acceptable carriers; and devices or other materials for administration to a subject.

Instructions for use can include instructions for therapeutic applications, suggested dosages, dose intervals, and/or modes of administration, etc. Other instructions can include instructions on coupling of the antibody to a label or a therapeutic agent, or for purification of a conjugated antibody, e.g., from unreacted conjugation components.

The kit may or may not contain at least one nucleic acid encoding the present antibodies or fragment thereof, and instructions for expression of the nucleic acids. Other possible components of the kit include expression vectors and cells.

The present antibodies, antigen-binding portions thereof, compositions and methods can be used in all vertebrates, e.g., mammals and non-mammals, including human, mice, rats, guinea pigs, hamsters, dogs, cats, cows, horses, goats, sheep, pigs, monkeys, apes, gorillas, chimpanzees, rabbits, ducks, geese, chickens, amphibians, reptiles and other animals. A subject may be a vertebrate, an experimental animal or a transgenic animal. The present compositions and methods may be for veterinary use.

The invention will be further clarified by the following examples, which are intended to be purely exemplary of the invention and in no way limiting.

### EXAMPLES

### EXAMPLE 1: Antigen preparation

*Marburgvirus* (MARV; Ravn and Angola strains) and *ebolavirus* glycoprotein (GP) antigens were produced by stable cell line expression in *Drosophila* S2 and *Spodoptera* Sf9, or by transient transfection in Gnt-/- HEK293 cells. Proteins were engineered with either a strep or HA tag at the C-terminus to facilitate purification using streptactin (Qiagen) or anti-HA affinity resin (Roche), respectively. GP ectodomain constructs (GPe) lack the transmembrane (TM) domain and consist of residues 1-637. GP ectodomain mucin-deleted constructs (GPeΔmuc) also lack the mucin-like domain: Δ257-425 for all MARV strains, Δ314-463 for EBOV (Ebola virus), SUDV (Sudan virus), BDBV (Bundibugyo virus) and Δ316-470 for RESTV (Reston virus). As a control for epitope-mapping experiments, an additional MARV GP construct was purified from S2 cells lacking both the GP1 mucin domain (Δ257-425) and the GP2-wing (Δ436-483), termed GPeΔmucΔw. GP and GP construct sequences are provided in Table 1. To mimic endosomal cathepsin protease cleavage, cleaved MARV GP (GPcl) was produced by incubation of MARV Ravn strain GPeΔmuc with trypsin at a ratio of 1:100 in TBS pH 7.5 at 37°C for 1 hour. Cleaved EBOV GP was produced by treatment with thermolysin at a ratio of 1:50 overnight at room temperature in TBS pH 7.5 containing 1mM CaCl₂. GPe proteins were further purified by Superose 6 and all other GP proteins were purified by Superdex 200 size exclusion chromatography. The GP schematic and construct design for the engineered peptides are shown in the diagram in Figure 1, and a summary of the GP constructs is shown in Table 2.

**Table 1: Amino acid and nucleotide sequences of antibody domains and antigens**

| **Antibody Name** | **Chain, Region** | **Origin** | **Sequence** | **SEQ ID NO** |
|---|---|---|---|---|
| CAN30G5 | K, Variable region | Murine sequence | gatattgtgctgacccaatctccactctccctgcctgtcagtcttgg agatcaagcctccatctcttgcagatctagtcagagccttgtaca cagtaatggaaacacctatttacattggtacctgcagaagccag gccagtctccaaacctcctgatctacaaagtttccaaccgattttct ggggtcccagacaggttcagtggcagtggatcagggacagatt tcacactcaagatcagcagagtggaggctgaggatctgggagt ttatttctgctctcaaagtacacatgttccgtggacgttcggtggag gcaccaagctggaaatcaaa | **1** |
| CAN30G5 | K, variable region | Murine sequence | DIVLTQSPLSLPVSLGDQASISCRSSQSLVHSN GNTYLHWYLQKPGQSPNLLIYKVSNRFSGVP DRFSGSGSGTDFTLKISRVEAEDLGVYFCSQS THVPWTFGGGTKLEIK | **2** |
| CAN30G5 | K, CDR1 | Murine sequence | cagagccttgtacacagtaatggaaacacctat | **3** |
| CAN30G5 | K, CDR2 | Murine sequence | aaagtttcc | **4** |
| CAN30G5 | K, CDR3 | Murine sequence | tctcaaagtacacatgttccgtggacg | **5** |
| CAN30G5 | K, CDR1 | Murine sequence | QSLVHSNGNTY | **6** |
| CAN30G5 | K, CDR2 | Murine sequence | KVS | **7** |
| CAN30G5 | K, CDR3 | Murine sequence | SQSTHVPWT | **8** |
| CAN30G5 | K, FR 1 | Murine sequence | gatattgtgctgacccaatctccactctccctgcctgtcagtcttgg agatcaagcctccatctcttgcagatctagt | **9** |
| CAN30G5 | K, FR2 | Murine sequence | ttacattggtacctgcagaagccaggccagtctccaaacctcctg atctac | **10** |
| CAN30G5 | K, FR3 | Murine sequence | aaccgattttctggggtcccagacaggttcagtggcagtggatca gggacagatttcacactcaagatcagcagagtggaggctgag gatctgggagtttatttctgc | **11** |
| CAN30G5 | K, FR4 | Murine sequence | ttcggtggaggcaccaagctggaaatcaaa | **12** |
| CAN30G5 | H, Variable region | Murine sequence | gaggtgcagcttgttgagtctggtggaggattggtgcagcctaaa ggatcattgaaactctcatgtgccgcctctggtttcgccttcaattcc tatgccatgcactgggtctgccaggctccaggaaagggtttgga atgggttgctcgcataagaattaaaagtggtaattatgcaacatct tatgccggttcagtgacagacagattcaccgtctccagagatgat tcacaaaacttgttctatctgcaaatgaacaacctgaaaactgag gacacagccatgtattactgtgtgagagagtgggaaggggctat ggactactggggtcaaggaacctcagtcaccgtctcctcag | **13** |
| CAN30G5 | H, variable region | Murine sequence | EVQLVESGGGLVQPKGSLKLSCAASGFAFNS YAMHWVCQAPGKGLEWVARIRIKSGNYATSY AGSVTDRFTVSRDDSQNLFYLQMNNLKTEDT | **14** |
| | | | AMYYCVREWEGAMDYWGQGTSVTVSS | |
| CAN30G5 | H, CDR1 | Murine sequence | ggtttcgccttcaattcctatgcc | **15** |
| CAN30G5 | H, CDR2 | Murine sequence | ataagaattaaaagtggtaattatgcaaca | **16** |
| CAN30G5 | H, CDR3 | Murine sequence | gtgagagagtgggaaggggctatggactac | **17** |
| CAN30G5 | H, CDR1 | Murine sequence | GFAFNSYA | **18** |
| CAN30G5 | H, CDR2 | Murine sequence | IRIKSGNYAT | **19** |
| CAN30G5 | H, CDR3 | Murine sequence | VREWEGAMDY | **20** |
| CAN30G5 | H, FR1 | Murine sequence | gaggtgcagcttgttgagtctggtggaggattggtgcagcctaaa ggatcattgaaactctcatgtgccgcctct | **21** |
| CAN30G5 | H, FR2 | Murine sequence | atgcactgggtctgccaggctccaggaaagggtttggaatgggt tgctcgc | **22** |
| CAN30G5 | H, FR3 | Murine sequence | tcttatgccggttcagtgacagacagattcaccgtctccagagat gattcacaaaacttgttctatctgcaaatgaacaacctgaaaact gaggacacagccatgtattactgt | **23** |
| CAN30G5 | H, FR4 | Murine sequence | tggggtcaaggaacctcagtcaccgtctcctcag | **24** |
| CAN54G2 | K, Variable region | Murine sequence | gatgttgtgatgacccagactcccctcactttgtcggttaccattgg acagccagcctccatctcttgcacgtcaagtcagagcctcttaaa tagtgatgggggagacatatttgaattggttgttacagaggccagg ccagtctccaaagcgcctaatccatctggtgtctaaactggactct ggagtccctgacaggatcactggcagtggatctgggacagattt cacactgaaaatcaacagagtggaggctgaggatttgggaattt attattgctggcaaggtacacattttccgtggacgttcggtggagg caccaagctggaaatcaaac | **25** |
| CAN54G2 | K, variable region | Murine sequence | DVVMTQTPLTLSVTIGQPASISCTSSQSLLNSD GETYLNWLLQRPGQSPKRLIHLVSKLDSGVPD RITGSGSGTDFTLKINRVEAEDLGIYYCWQGT HFPWTFGGGTKLEIK | **26** |
| CAN54G2 | K, CDR1 | Murine sequence | cagagcctcttaaatagtgatggggagacatat | **27** |
| CAN54G2 | K, CDR2 | Murine sequence | ctggtgtct | **28** |
| CAN54G2 | K, CDR3 | Murine sequence | tggcaaggtacacattttccgtggacg | **29** |
| CAN54G2 | K, CDR1 | Murine sequence | QSLLNSDGETY | **30** |
| CAN54G2 | K, CDR2 | Murine sequence | LVS | **31** |
| CAN54G2 | K, CDR3 | Murine sequence | WQGTHFPWT | **32** |
| CAN54G2 | K, FR1 | Murine sequence | gatgttgtgatgacccagactcccctcactttgtcggttaccattgg | **33** |
| | | | acagccagcctccatctcttgcacgtcaagt | |
| CAN54G2 | K, FR2 | Murine sequence | ttgaattggttgttacagaggccaggccagtctccaaagcgccta atccat | **34** |
| CAN54G2 | K, FR3 | Murine sequence | aaactggactctggagtccctgacaggatcactggcagtggatc tgggacagatttcacactgaaaatcaacagagtggaggctgag gatttgggaatttattattgc | **35** |
| CAN54G2 | K, FR4 | Murine sequence | ttcggtggaggcaccaagctggaaatcaaac | **36** |
| CAN54G2 | H, Variable region | Murine sequence | caggtccagttgcagcagtctggaactgagctggtaaggcctgg gacttcagtgaagatatcctgcaaggcttctggatacgacttcact aacttttggctaggttggataaagcagaggcctggacatggactt gaatggattggagatatttaccctggaggtgataatacttactaca atgagaagttcaagggcaaagtcacgctgactgcagacaaat cctcgaacacagcctatatgcagttcagtagcctgacatctgag gactctgctgtctatttctgttttatgattctctatactttggactactgg ggtcaaggaacctcagtcaccgtctcctcag | **37** |
| CAN54G2 | H, variable region | Murine sequence | QVQLQQSGTELVRPGTSVKISCKASGYDFTN FWLGWIKQRPGHGLEWIGDIYPGGDNTYYNE KFKGKVTLTADKSSNTAYMQFSSLTSEDSAVY FCFMILYTLDYWGQGTSVTVSS | **38** |
| CAN54G2 | H, CDR1 | Murine sequence | ggatacgacttcactaacttttgg | **39** |
| CAN54G2 | H, CDR2 | Murine sequence | atttaccctggaggtgataatact | **40** |
| CAN54G2 | H, CDR3 | Murine sequence | tttatgattctctatactttggactac | **41** |
| CAN54G2 | H, CDR1 | Murine sequence | GYDFTNFW | **42** |
| CAN54G2 | H, CDR2 | Murine sequence | IYPGGDNT | **43** |
| CAN54G2 | H, CDR3 | Murine sequence | FMILYTLDY | **44** |
| CAN54G2 | H, FR1 | Murine sequence | caggtccagttgcagcagtctggaactgagctggtaaggcctgg gacttcagtgaagatatcctgcaaggcttct | **45** |
| CAN54G2 | H, FR2 | Murine sequence | ctaggttggataaagcagaggcctggacatggacttgaatggat tggagat | **46** |
| CAN54G2 | H, FR3 | Murine sequence | tactacaatgagaagttcaagggcaaagtcacgctgactgcag acaaatcctcgaacacagcctatatgcagttcagtagcctgaca tctgaggactctgctgtctatttctgt | **47** |
| CAN54G2 | H, FR4 | Murine sequence | tggggtcaaggaacctcagtcaccgtctcctcag | **48** |
| CAN30G2 | K, Variable region | Murine sequence | gatgttttgatgacccaaactccactctccctgcctgtcagtcttgg agatcaagcctccatctcttgcagatctagtcagagcattgtacat agtaatggagacacctatttagaatggtacctgcagaaaccag gccagtctccaaagctcctgatctacaaagtttccaaccgattttct ggggtcccagacaggttcagtggcagtggatcagggacagatt tcacactcaggatcagcagagtggaggctgaggatctgggagt | **49** |
| | | | ttattactgctttcaaggttcacattttccgtggacgttcggtggagg caccaagctggaaatcaaac | |
| CAN30G2 | K, variable region | Murine sequence | DVLMTQTPLSLPVSLGDQASISCRSSQSIVHS NGDTYLEWYLQKPGQSPKLLIYKVSNRFSGV PDRFSGSGSGTDFTLRISRVEAEDLGVYYCFQ GSHFPWTFGGGTKLEIK | **50** |
| CAN30G2 | K, CDR1 | Murine sequence | cagagcattgtacatagtaatggagacacctat | **51** |
| CAN30G2 | K, CDR2 | Murine sequence | aaagtttcc | **52** |
| CAN30G2 | K, CDR3 | Murine sequence | tttcaaggttcacattttccgtggacg | **53** |
| CAN30G2 | K, CDR1 | Murine sequence | QSIVHSNGDTY | **54** |
| CAN30G2 | K, CDR2 | Murine sequence | KVS | **55** |
| CAN30G2 | K, CDR3 | Murine sequence | FQGSHFPWT | **56** |
| CAN30G2 | K, FR1 | Murine sequence | gatgttttgatgacccaaactccactctccctgcctgtcagtcttgg agatcaagcctccatctcttgcagatctagt | **57** |
| CAN30G2 | K, FR2 | Murine sequence | ttagaatggtacctgcagaaaccaggccagtctccaaagctcct gatctac | **58** |
| CAN30G2 | K, FR3 | Murine sequence | aaccgattttctggggtcccagacaggttcagtggcagtggatca gggacagatttcacactcaggatcagcagagtggaggctgag gatctgggagtttattactgc | **59** |
| CAN30G2 | K, FR4 | Murine sequence | ttcggtggaggcaccaagctggaaatcaaac | **60** |
| CAN30G2 | H, Variable region | Murine sequence | gatgtgcagctggtggagtctgggggaggcttagtgcagcctgg agggtcccggaaactctcctgtacagcctctggattcactttcagt agctttggaatgcactgggttcgtcaggctccagagaaggggct ggagtgggtcgcatacattagtagtggcagtagtaaaatctacta tgcagacacggtgaagggccgattcaccatctccagagacaat cccaagaacaccctgttcctgcaaatgaccagtctaaggtctga ggacacggccatgtattactgtgcaagagggtggtacgagggg gcctggtttgcttactggggccaagggactctggtcactgtctctg cag | **61** |
| CAN30G2 | H, variable region | Murine sequence | DVQLVESGGGLVQPGGSRKLSCTASGFTFSS FGMHWVRQAPEKGLEWVAYISSGSSKIYYAD TVKGRFTISRDNPKNTLFLQMTSLRSEDTAMY YCARGWYEGAWFAYWGQGTLVTVSA | **62** |
| CAN30G2 | H, CDR1 | Murine sequence | ggattcactttcagtagctttgga | **63** |
| CAN30G2 | H, CDR2 | Murine sequence | attagtagtggcagtagtaaaatc | **64** |
| CAN30G2 | H, CDR3 | Murine sequence | gcaagagggtggtacgagggggcctggtttgcttac | **65** |
| CAN30G2 | H, CDR1 | Murine sequence | GFTFSSFG | **66** |
| CAN30G2 | H, CDR2 | Murine sequence | ISSGSSKI | **67** |
| CAN30G2 | H, CDR3 | Murine sequence | ARGWYEGAWFAY | **68** |
| CAN30G2 | H, FR1 | Murine sequence | gatgtgcagctggtggagtctgggggaggcttagtgcagcctgg agggtcccggaaactctcctgtacagcctct | **69** |
| CAN30G2 | H, FR2 | Murine sequence | atgcactgggttcgtcaggctccagagaaggggctggagtggg tcgcatac | **70** |
| CAN30G2 | H, FR3 | Murine sequence | tactatgcagacacggtgaagggccgattcaccatctccagag acaatcccaagaacaccctgttcctgcaaatgaccagtctaag gtctgaggacacggccatgtattactgt | **71** |
| CAN30G2 | H, FR4 | Murine sequence | tggggccaagggactctggtcactgtctctgcag | **72** |
| CAN40G1 | K, Variable region | Murine sequence | gacattgtgctgacccaatctccagcttctttggctgtgtctctagg gcagagggcctccatctcctgcaaggccagccaaagtgttgat catgatggtgatagttatatgaactggtaccaacagaaaccagg acagccacccaaactcctcatctatgctacatccaatctagaatc tgggatcccagccaggtttagtggcagtgggtctgggacagact tcaccctcaacatccatcctgtggaggaggaggatgctgcaac ctattactgtcagcagagttatgaggttccgctcacgttcggtgctg ggaccaagctggagctgaaac | **73** |
| CAN40G1 | K, variable region | Murine sequence | DIVLTQSPASLAVSLGQRASISCKASQSVDHD GDSYMNWYQQKPGQPPKLLIYATSNLESGIP ARFSGSGSGTDFTLNIHPVEEEDAATYYCQQ SYEVPLTFGAGTKLELK | **74** |
| CAN40G1 | K, CDR1 | Murine sequence | caaagtgttgatcatgatggtgatagttat | **75** |
| CAN40G1 | K, CDR2 | Murine sequence | gctacatcc | **76** |
| CAN40G1 | K, CDR3 | Murine sequence | cagcagagttatgaggttccgctcacg | **77** |
| CAN40G1 | K, CDR1 | Murine sequence | QSVDHDGDSY | **78** |
| CAN40G1 | K, CDR2 | Murine sequence | ATS | **79** |
| CAN40G1 | K, CDR3 | Murine sequence | QQSYEVPLT | **80** |
| CAN40G1 | K, FR1 | Murine sequence | gacattgtgctgacccaatctccagcttctttggctgtgtctctagg gcagagggccctccatctcctgcaaggccagc | **81** |
| CAN40G1 | K, FR2 | Murine sequence | atgaactggtaccaacagaaaccaggacagccacccaaact cctcatctat | **82** |
| CAN40G1 | K, FR3 | Murine sequence | aatctagaatctgggatcccagccaggtttagtggcagtgggtct gggacagacttcaccctcaacatccatcctgtggaggaggagg atgctgcaacctattactgt | **83** |
| CAN40G1 | K, FR4 | Murine sequence | ttcggtgctgggaccaagctggagctgaaac | **84** |
| CAN40G1 | H, Variable region | Murine sequence | gaggtccagctgcaacagtctggacctgagctggtgaagcctg gggcttcagtgaagatatcctgcaggacttctggatacacattca ctgaatacaccattcactgggtgaagcagagccgtggaaaga gccttgagtggattggaggtattaatcctaaccatggtggtactct ctacaaccagaagttcaaggtcaaggccacattgactgtagac aagtcctccagcacagcctacatggagctccgcagcctgacat ctgaggattctgcagtctattactgtgcaagatttacttacgactact ggggccaaggcaccactctcacagtctcctcag | **85** |
| CAN40G1 | H, variable region | Murine sequence | EVQLQQSGPELVKPGASVKISCRTSGYTFTEY TIHWVKQSRGKSLEWIGGINPNHGGTLYNQK FKVKATLTVDKSSSTAYMELRSLTSEDSAVYY CARFTYDYWGQGTTLTVSS | **86** |
| CAN40G1 | H, CDR1 | Murine sequence | ggatacacattcactgaatacacc | **87** |
| CAN40G1 | H, CDR2 | Murine sequence | attaatcctaaccatggtggtact | **88** |
| CAN40G1 | H, CDR3 | Murine sequence | gcaagatttacttacgactac | **89** |
| CAN40G1 | H, CDR1 | Murine sequence | GYTFTEYT | **90** |
| CAN40G1 | H, CDR2 | Murine sequence | INPNHGGT | **91** |
| CAN40G1 | H, CDR3 | Murine sequence | ARFTYDY | **92** |
| CAN40G1 | H, FR1 | Murine sequence | gaggtccagctgcaacagtctggacctgagctggtgaagcctg gggcttcagtgaagatatcctgcaggacttct | **93** |
| CAN40G1 | H, FR2 | Murine sequence | attcactgggtgaagcagagccgtggaaagagccttgagtgga ttggaggt | **94** |
| CAN40G1 | H, FR3 | Murine sequence | ctctacaaccagaagttcaaggtcaaggccacattgactgtaga caagtcctccagcacagcctacatggagctccgcagcctgaca tctgaggattctgcagtctattactgt | **95** |
| CAN40G1 | H, FR4 | Murine sequence | tggggccaaggcaccactctcacagtctcctcag | **96** |
| CAN30G1 | K, Variable region | Murine sequence | gaaaatgttctcacccagtctccagcaatcatgtctgcatctccag ggaaaaggtcaccatgacctgcagtgccagctcaagtgtaac ttacatgcactggtaccagcagaagtcaagcacctcccccaaa ctctggatttatgacacatccaaactggcttctggagtcccaggtc gcttcagtggcagtgggtctggaaactcttactctctcacgatcag cagcatggaggctgaagatgttgccacttattactgttttcagggg agtgggtacccgtacacgttcggaggggggaccaagctggaa ataaaac | **97** |
| CAN30G1 | K, variable region | Murine sequence | ENVLTQSPAIMSASPGEKVTMTCSASSSVTY MHWYQQKSSTSPKLWIYDTSKLASGVPGRFS GSGSGNSYSLTISSMEAEDVATYYCFQGSGY PYTFGGGTKLEIK | **98** |
| CAN30G1 | K, CDR1 | Murine sequence | gccagctcaagtgtaacttac | **99** |
| CAN30G1 | K, CDR2 | Murine sequence | gacacatcc | **100** |
| CAN30G1 | K, CDR3 | Murine sequence | tttcaggggagtgggtacccgtacacg | **101** |
| CAN30G1 | K, CDR1 | Murine sequence | ASSSVTY | **102** |
| CAN30G1 | K, CDR2 | Murine sequence | DTS | **103** |
| CAN30G1 | K, CDR3 | Murine sequence | FQGSGYPYT | **104** |
| CAN30G1 | K, FR1 | Murine sequence | gaaaatgttctcacccagtctccagcaatcatgtctgcatctccag gggaaaaggtcaccatgacctgcagt | **105** |
| CAN30G1 | K, FR2 | Murine sequence | atgcactggtaccagcagaagtcaagcacctcccccaaactct ggatttat | **106** |
| CAN30G1 | K, FR3 | Murine sequence | aaactggcttctggagtcccaggtcgcttcagtggcagtgggtct ggaaactcttactctctcacgatcagcagcatggaggctgaaga tgttgccacttattactgt | **107** |
| CAN30G1 | K, FR4 | Murine sequence | ttcggaggggggaccaagctggaaataaaac | **108** |
| CAN30G1 | H, Variable region | Murine sequence | cagatccagttggtgcagtctggacctgagctgaagaagcctgg agagacagtcaagatctcctgcaaggcttctgggtataccttcac aaactatggaatgaactgggtgaagcaggctccaggaaaggg tttaaagtggatgggctggataaacacctacactggaaagcca acatatgttgatgacttcaagggacggtttgccttctctttggaaac ctctgccaacactgcctatttgcagatcaacaacctcaaaaatg aggacacggctacatatttctgtgaaagtggaggttacgacgag gactactggggccaaggcaccactctcacagtctcctcag | **109** |
| CAN30G1 | H, variable region | Murine sequence | QIQLVQSGPELKKPGETVKISCKASGYTFTNY GMNWVKQAPGKGLKWMGWINTYTGKPTYVD DFKGRFAFSLETSANTAYLQINNLKNEDTATY FCESGGYDEDYWGQGTTLTVSS | **110** |
| CAN30G1 | H, CDR1 | Murine sequence | gggtataccttcacaaactatgga | **111** |
| CAN30G1 | H, CDR2 | Murine sequence | ataaacacctacactggaaagcca | **112** |
| CAN30G1 | H, CDR3 | Murine sequence | gaaagtggaggttacgacgaggactac | **113** |
| CAN30G1 | H, CDR1 | Murine sequence | GYTFTNYG | **114** |
| CAN30G1 | H, CDR2 | Murine sequence | INTYTGKP | **115** |
| CAN30G1 | H, CDR3 | Murine sequence | ESGGYDEDY | **116** |
| CAN30G1 | H, FR1 | Murine sequence | cagatccagttggtgcagtctggacctgagctgaagaagcctgg agagacagtcaagatctcctgcaaggcttct | **117** |
| CAN30G1 | H, FR2 | Murine sequence | atgaactgggtgaagcaggctccaggaaagggtttaaagtgga tgggctgg | **118** |
| CAN30G1 | H, FR3 | Murine sequence | acatatgttgatgacttcaagggacggtttgccttctctttggaaac ctctgccaacactgcctatttgcagatcaacaacctcaaaaatg aggacacggctacatatttctgt | **119** |
| CAN30G1 | H, FR4 | Murine sequence | tggggccaaggcaccactctcacagtctcctcag | **120** |
| CAN30G3 | K, Variable region | Murine sequence | gatgttttgatgacccaaactccactctccctgcctgtcagtcttgg agatcaagcctccatctcttgcagatctagtcagaacattgtacat agtaatggaaacacctatttagaatggtacctgcagaaatcagg ccagtctccaaagctcctgatctacaaagtttccaaccgattttctg gggtcccagacaggttcagtggcagtggatcagggacagattt cacactcaagatcagcagagtggaggctgaggatctgggagtt tattactgctttcaaggttcacattttccgtggacgttcggtggaggc accaagctggaaatcaaac | **121** |
| CAN30G3 | K, variable region | Murine sequence | DVLMTQTPLSLPVSLGDQASISCRSSQNIVHS NGNTYLEWYLQKSGQSPKLLIYKVSNRFSGV PDRFSGSGSGTDFTLKISRVEAEDLGVYYCFQ GSHFPWTFGGGTKLEIK | **122** |
| CAN30G3 | K, CDR1 | Murine sequence | cagaacattgtacatagtaatggaaacacctat | **123** |
| CAN30G3 | K, CDR2 | Murine sequence | aaagtttcc | **124** |
| CAN30G3 | K, CDR3 | Murine sequence | tttcaaggttcacattttccgtggacg | **125** |
| CAN30G3 | K, CDR1 | Murine sequence | QNIVHSNGNTY | **126** |
| CAN30G3 | K, CDR2 | Murine sequence | KVS | **127** |
| CAN30G3 | K, CDR3 | Murine sequence | FQGSHFPWT | **128** |
| CAN30G3 | K, FR1 | Murine sequence | gatgttttgatgacccaaactccactctccctgcctgtcagtcttgg agatcaagcctccatctcttgcagatctagt | **129** |
| CAN30G3 | K, FR2 | Murine sequence | ttagaatggtacctgcagaaatcaggccagtctccaaagctcct gatctac | **130** |
| CAN30G3 | K, FR3 | Murine sequence | aaccgattttctggggtcccagacaggttcagtggcagtggatca gggacagatttcacactcaagatcagcagagtggaggctgag gatctgggagtttattactgc | **131** |
| CAN30G3 | K, FR4 | Murine sequence | ttcggtggaggcaccaagctggaaatcaaac | **132** |
| CAN30G3 | H, Variable region | Murine sequence | caggtgcagctgaaggagtcaggacctggcctggtggcgccct cacagagcctgtccatcacatgcactgtctcagggttctccttaac cgactatggtataacctggattcgccagcctccaggaaagggtc tggagtggctgggagtaatatggggtggtggaagcgcatactat aatttagttctcaaatccagactgagcatcagcaaggacaactc caagagtcaagttttcttaaaaatgaacagtctgcaaactgatga cacagccatgtactactgtgccaaacatcggactttgattacgac tgctatggactactggggtcaaggaatttcagtcaccgtctcctca g | **133** |
| CAN30G3 | H, variable region | Murine sequence | QVQLKESGPGLVAPSQSLSITCTVSGFSLTDY GITWIRQPPGKGLEWLGVIWGGGSAYYNLVL KSRLSISKDNSKSQVFLKMNSLQTDDTAMYYC AKHRTLITTAMDYWGQGISVTVSS | **134** |
| CAN30G3 | H, CDR1 | Murine sequence | gggttctccttaaccgactatggt | **135** |
| CAN30G3 | H, CDR2 | Murine sequence | atatggggtggtggaagcgca | **136** |
| CAN30G3 | H, CDR3 | Murine sequence | gccaaacatcggactttgattacgactgctatggactac | **137** |
| CAN30G3 | H, CDR1 | Murine sequence | GFSLTDYG | **138** |
| CAN30G3 | H, CDR2 | Murine sequence | IWGGGSA | **139** |
| CAN30G3 | H, CDR3 | Murine sequence | AKHRTLITTAMDY | **140** |
| CAN30G3 | H, FR1 | Murine sequence | caggtgcagctgaaggagtcaggacctggcctggtggcgccct cacagagcctgtccatcacatgcactgtctca | **141** |
| CAN30G3 | H, FR2 | Murine sequence | ataacctggattcgccagcctccaggaaagggtctggagtggct gggagta | **142** |
| CAN30G3 | H, FR3 | Murine sequence | tactataatttagttctcaaatccagactgagcatcagcaaggac aactccaagagtcaagttttcttaaaaatgaacagtctgcaaact gatgacacagccatgtactactgt | **143** |
| CAN30G3 | H, FR4 | Murine sequence | tggggtcaaggaatttcagtcaccgtctcctcag | **144** |
| CAN30G4 | K, Variable region | Murine sequence | gatgttttgatgacccaaactccactctccctgcctgtcagtcttgg agatcaagcctccatctcttgcagatctagtcagaacattgtacat agtgatggaaacacctatttagaatggtacctgcagaaaccag gccagtctccaaagctcctgatctacaaattttccaaccgattttct ggggtcccagacaggttcagtggcagtggatcagggacagatt tcacactcaagatcagcagagtggaggctgaggatctgggagt ttattactgctttcaaggttcacatgttcctcccacgttcggtgctgg gaccaagctggagctgaaac | **145** |
| CAN30G4 | K, variable region | Murine sequence | DVLMTQTPLSLPVSLGDQASISCRSSQNIVHS DGNTYLEWYLQKPGQSPKLLIYKFSNRFSGVP DRFSGSGSGTDFTLKISRVEAEDLGVYYCFQ GSHVPPTFGAGTKLELK | **146** |
| CAN30G4 | K, CDR1 | Murine sequence | cagaacattgtacatagtgatggaaacacctat | **147** |
| CAN30G4 | K, CDR2 | Murine sequence | aaattttcc | **148** |
| CAN30G4 | K, CDR3 | Murine sequence | tttcaaggttcacatgttoctcccacg | **149** |
| CAN30G4 | K, CDR1 | Murine sequence | QNIVHSDGNTY | **150** |
| CAN30G4 | K, CDR2 | Murine sequence | KFS | **151** |
| CAN30G4 | K, CDR3 | Murine sequence | FQGSHVPPT | **152** |
| CAN30G4 | K, FR1 | Murine sequence | gatgttttgatgacccaaactccactctccctgcctgtcagtcttgg agatcaagcctccatctcttgcagatctagt | **153** |
| CAN30G4 | K, FR2 | Murine sequence | ttagaatggtacctgcagaaaccaggccagtctccaaagctcct gatctac | **154** |
| CAN30G4 | K, FR3 | Murine sequence | aaccgattttctggggtcccagacaggttcagtggcagtggatca gggacagatttcacactcaagatcagcagagtggaggctgag gatctgggagtttattactgc | **155** |
| CAN30G4 | K, FR4 | Murine sequence | ttcggtgctgggaccaagctggagctgaaac | **156** |
| CAN30G4 | H, Variable region | Murine sequence | gaagtgaagctggtggagtctgggggaggcttagtgaagtctg gagggtccctgaaactctcctgtgcagtctctggattcactttcagt acctatgccatgtcttgggttcgccagattccggagaagaggctg gagtgggtcgcaaccattagtaatggtggtagttatatctactattc agacagtgtgaagggtcgattcaccatctccagagacaatgcc aagaacaccctgtacctgcaaatgagcagtctgaggtctgagg acacggccatgtattactgtgcacgacatagggagtcctataggt acgactggtttgcttactggggccaagggactctggtcactgtctc tgcag | **157** |
| CAN30G4 | H, variable region | Murine sequence | EVKLVESGGGLVKSGGSLKLSCAVSGFTFSTY AMSWVRQIPEKRLEWVATISNGGSYIYYSDSV KGRFTISRDNAKNTLYLQMSSLRSEDTAMYYC ARHRESYRYDWFAYWGQGTLVTVSA | **158** |
| CAN30G4 | H, CDR1 | Murine sequence | ggattcactttcagtacctatgcc | **159** |
| CAN30G4 | H, CDR2 | Murine sequence | attagtaatggtggtagttatatc | **160** |
| CAN30G4 | H, CDR3 | Murine sequence | gcacgacatagggagtcctataggtacgactggtttgcttac | **161** |
| CAN30G4 | H, CDR1 | Murine sequence | GFTFSTYA | **162** |
| CAN30G4 | H, CDR2 | Murine sequence | ISNGGSYI | **163** |
| CAN30G4 | H, CDR3 | Murine sequence | ARHRESYRYDWFAY | **164** |
| CAN30G4 | H, FR1 | Murine sequence | gaagtgaagctggtggagtctgggggaggcttagtgaagtctg gagggtccctgaaactctcctgtgcagtctct | **165** |
| CAN30G4 | H, FR2 | Murine sequence | atgtcttgggttcgccagattccggagaagaggctggagtgggt cgcaacc | **166** |
| CAN30G4 | H, FR3 | Murine sequence | tactattcagacacagtgtgaagggtcgattcaccatctccagagac aatgccaagaacaccctgtacctgcaaatgagcagtctgaggt ctgaggacacggccatgtattactgt | **167** |
| CAN30G4 | H, FR4 | Murine sequence | tggggccaagggactctggtcactgtctctgcag | **168** |
| Ravn GPeΔmuc | GPeΔmucΔTM | Synthetic Marburg virus | MKTIYFLISLILIQSIKTLPVLEIASNSQPQDVDS | **169** |
| | | | VCSGTLQKTEDVHLMGFTLSGQKVADSPLEA SKRWAFRTGVPPKNVEYTEGEEAKTCYNISV TDPSGKSLLLDPPSNIRDYPKCKTVHHIQGQN PHAQGIALHLWGAFFLYDRVASTTMYRGKVF TEGNIAAMIVNKTVHRMIFSRQGQGYRHMNLT STNKYWTSSNETQRNDTGCFGILQEYNSTNN QTCPPSLKPPSLPTVTPSIHSTNTQINTAKSGT MRPPIYFRKKRSIFWKEGDIFPFLDGLINTEIDF DPIPNTETIFDESPSFNTSTNEEQHTPPNISLTF SYFPDKNGDTAYSGENENDCDAELRIWSVQE DDLAAGLSWIPFFGPGIEGLYTAGLIKNQNNLV CRLRRLANQTAKSLELLLRVTTEERTFSLINRH AIDFLLTRWGGTCKVLGPDCCIGIEDLSKNISE QIDKIRKDEQKEET | |
| Angola GPeΔmuc | GPeΔmucΔTM | Synthetic Marburg virus | MKTTCLLISLILIQGVKTLPILEIASNIQPQNVDS VCSGTLQKTEDVHLMGFTLSGQKVADSPLEA SKRWAFRAGVPPKNVEYTEGEEAKTCYNISV TDPSGKSLLLDPPTNIRDYPKCKTIHHIQGQNP HAQGIALHLWGAFFLYDRIASTTMYRGKVFTE GNIAAMIVNKTVHKMIFSRQGQGYRHMNLTST NKYWTSSNGTQTNDTGCFGTLQEYNSTKNQ TCAPSKKPLPLPTAHPEVKLTSTSTDATKLNTT QHLVYFRRKRNILWREGDMFPFLDGLINAPID FDPVPNTKTIFDESSSSGASAEEDQHASPNIS LTLSYFPKVNENTAHSGENENDCDAELRIWSV QEDDLAAGLSWIPFFGPGIEGLYTAGLIKNQN NLVCRLRRLANQTAKSLELLLRVTTEERTFSLI NRHAIDFLLARWGGTCKVLGPDCCIGIEDLSR NISEQIDQIKKDEQKEGT | 170 |
| Musoke GPeΔmuc | GPeΔmucΔTM | Synthetic Marburg virus | MKTTCFLISLILIQGTKNLPILEIASNNQPQNVD SVCSGTLQKTEDVHLMGFTLSGQKVADSPLE ASKRWAFRTGVPPKNVEYTEGEEAKTCYNIS VTDPSGKSLLLDPPTNIRDYPKCKTIHHIQGQN PHAQGIALHLWGAFFLYDRIASTTMYRGKVFT EGNIAAMIVNKTVHKMIFSRQGQGYRHMNLTS TNKYWTSSNGTQTNDTGCFGALQEYNSTKN QTCAPSKIPPPLPTARPEIKLTSTPTDATKLNT TQHLVYFRRKRSILWREGDMFPFLDGLINAPI DFDPVPNTKTIFDESSSSGASAEEDQHASPNI SLTLSYFPNINENTAYSGENENDCDAELRIWS VQEDDLAAGLSWIPFFGPGIEGLYTAVLIKNQN NLVCRLRRLANQTAKSLELLLRVTTEERTFSLI NRHAIDFLLTRWGGTCKVLGPDCCIGIEDLSK NISEQIDQIKKDEQKEGT | 171 |
| Ci67 GPeΔmuc | GPeΔmucΔTM | Synthetic Marburg virus | MKTTCLFISLILIQGIKTLPILEIASNNQPQNVDS VCSGTLQKTEDVHLMGFTLSGQKVADSPLEA SKRWAFRTGVPPKNVEYTEGEEAKTCYNISV TDPSGKSLLLDPPTNIRDYPKCKTIHHIQGQNP HAQGIALHLWGAFFLYDRIASTTMYRGRVFTE GNIAAMIVNKTVHKMIFSRQGQGYRHMNLTST NKYWTSNNGTQTNDTGCFGALQEYNSTKNQ TCAPSKIPSPLPTARPEIKPTSTPTDATTLNTT QHLVYFRKKRSILWREGDMFPFLDGLINAPIDF DPVPNTKTIFDESSSSGASAEEDQHASPNISL TLSYFPNINENTAYSGENENDCDAELRIWSVQ EDDLAAGLSWIPFFGPGIEGLYTAGLIKNQNNL VCRLRRLANQTAKSLELLLRVTTEERTFSLINR HAIDFLLTRWGGTCKVLGPDCCIGIEDLSRNIS EQIDQIKKDEQKEGT | 172 |
| Ravn (GenBank Accession No. ACD13005.1) | GP | Marburg virus, Kenya 1987 | MKTIYFLISLILIQSIKTLPVLEIASNSQPQDVDS | 173 |
| | | | VCSGTLQKTEDVHLMGFTLSGQKVADSPLEA SKRWAFRTGVPPKNVEYTEGEEAKTCYNISV TDPSGKSLLLDPPSNIRDYPKCKTVHHIQGQN PHAQGIALHLWGAFFLYDRVASTTMYRGKVF TEGNIAAMIVNKTVHRMIFSRQGQGYRHMNLT STNKYWTSSNETQRNDTGCFGILQEYNSTNN QTCPPSLKPPSLPTVTPSIHSTNTQINTAKSGT MNPSSDDEDLMISGSGSGEQGPHTTLNVVTE QKQSSTILSTPSLHPSTSQHEQNSTNPSRHAV TEHNGTDPTTQPATLLNNTNTTPTYNTLKYN LSTPSPPTRNITNNDTQRELAESEQTNAQLNT TLDPTENPTTAQDTNSTTNIIMTTSDITSKHPT NSSPDSSPTTRPPIYFRKKRSIFWKEGDIFPFL DGLINTEIDFDPIPNTETIFDESPSFNTSTNEEQ HTPPNISLTFSYFPDKNGDTAYSGENENDCDA ELRIWSVQEDDLAAGLSWIPFFGPGIEGLYTA GLIKNQNNLVCRLRRLANQTAKSLELLLRVTTE ERTFSLINRHAIDFLLTRWGGTCKVLGPDCCIG IEDLSKNISEQIDKIRKDEQKEETGWGLGGKW WTSDWGVLTNLGILLLLSIAVLIALSCICRIFTKY IG | |
| Angola (GenBank Accession No. ABE27064.1) | GP | Marburg virus, Angola, Lake Victoria, 2005 | MKTTCLLISLILIQGVKTLPILEIASNIQPQNVDS VCSGTLQKTEDVHLMGFTLSGQKVADSPLEA SKRWAFRAGVPPKNVEYTEGEEAKTCYNISV TDPSGKSLLLDPPTNIRDYPKCKTIHHIQGQNP HAQGIALHLWGAFFLYDRIASTTMYRGKVFTE GNIAAMIVNKTVHKMIFSRQGQGYRHMNLTST NKYWTSSNGTQTNDTGCFGTLQEYNSTKNQ TCAPSKKPLPLPTAHPEVKLTSTSTDATKLNTT DPNSDDEDLTTSGSGSGEQEPYTTSDAATKQ GLSSTMPPTPSPQPSTPQQGGNNTNHSQGV VTEPGKTNTTAQPSMPPHNTTTISTNNTSKHN LSTPSVPIQNATNYNTQSTAPENEQTSAPSKT TLLPTENPTTAKSTNSTKSPTTTVPNTTNKYST SPSPTPNSTAQHLVYFRRKRNILWREGDMFP FLDGLINAPIDFDPVPNTKTIFDESSSSGASAE EDQHASPNISLTLSYFPKVNENTAHSGENEND CDAELRIWSVQEDDLAAGLSWIPFFGPGIEGL YTAGLIKNQNNLVCRLRRLANQTAKSLELLLR VTTEERTFSLINRHAIDFLLARWGGTCKVLGP DCCIGIEDLSRNISEQIDQIKKDEQKEGTGWGL GGKWWTSDWGVLTNLGILLLLSIAVLIALSCIC RIFTKYIG | 174 |
| Musoke (GenBank Accession No. CAA78117.1) | GP | Marburg virus, Kenya, 1980 | MKTTCFLISLILIQGTKNLPILEIASNNQPQNVD SVCSGTLQKTEDVHLMGFTLSGQKVADSPLE ASKRWAFRTGVPPKNVEYTEGEEAKTCYNIS VTDPSGKSLLLDPPTNIRDYPKCKTIHHIQGQN PHAQGIALHLWGAFFLYDRIASTTMYRGKVFT EGNIAAMIVNKTVHKMIFSRQGQGYRHMNLTS TNKYWTSSNGTQTNDTGCFGALQEYNSTKN QTCAPSKIPPPLPTARPEIKLTSTPTDATKLNT TDPSSDDEDLATSGSGSGEREPHTTSDAVTK QGLSSTMPPTPSPQPSTPQQGGNNTNHSQD AVTELDKNNTTAQPSMPPHNTTTISTNNTSKH NFSTLSAPLQNTTNDNTQSTITENEQTSAPSIT TLPPTGNPTTAKSTSSKKGPATTAPNTTNEHF TSPPPTPSSTAQHLVYFRRKRSILWREGDMF PFLDGLINAPIDFDPVPNTKTIFDESSSSGASA EEDQHASPNISLTLSYFPNINENTAYSGENEN DCDAELRIWSVQEDDLAAGLSWIPFFGPGIEG | 175 |
| | | | LYTAVLIKNQNNLVCRLRRLANQTAKSLELLLR VTTEERTFSLINRHAIDFLLTRWGGTCKVLGP DCCIGIEDLSKNISEQIDQIKKDEQKEGTGWGL GGKWWTSDWGVLTNLGILLLLSIAVLIALSCIC RIFTKYIG | |
| Ci67 (GenBank Accession No. ABS17558.1) | GP | Marburg virus, Lake Victoria | MKTTCLFISLILIQGIKTLPILEIASNNQPQNVDS VCSGTLQKTEDVHLMGFTLSGQKVADSPLEA SKRWAFRTGVPPKNVEYTEGEEAKTCYNISV TDPSGKSLLLDPPTNIRDYPKCKTIHHIQGQNP HAQGIALHLWGAFFLYDRIASTTMYRGRVFTE GNIAAMIVNKTVHKMIFSRQGQGYRHMNLTST NKYWTSNNGTQTNDTGCFGALQEYNSTKNQ TCAPSKIPSPLPTARPEIKPTSTPTDATTLNTT DPNNDDEDLITSGSGSGEQEPYTTSDAVTKQ GLSSTMPPTPSPQPSTPQQEGNNTDHSQGT VTEPNKTNTTAQPSMPPHNTTAISTNNTSKNN FSTLSVSLQNTTNYDTQSTATENEQTSAPSKT TLPPTGNLTTAKSTNNTKGPTTTAPNMTNGHL TSPSPTPNPTTQHLVYFRKKRSILWREGDMFP FLDGLINAPIDFDPVPNTKTIFDESSSSSGASAE EDQHASPNISLTLSYFPNINENTAYSGENEND CDAELRIWSVQEDDLAAGLSWIPFFGPGIEGL YTAGLIKNQNNLVCRLRRLANQTAKSLELLLR VTTEERTFSLINRHAIDFLLTRWGGTCKVLGP DCCIGIEDLSRNISEQIDQIKKDEQKEGTGWGL GGKWWTSDWGVLTNLGILLLLLSIAVLIALSCIC RIFTKYIG | 176 |
| Zaire Ebola virus Mayinga strain (GenBank Accession No. U23187.1) | GP | Ebola virus, 1976 | MGVTGILQLPRDRFKRTSFFLWVIILFQRTFSIP LGVIHNSTLQVSDVDKLVCRDKLSSTNQLRSV GLNLEGNGVATDVPSATKRWGFRSGVPPKVV NYEAGEWAENCYNLEIKKPDGSECLPAAPDGI RGFPRCRYVHKVSGTGPCAGDFAFHKEGAFF LYDRLASTVIYRGTTFAEGVVAFLILPQAKKDF FSSHPLREPVNATEDPSSGYYSTTIRYQATGF GTNETEYLFEVDNLTYVQLESRFTPQFLLQLN ETIYTSGKRSNTTGKLIWKVNPEIDTTIGEWAF WETKKNLTRKIRSEELSFTVVSNGAKNISGQS PARTSSDPGTNTTTEDHKIMASENSSAMVQV HSQGREAAVSHLTTLATISTSPQSLTTKPGPD NSTHNTPVYKLDISEATQVEQHHRRTDNDSTA SDTPSATTAAGPPKAENTNTSKSTDFLDPATT TSPQNHSETAGNNNTHHQDTGEESASSGKL GLITNTIAGVAGLITGGRRTRREAIVNAQPKCN PNLHYWTTQDEGAAIGLAWIPYFGPAAEGIYIE GLMHNQDGLICGLRQLANETTQALQLFLRATT ELRTFSILNRKAIDFLLQRWGGTCHILGPDCCI EPHDWTKNITDKIDQIIHDFVDKTLPDQGDND NWWTGWRQWIPAGIGVTGVIIAVIALFCICKFV F | 177 |
| Ebola Zaire 1995, P87666 | GP | Ebola virus, 1995 | MGVTGILQLPRDRFKRTSFFLWVIILFQRTFSIP LGVIHNSTLQVSDVDKLVCRDKLSSTNQLRSV GLNLEGNGVATDVPSATKRWGFRSGVPPKVV NYEAGEWAENCYNLEIKKPDGSECLPAAPDGI RGFPRCRYVHKVSGTGPCAGDFAFHKEGAFF LYDRLASTVIYRGTTFAEGVVAFLILPQAKKDF FSSHPLREPVNATEDPSSGYYSTTIRYQATGF GTNETEYLFEVDNLTYVQLESRFTPQFLLQLN ETIYTSGKRSNTTGKLIWKVNPEIDTTIGEWAF WETKKNLTRKIRSEELSFTAVSNRAKNISGQS PARTSSDPGTNTTTEDHKIMASENSSAMVQV | 178 |
| | | | HSQGREAAVSHLTTLATISTSPQPPTTKPGPD NSTHNTPVYKLDISEATQVEQHHRRTDNDSTA SDTPPATTAAGPLKAENTNTSKGTDLLDPATT TSPQNHSETAGNNNTHHQDTGEESASSGKL GLITNTIAGVAGLITGGRRARREAIVNAQPKCN PNLHYWTTQDEGAAIGLAWIPYFGPAAEGIYT EGLMHNQDGLICGLRQLANETTQALQLFLRAT TELRTFSILNRKAIDFLLQRWGGTCHILGPDCC IEPHDWTKNITDKIDQIIHDFVDKTLPDQGDND NWWTGWRQWIPAGIGVTGVIIAVIALFCICKFV F | |
| Ebola Sudan Maleo 1979 | GP | Sudan ebolavirus, 1979 | MEGLSLLQLPRDKFRKSSFFVWVIILFQKAFS MPLGVVTNSTLEVTEIDQLVCKDHLASTDQLK SVGLNLEGSGVSTDIPSATKRWGFRSGVPPQ VVSYEAGEWAENCYNLEIKKPDGSECLPPPP DGVRGFPRCRYVHKAQGTGPCPGDYAFHKD GAFFLYDRLASTVIYRGVNFAEGVIAFLILAKPK ETFLQSPPIREAANYTENTSSYYATSYLEYEIE NFGAQHSTTLFKINNNTFVLLDRPHTPQFLFQ LNDTIQLHQQLSNTTGKLIWTLDANINADIGEW AFWENKKNLSEQLRGEELSFETLSLNETEDD DATSSRTTKGRISDRATRKYSDLVPKDSPGM VSLHVPEGETTLPSQNSTEGRRVDVNTQETIT ETTATIIGTNGNNMQISTIGTGLSSSQILSSSPT MAPSPETQTSTTYTPKLPVMTTEEPTTPPRNS PGSTTEAPTLTTPENITTAVKTVWAQESTSNG LITSTVTGILGSLGLRKRSRRQVNTRATGKCN PNLHYWTAQEQHNAAGIAWIPYFGPGAEGIYT EGLMHNQNALVCGLRQLANETTQALQLFLRA TTELRTYTILNRKAIDFLLRRWGGTCRILGPDC CIEPHDWTKNITDKINQIIHDFIDNPLPNQDNDD NWWTGWRQWIPAGIGITGIIIAIIALLCVCKLLC | 179 |
| Makona Ebola virus (GenBank Accession No. AJF38895.1) | Virion spike GP precursor | Zaire ebolavirus | MGVTGILQLPRDRFKRTSFFLWVIILFQRTFSIP LGVIHNSTLQVSDVDKLVCRDKLSSTNQLRSV GLNLEGNGVATDVPSVTKRWGFRSGVPPKVV NYEAGEWAENCYNLEIKKPDGSECLPAAPDGI RGFPRCRYVHKVSGTGPCAGDFAFHKEGAFF LYDRLASTVIYRGTTFAEGVVAFLILPQAKKDF FSSHPLREPVNATEDPSSGYYSTTIRYQATGF GTNETEYLFEVDNLTYVQLESRFTPQFLLQLN ETIYASGKRSNTTGKLIWKVNPEIDTTIGEWAF WETKKNLTRKIRSEELSFTAVSNGPKNISGQS PARTSSDPETNTTNEDHKIMASENSSAMVQV HSQGRKAAVSHLTTLATISTSPQPPTTKTGPD NSTHNTPVYKLDISEATQVGQHHRRADNDST ASDTPPATTAAGPLKAENTNTSKSADSLDLAT TTSPQNYSETAGNNNTHHQDTGEESASSGKL GLITNTIAGVAGLITGGRRTRREVIVNAQPKCN PNLHYWTTQDEGAAIGLAWIPYFGPAAEGIYT EGLMHNQDGLICGLRQLANETTQALQLFLRAT TELRTFSILNRKAIDFLLQRWGGTCHILGPDCC IEPHDWTKNITDKIDQIIHDFVDKTLPDQGDND NWWTGWRQWIPAGIGVTGVIIAVIALFCICKFV F | 180 |
| Zaire Ebola virus (GenBank Accession No. AAM76034) | Surface GP precursor | Zaire ebolavirus, 1976 | MGVTGILQLPRDRFKRTSFFLWVIILFQRTFSIP LGVIHNSTLQVSDVDKLVCRDKLSSTNQLRPV GLNLEGNGVATDVPSATKRWGFRSGVPPKVV NYEAGEWAENCYNLEIKKPDGSECLPAAPDGI RGFPRCRYVHKVSGTGPCAGDFAFHKEGAFF LYDRLASTVIYRGTTFAEGVVAFLILPQAKKDF | 181 |
| | | | FSSHPLREPVNATEDPSSGYYSTTIRYQATGF GTNETEYLFEVDNLTYVQLEPRFTPQFLLQLN ETIYTSGKRSNTTGKLIWKVNPEIDTTIGEWAF WETKKNLTRKIRSEELSFTVVSNTHHQDTGEE SASSGKLGLITNTIAGVAGLITGGRRTRREAIV NAQPKCNPNLHYWTTQDEGAAIGLAWIPYFG PAAEGIYTEGLMHNQDGLICGLRQLANETTQA LQLFLRATTELRTFSILNRKAIDFLLQRWGGTC HILGPDCCIEPHDWTKNITDKIDQIIHDFVDKTL PD | |
| Makona Ebola virus (GenBank Accession No. AAQ55048.1) | Virion spike GP precursor | Zaire ebolavirus | MGVTGILQLPRDRFKRTSFFLWVIILFQRTFSIP LGVIHNSTLQVSEVDKLVCRDKLSSTNQLRSV GLNLEGNGVATDVPSATKRWGFRSGVPPKVV NYEAGEWAENCYNLEIKKPDGSECLPAAPDGI RGFPRCRYVHKVSGTGPCAGDFAFHKEGAFF LYDRLASTVIYRGTTFAEGVVAFLILPQAKKDF FSSHPLREPVNATEDPSSGYYSTTIRYQATGF GTNETEYLFEVDNLTYVQLESRFTPQFLLQLN ETIYTSGKRSNTTGKLIWKVNPEIDTTIGEWAF WETKKNLTRKIRSEELSFTAVSNTHHQDTGEE SASSGKLGLITNTIAGVAGLITGGRRARREAIV NAQPKCNPNLHYWTTQDEGAAIGLAWIPYFG PAAEGIYTEGLMHNQDGLICGLRQLANETTQA LQLFLRATTELRTFSILNRKAIDFLLQRWGGTC HILGPDCCIEPHDWTKNITDKIDQIIHDFVDKTL PD | 182 |
| Sudan ebolavirus (GenBank Accession No. AGL73446) | GP | Sudan ebolavirus | MGGLSLLQLPRDKFRKSSFFVWVIILFQKAFS MPLGVVTNSTLEVTEIDQLVCKDHLASTDQLK SVGLNLEGSGVSTDIPSATKRWGFRSGVPPK VVSYEAGEWAENCYNLEIKKPDGSECLPPPP DGVRGFPRCRYVHKAQGTGPCPGDYAFHKD GAFFLYDRLASTVIYRGVNFAEGVIAFLILAKPK ETFLQSPPIREAVNYTENTSSYYATSYLEYEIE NFGAQHSTTLFKIDNNTFVRLDRPHTPQFLFQ LNDTIHLHQQLSNTTGRLIWTLDANINADIGEW AFWENKKNLSEQLRGEELSFEALSNITTAVKT VLPQESTSNGLITSTVTGILGSLGLRKRSRRQT NTKATGKCNPNLHYWTAQEQHNAAGIAWIPY FGPGAEGIYTEGLMHNQNALVCGLRQLANET TQALQLFLRATTELRTYTILNRKAIDFLLRRWG GTCRILGPDCCIEPHDWTKNITDKINQIIHDFID NPLPN | 183 |

In Table 1, the CDRs are IMGT numbering. H: heavy chain; K: kappa chain.

### EXAMPLE 2: Production of GP-Fab complex

Ravn GPeΔmuc (SEQ ID NO:169) was produced in *Drosophila* S2 cells, purified by streptactin affinity via a C-terminal strep tag, and the trimeric portion isolated on a Superdex 200 sizing column. A CAN30G4 Fab fragment was generated by standard papain digestion of the CAN30G4 IgG antibody (sequences of the CAN30G4 antibody are provided in Table 1) and purified by Mono Q ion-exchange chromatography. Five molar excess Fab was added to the trimeric GPeΔmuc and allowed to bind overnight at 4°C. The complex created was referred to as CAN30G4 Fab-Ravn GPeΔmuc, which was then used to immunize mice (see Example 3). A diagram of the antigen-antibody complex is shown in Figure 12 (but in the absence of a chemical cross-linking reagent).

Figure 13 shows crystals of Marburg virus GP (Ravn) in complex with Fab fragments from mAb CAN54G1. This complex nucleates crystals in multiple conditions.

### EXAMPLE 3: Derivation and screening of monoclonal antibodies binding to Marburg virus (MARV)

Monoclonal antibodies (mAbs) to Marburg virus (MARV) were generated by immunizing mice to produce an antibody directed to an epitope in the glycoprotein (GP) of MARV. Immunization of mice was performed according to standard operating procedures. Six week-old female BALB/c mice (University of Manitoba, using Animal Use Protocols approved by the Protocol Management and Review Committee) were injected subcutaneously (SC) with 20 µg of inert MARV Ravn GPeΔmuc (SEQ ID NO:169) or MARV Angola GPeΔmuc (SEQ ID NO:170) in Freund's Complete Adjuvant (CFA) (Brenntag Biosector) on day 1. On day 32 the mice received 20 µg of the same MARV GP injected intraperitoneally (I.P.) in Incomplete Freund's Adjuvant (IFA) (Brenntag Biosector) in a total volume of 100 µl. On day 56, the mice received 20 µg of the same antigen in a total volume of 100 µl I.P. with IFA. Serum analysis from test bleeds at this point showed specific serum IgG titers to MARV GP (data not shown). Mice received 1-2 boosters of recombinant MARV GP protein (10 µg in IFA I.P.) prior to a final push of 5 µg purified GP (in PBS by IP) before conducting fusions. Standard protocols were used to produce hybridoma cell lines, and monoclonal antibodies were purified on Protein G resin. Mouse sera were screened on Ravn GPeΔmuc, and spleen fusion and hybridoma screening was performed on those mice with specific and strong interaction with Ravn GP. Spleens were harvested three days after the final push and mice were euthanized by anesthesia overdose and exsanguinated by cardiac puncture. The spleens were subsequently excised under aseptic conditions and cell fusions performed essentially according to standard techniques. Positive selected IgG-secreting clones were subjected to large-scale production and subsequent characterization by immunological methods. Isotyping was performed using a commercial murine isotyping dipstick test (Roche) according to the manufacturer's instructions. Hybridoma culture supernatants were concentrated 5-10 fold using Amicon stirred cell nitrogen concentrators with 30 kDa cutoff Millipore (YM-30) membranes (both from Millipore, Billerica, MA).Mice immunized with Angola GPeΔmuc purified from S2 cells yielded antibody 40G1. Mice immunized with Ravn GPeΔmuc purified from Gnt -/- HEK293 cells raised antibodies 30G1, 30G3, 30G4 and 30G5. Mice immunized with Ravn GPeΔmuc, and then boosted with a complex of Ravn GPeΔmuc bound to 30G4 Fab (CAN30G4 Fab-Ravn GPeΔmuc), raised antibodies 54G1, 54G2 and 54G3 as presented in Table 4.

**Table 2: Summary of the MARV and EBOV GP constructs**

| Construct | Description* | Deleted Regions* |
|---|---|---|
| MARV GP | Full length GP (1-681) | |
| MARV GPe | Deleted transmembrane (TM) domain | 638-681 (TM) |
| MARV GPeΔmuc | Deleted TM & mucin-like domain (muc) | 257-425 (TM) |
| | | 638-681 (muc) |
| MARV GPeΔmucΔw | Deleted TM, muc & GP2 wing (w) | 257-425 (TM) |
| | | 638-681 (muc) |
| | | 436-483 (w) |
| MARV GPcl | Cleaved GP with deleted TM & muc | 257-425 (TM) |
| | | 638-681 (muc) |
| EBOV GP | Full length (1-676) | |
| EBOV GPe | Deleted TM domain | 638-676 |
| EBOV GPeΔmuc - EBOV, SUDV, BDBV | Deleted TM & mucin-like domain (muc) | 314-463 |
| EBOV GPeΔmuc - RESTV | Deleted TM & mucin-like domain (muc) | 316-470 |

| | | |
|---|---|---|
| * Numbering of the amino acids begins at the first amino acid of the MARV or EBOV GP signal sequence, as indicated on Figure 1. | | |

Antibodies were screened via ELISA method against either Ravn GP or Angola GP. Briefly, 96-well MaxiSorp plates (NUNC) were coated with 200 ng/well of antigen, covered and incubated overnight at 4°C. Plates were washed 5X in Milli-Q water to remove any unbound antigen and then blocked with Blocking Buffer (5% Skim Milk Powder (SMP) in Phosphate Buffered Saline (PBS)). Plates were incubated for 1 hour at 37°C and then washed 5X in Milli-Q water. Plates were then coated with hybridoma supernatant and serially diluted 2-fold in Dilution Buffer (2.5% SMP in PBS) starting at 1 µg/mL. After a 1 hour incubation period at 37°C, plates were then washed 5X in Milli-Q water. Goat anti-Mouse IgG-HRP was then added to the plate at a 1:2000 dilution in Dilution Buffer and incubated again for 1 hour at 37°C. Plates were then washed and substrate added to the plates. Plates were read using 405nm wavelength after 15 minutes, 30 minutes or 1 hour (incubation at room temperature).

From this immunization and screening, a panel of 27 antibodies was raised against Marburg Ravn GP and 1 against the Marburg Angola GP (Table 3).

**Table 3: List of anti-Marburg GP antibodies generated**

| **mAb ID** | **Isotype** | **Specificity** |
|---|---|---|
| CAN30G1-2-2 | IgG1/kappa | Binds Ravn GP1 subunit |
| CAN30G2-2-1 | IgG1/kappa | Reactive with HA purification tag and not GP |
| CAN30G3-2-3 | IgG1/kappa | Largely specific for Ravn GP |
| CAN30G4-1-2 | IgG1/kappa | Marburg virus cross-reactive, directed against GP2 and binds both GPΔmucΔtm and GP2 |
| CAN30G5-1-1 | IgG1/kappa | Binds both GPΔmucΔtm and GP2 from Ravn |
| CAN30G6-1-2 | IgG1/kappa | Specific for GPΔmucΔtm (does not bind GP2 expressed alone), is specific for Ravn |
| CAN54G1-1 | IgG1/kappa | Binds to Ravn GP1/2 |
| CAN54G2-2 | IgG1/kappa | Binds epitope on Ravn GP2 |
| CAN54G3-2 | IgG1/kappa | Binds epitope on Ravn GP2 |
| CAN61G1-2 | IgG2a/kappa | Binds to both Ravn GP1/2 (stronger to GP1) |
| CAN61 G2-1 | IgG1/kappa | Binds to both Ravn GP1/2 |
| CAN61 G3-2 | IgG1/kappa | Binds to both Ravn GP1/2 |
| CAN61 G4-1 | IgG1/kappa | Binds to Ravn GP1 |
| CAN61 G5-1 | IgG1/kappa | Binds to both Ravn GP1/2 |
| CAN61G6-3 | IgG1/kappa | Binds to both Ravn GP1/2 (stronger to GP1) |
| CAN61G7-1 | IgG1/kappa | Binds to Ravn GP2 |
| CAN61 G8-1 | IgG1/kappa | Binds to Ravn GP1 |
| CAN62G1-3 | IgG1/kappa | Recognizes Ravn GP1/2 |
| CAN62G2-1 | IgG1/kappa | Recognizes Ravn GP1/2 (stronger to GP1) |
| CAN62G5-3 | IgG2a/kappa | Recognizes Ravn GP1/2 (stronger to GP2) |
| CAN62G6-1 | | Binds to Ravn GP2 |
| CAN62G7-1 | IgG2b/kappa | Recognizes Ravn GP1/2 |
| CAN63G1-1 | IgG2a/kappa | Binds to Ravn GP2 subunit |
| CAN63G2-2 | IgG2a/kappa | Binds to Ravn GP2 subunit |
| CAN63G3-1 | IgG1/kappa | Likely binds to conformational epitope on Ravn GP |
| CAN63G4-1 | IgG1/kappa | Binds to Ravn GP1/2 |
| CAN63G7-3 | IgG3/kappa | Likely binds to conformational epitope on Ravn GP |
| CAN63G8-2 | IgG1/kappa | Binds to Ravn GP1 subunit |
| | | |
| CAN40G1 | IgG1/kappa | Binds to Angola GP |

A list of immunogens and boosting immunogens used for selected anti-Marburg GP mAbs is provided in Table 4.

**Table 4: List of selected anti-Marburg GP antibodies generated**

| **mAb ID** | **Immunogen** | **Boosting Immunogen** | **Isotype** |
|---|---|---|---|
| CAN30G1 | Ravn GPeΔmuc | Ravn GPeΔmuc | IgG1/kappa |
| CAN30G2 | Ravn GPeΔmuc | Ravn GPeΔmuc | IgG1/kappa |
| CAN30G3 | Ravn GPeΔmuc | Ravn GPeΔmuc | IgG1/kappa |
| CAN30G4 | Ravn GPeΔmuc | Ravn GPeΔmuc | IgG1/kappa |
| CAN30G5 | Ravn GPeΔmuc | Ravn GPeΔmuc | IgG1/kappa |
| CAN54G1 | Ravn GPeΔmuc | Ravn GPeΔmuc-CAN30G4 Fab complex | IgG1/kappa |
| CAN54G2 | Ravn GPeΔmuc | Ravn GPeΔmuc-CAN30G4 Fab complex | IgG1/kappa |
| CAN54G3 | Ravn GPeΔmuc | Ravn GPeΔmuc-CAN30G4 Fab complex | IgG1/kappa |
| CAN40G1 | Angola GPeΔmuc | Angola GPeΔmuc | IgG1/kappa |

### EXAMPLE 4: ELISA testing of mouse anti-Marburg GP monoclonal antibodies

An ELISA was performed to test the binding of the mAbs against multiple strains of Marburg GP, GPe, GPeΔmuc, and to determine if the mAbs are cross-reactive to various strains of Ebola virus (EBOV) GP, GPe and GpeΔmuc. The ELISA plate was coated with 200 ng/well of antigen. The wells were blocked with 5% skim milk then probed with serially diluted generated mAbs starting (0.1 µg/mL to 1 µg/mL). Binding was detected with commercial goat anti-Mouse IgG-HRP. The plate was read at 405nm after a minimum of 15 minutes incubation with substrate.

The CAN30, CAN54 and CAN40 series mAbs were all tested for binding to different strains of MARV (Musoke, Ci67, Angola, and Ravn) and EBOV engineered GPs (GPe, GPeΔmuc and GPcl). Figure 2 lists the results in table form. The results show the binding of all antibodies to MARV GPeΔmuc and GPcl in multiple Marburg strains. CAN30G1, CAN30G4, CAN40G1 and CAN54G3 show binding to the GPe of all MARV strains tested. All other mAbs tested only showed binding to MARV Ravn GPe. CAN30G1, CAN40G1, CAN54G1 and CAN54G3 showed binding to MARV Ravn GPeΔmucΔw. CAN40G1 was the only cross-reactive mAb, showing binding to EBOV GPeΔmuc and EBOV GPcl.

Figure 3 shows the ELISA results when using the CAN30 series mAbs to test binding to Marburg virus Ravn GPeΔmuc, Angola GPeΔmuc and Popp GPeΔmuc. As shown in Figure 3, all CAN30 mAbs, except for CAN30G6, showed binding to Ravn GpeΔmuc, Angola GPeΔmuc and Popp GPeΔmuc.

CAN40G1 (anti-MARV Angola mAb) was tested for cross-reactivity to various MARV strains and ebolaviruses. To determine cross-reactivity, ELISAs were performed using the GPeΔmuc of the Ravn, Angola, Popp, and Musoke strains of MARV, the GPeΔmuc of the ebolaviruses EBOV, SUDV, and BDBV, or the cleaved MARV and EBOV GPs (GPcl) as coating antigens. CAN40G1 was further evaluated for binding to the complete, mucin-containing ectodomain of MARV Angola, EBOV, SUDV, and BDBV and to the secreted sGP of EBOV and RESTV. As shown in Figures 4, 5A and 5B, CAN40G1binds to MARV GP and mucin-deleted GP from multiple MARV strains. CAN40G1 is also cross-reactive to EBOV GP as well as EBOV GPeΔmuc.

For animals boosted with CAN30G4 Fab-RavnGPeΔmuc and the CAN54 mAbs that were generated from hybridoma fusions, cross reactivity was directed towards GP1 and GP2 eptitopes that were unique in comparison to CAN40G1 immunized with the Angola strain GPΔmuc. The CAN54 mAbs were also directed away from the immunodominant GP2 wing elicited by immunizations with RavnGPeΔmuc (e.g. CAN30 fusions), however, one mAb clone did result in immunorecognition towards the GP2 wing elicted by several CAN30 mAbs, but specificity against the GPs across the Marburg strains was unique in relation to GPe and GPeΔmuc.

### EXAMPLE 5: Western blots performed with mouse anti-Marburg GP monoclonal antibodies

A 4-12% gradient SDS-PAGE gel is run for 1.5 hours at 200 volts with a combination of MARV and EBOV proteins. The gel is then transferred to a nitrocellulose membrane for a minimum of 1 hour at 45 volts. The membrane is blocked overnight at 4°C with 5% skim milk in 1xTBST. The next day the mAbs (1° Ab) are diluted in 2.5% skim milk in 1xTBST at concentrations ranging from 2 µg/mL to 5 µg/mL depending on the antibody and used to probe the membrane containing the transferred proteins for 2 hours at room temperature (RT). The membranes are then washed with 1xTBST to remove unbound 1° Ab and probed with anti-mouse IgG-HRP (2° Ab) at a dilution of 1:4000 to 1:5000 for 1.5 hours at RT.

### EXAMPLE 6: Pseudovirus neutralization assay performed with mouse anti-Marburg GP monoclonal antibodies

Antibodies were tested for neutralization of recombinant Vesicular Stomatitis Virus (VSV) pseudotyped with MARV GP. VSV pseudovirions containing a GFP gene in place of the VSV G gene (VSVAG) and bearing the glycoprotein of MARV Ravn were generated as previously described (Takeda et al. Proc Natl Acad Sci USA, 1997. 94(26): 14764-14769). Experiments were performed in triplicate with VSVΔG bearing either full-length MARV Ravn GP (VSVΔG-GP) or mucin-deleted Δ257-425 GP (VSVΔG-GPΔmuc). Pseudovirions were incubated with anti-VSV G mAb for 1 hour at RT, then incubated with 2.5, 10 or 50 µg/mL of each anti-MARV GP mAb in DMEM-10%FBS for an additional hour. Pseudovirion/mAb complexes were added to Vero cells at a multiplicity of infection (MOI) of 0.01. After 48 hours, infection was evaluated by counting GFP-expressing cells.

As shown in Figure 6A, mAbs CAN30G4 and CAN30G5 could suppress infectivity down to 20% or less against mucin-deleted MARV GP. In contrast, polyclonal sera obtained at time of exsanguination from immunized mice (Grp30polyAb) neutralized only slightly better (approximately 10% infectivity remained).

The best neutralizing mAbs in this panel neutralized mucin-containing Marburg virus GP-pseudotypes approximately 50%, while polyclonal sera neutralized the same viruses to approximately 40% infectivity (Figure 6B). One possible explanation for why the mucin-deleted virus was easier to neutralize than the mucin-containing virus is that the large mucin-like domain could restrict access to antibody epitopes on GP, as has been suggested for Ebola virus. Recent studies in the field suggest that a cocktail of monoclonal antibodies offers better *in vivo* protection against Ebola virus than individual mAbs alone, and hence, combinations of some of these mAbs may improve upon neutralization capacity over any single mAb alone. mAbs CAN30G3, CAN30G5, CAN54G1 and CAN54G2 neutralized mucin containing (full length) viruses approximately 50% (Figure 6B). In both examples where antibodies were directed away from the GP2 wing (CAN54G1 and CAN54G3), neutralization was on par with other antibodies directed towards GP1 and GP2 (e.g. CAN30G1 and CAN40G1).

### EXAMPLE 7: Epitope mapping with pin peptides

Pin peptides were designed to cover the GP1 and GP2 subunits of Marburg Musoke GP (NCBI Accession number NC_001608) and Marburg Ravn GP (NCBI Accession number AB_04Y1906) by designing 15mers overlapping by 10 amino acids and removing the mucin domain and transmembrane domain along with the signal peptide sequence and cytoplasmic tail (Feldmann et al., 2001, J Gen Virol. 82(Pt 12):2839-2848; Will et al., 1993, J Virol. 67(3):1203-1210). MARV-Angola and MARV-Musoke are approximately 93% identical in GP protein sequence, and MARV and RAW are approximately 78% identical in GP protein sequence. Because of the similarity between Angola and Musoke, Musoke and Ravn pins were designed. Internal cysteines were replaced by methionine to prevent dimerization of peptide with conserved substitution.

For the assay, pins were activated by rinsing in methanol for a few seconds and allowed to air-dry. Pins were then blocked with 200 µL of Blocking Buffer (1% SMP + 1% Tween-20 in PBS) in 96-well round bottom plates and incubated for 2 hours at RT. Pins were then washed with Wash Solution (0.9% w/v NaCl + 0.05% Tween-20 in PBS) 3X for ∼1 min/wash. Pins were then immediately coated with 100 µL of a 1/5 dilution of supernatant in Dilution Buffer (0.1% SMP + 0.1% Tween-20 in PBS) in new 96-well round bottom plates and left covered overnight at 4°C. The next day, pins were washed 3X in wash solution and then incubated at room temperature for 1 hour in a 1:5000 dilution of Goat anti-mouse IgG-HRP in dilution buffer with 100 µL/well. After incubation, pins were washed 3x in wash solution. ABTS substrate was then applied at 200 µL/well to 96-well flat-bottom MaxiSorp plates and readings taken at 15 minutes, 30 minutes and 1 hour.

Table 5 shows the results of epitope mapping. Figures 7A, 7B, 7C and 7D show the schematics of the MARV GP protein and the epitopes for CAN30G3, CAN30G4, CAN30G5 and CAN54G2. Although the results indicate CAN30G3, CAN30G4, CAN30G5 and CAN54G2 bind to the GP2 subunit of MARV along an overlapping epitope, specificity is modulated more specifically towards the N-terminal residues. These sutle differences are reflected in specificity across the different Marburg strains (Fig 2, 6a and 6b). CAN40G1 may bind a conformational epitope. CAN54G1 is shown to bind to GP2 but in a region outside of the N-terminal residues bound by the other GP2 specific antibodies.

**Table 5: Pin peptide mapping of mAbs to Marburg Ravn GP and Marburg Angola GP**

| **mAb ID** | **Marburg pins** | **GP subunit** | **Pins** | **Absorbance (1 hour)** | **Peptide Sequence** |
|---|---|---|---|---|---|
| CAN30G1 | Ravn | N/A | N/A | N/A | unreactive to pins (tested 2x), likely recognizes conforma tional epitope |
| CAN30G2 | Ravn | GP2 | D4 | 0.848 | LINTEIDFDPIPNTE (SEQ ID NO:184) |
| | | | D5 | 0.373 | |
| | | | | | IDFDPIPNTETIFDE (SEQ ID NO:185) |
| CAN30G3 | Ravn | GP2 | | | IDFDPIPNTETIFDE (SEQ ID NO:186) |
| | | | | | |
| | | | D5 | 1.840 | IPNTETIFDESPSFN (SEQ ID NO:187) |
| | | | D6 | 1.116 | |
| CAN30G4 | Ravn | GP2 | | | PNLDGLINTEIDFDP (SEQ ID NO:188) |
| | | | | | LINTEIDFDPIPNTE (SEQ ID NO:189) |
| | | | D3 | 0.836 | |
| | | | D4 | Overflow* | IDFDPIPNTETIFDE (SEQ ID NO:190) |
| | | | D5 | 1.966 | |
| CAN30G5 | Ravn | GP2 | | | LINTEIDFDPIPNTE (SEQ ID NO:191) |
| | | | | | IDFDPIPNTETIFDE (SEQ ID NO:192) |
| | | | D4 | 0.631 | |
| | | | D5 | Overflow | IPNTETIFDESPSEN |
| | | | D6 | Overflow | (SEQ ID NO:193) |
| CAN30G6 | Ravn | GP1/GP 2 | Multiple | N/A | Likely binds conformational epitope, or repeat sequence |
| CAN54G1 | Ravn | GP1/GP 2 | Multiple | N/A | Likely binds conformational epitope, or repeat sequence |
| CAN54G2 | Ravn | GP2 | D5 | | LINTEIDFDPIPNTE (SEQ ID NO:194) |
| | | | D6 | | |
| | | | | 2.757 | IDFDPIPNTETIFDE |
| | | | | 1.849 | (SEQ ID NO:195) |
| CAN54G3 | Ravn | GP2 | | | LINTEIDFDPIPNTE (SEQ ID NO:196) |
| | | | D5 | 2.220 | |
| | | | D6 | 1.444 | |
| | | | | | IDFDPIPNTETIFDE (SEQ ID NO:197) |
| | | | | | |
| CAN40G1 | Ravn | N/A | N/A | N/A | unreactive to pins (tested 2x), likely recognizes conformational epitope |
| CAN40G1 | Musoke | GP1/GP 2 | Multiple | N/A | May recognize a repeat sequence or conformational epitope |

| | | | | | |
|---|---|---|---|---|---|
| *Note: Overflow value is greater than 3.9 | | | | | |

### EXAMPLE 8: Mouse in vivo protection experiments

All procedures with infectious marburgviruses were performed in biosafety level 4 (BSL-4) facilities. BALB/c mice were challenged intraperitoneally (IP) with 1000 plaque-forming units (p.f.u.) mouse-adapted MARV. One hour post-exposure, the mice were treated IP with 500 µg (0.5 ml of 1.0 mg/ml mAb in PBS solution) of purified monoclonal antibody or PBS alone. One study also included a negative control group treated with 500 µg of anti-HA IgG at 1.0 mg/mL. Clinical signs for infection were monitored for 28 days post-exposure at which point the study ended, and mice were euthanized.

Two separate studies were performed. Table 6 shows the characteristics of the mAbs as well as the results from the in vivo protection studies. Figure 8 is a line graph showing the results from the two in vivo studies. CAN30G5 and CAN54G2 showed the greatest protection with 100% and 90% survival, respectively, in both studies. CAN30G3 and CAN30G4 showed greater than 50% protection, while CAN40G1 showed 40% protection. In this case, although CAN54G2 and CAN30G3 may be predicted to show equivalent efficacy based on epitope mapping (Example 7) and cross reactivity (Figure 2) there was a marked difference in vivo against Marburg Ravn challenge in mouse adapted model.

**Table 6: Summary of antibodies generated to Marburg Ravn GP, Marburg Angola GP and Marburg Musoke GP and their protective properties In vivo.**

| mAb | Cross Reactivity with GPeΔmuc | Epitope | VSVdG/GP Neutralization | Protection in BALB/c mice | |
|---|---|---|---|---|---|
| | | | | Study 1 | Study 2 |
| 30G1 | R, A, M, P | GP1 | 5% | | |
| 30G3 | R, A, M, P | N-term GP2 | 40% | 8/10 | 6/10 |
| 30G4 | R, A, M, P | 451-475 | 50% | 6/10 | |
| 30G5 | R, A, M, P | 456-475 | 50% | 10/10 | 10/10 |
| 40G1 | R, A, M, P, Ebov | GP1 | 0% | | 4/10 |
| 54G1 | R, M, P | C-term GP2 | 50% | 0/10 | |
| 54G2 | R, A, M, P | N-term GP2 | 40% | 9/10 | 9/10 |
| 54G3 | R, A, M, P | GP1 | 0% | 10/10 | 1/10 |
| 61G2 | R, A, M, P | GP1 | 20% | | 1/10 |
| 61G5 | R, A, M, P | unknown | 30% | | |

### EXAMPLE 9: V-gene sequencing

RNA was isolated from the parental hybridoma clonal cell line using RNeasy Mini Kit. The amplification of V genes from the RNA was performed using the Qiagen OneStep RT-PCR Kit. Several combinations of primer sets were used. The results of the PCR amplification reactions were determined by examining the PCR products on an analytical agarose gel, and the visualized bands at approximately 300-500bp were gel isolated for cloning. The extracted DNA was directly TA cloned into the pCR2.1-TOPO vector using the low melt agarose method in TOPO TA Cloning manual. The clone reactions were sequenced in both directions using the M13 Forward and M13 Reverse primers. Sequence data was analyzed using DNAStar Lasergene Software.

Figures 9-11 show the resulting arranged V-gene sequences compared to IMGT/V-Quest reference directory sets and to the NCBI immunoglobulin blast search for CAN30G5, CAN54G2 and CAN40G1. The figures include results for both the VH and VL sequences of the murine parental clone.

### EXAMPLE 10: Efficacy of panfilovirus mAb In in vivo Ebola virus infection model

The panfilovirus murine antibody CAN40G1 was tested in an *in vivo* Ebola virus infection model. BALB/c mice were separated into pans and moved into a BSL-4 facility three days before challenge. Mice were challenged i.p. with a targeted 1000 PFU mouse adapted Ebola virus (ma-EBOV). All groups, except for No Treatment group, were treated i.p. with 500 µL of pre-formulated mAb 1-2 hours post challenge and 3 days post-challenge. Mice were treated with a positive control anti-EBOV mAb; CAN40G1; or no antibody (No Treatment). Mice were monitored for signs of disease and weight was checked daily. Study was terminated on day 28 post challenge. The survival data for the groups at 10 days post challenge is shown in Table 7.

**Table 7: Percentage survival of mice challenged with ma-EBOV at 10 days post challenge**

| **Treatment group** | **Percent survival** |
|---|---|
| Positive Control anti-EBOV mAb | 100 |
| CAN40G1 | 20 |
| No Treatment | 0 |

## Claims

1. A monoclonal antibody, or an antigen-binding portion thereof, that specifically binds to a filovirus, wherein the antibody or antigen-binding portion comprises:
(a) an immunoglobulin light chain variable region comprising LCDR1, LCDR2, and LCDR3, and an immunoglobulin heavy chain variable region comprising HCDR1, HCDR2, and HCDR3, wherein the LCDR1, LCDR2 and LCDR3 comprise the amino acid sequences set forth in SEQ ID NOs: 78, 79 and 80, respectively, and the HCDR1, HCDR2, and HCDR3 comprise the amino acid sequences set forth in SEQ ID NOs: 90, 91 and 92, respectively; or
(b) an immunoglobulin light chain variable region comprising the amino acid sequence set forth in SEQ ID NO:2 and an immunoglobulin heavy chain variable region comprising the amino acid sequence set forth in SEQ ID NO:14; or
(c) an immunoglobulin light chain variable region comprising the amino acid sequence set forth in SEQ ID NO:26 and an immunoglobulin heavy chain variable region comprising the amino acid sequence set forth in SEQ ID NO:38.

2. The monoclonal antibody or antigen-binding portion of claim 1(a), wherein:
the immunoglobulin light chain variable region comprises the amino acid
sequence set forth in SEQ ID NO:74 ; or
the immunoglobulin heavy chain variable region comprises the amino acid sequence set forth in SEQ ID NO:86.

3. The monoclonal antibody or antigen-binding portion of claim 1(a) or 2, wherein:
the immunoglobulin light chain variable region comprises the amino acid sequence set forth in SEQ ID NO:74 and the immunoglobulin heavy chain variable region comprises the amino acid sequence set forth in SEQ ID NO:86.

4. The monoclonal antibody or antigen-binding portion of any one of claims 1(a), 2 or 3, wherein the monoclonal antibody or antigen-binding portion is selected from the group consisting of: (i) a whole immunoglobulin molecule; (b) an scFv; (c) a Fab fragment; (d) an F(ab')₂; and (e) a disulfide linked Fv; and/or
wherein the antibody comprises an immunoglobulin constant region selected from the group consisting of IgG1, IgG2, IgG3, IgG4, IgA1, IgA2, IgD, IgE and IgM.

5. The monoclonal antibody or antigen-binding portion of any one of the preceding claims, wherein the filovirus is a member of the *Marburgvirus* genus; preferably wherein the filovirus is selected from the group consisting of Ravn, Angola, Musoke, and Popp Marburg virus strains; and/or
wherein the monoclonal antibody or antigen-binding portion binds to the Marburg virus glycoprotein.

6. The monoclonal antibody or antigen-binding portion according to any one of claims 1(a) or 2-4:
wherein the monoclonal antibody or antigen-binding portion binds to the GP1 subunit of the Marburg virus glycoprotein; and/or
wherein the monoclonal antibody or antigen-binding portion is cross-reactive to at least two filoviruses; preferably wherein the monoclonal antibody or antigen-binding portion binds to a filovirus that is a member of the *Ebolavirus* genus; preferably wherein the monoclonal antibody or antigen-binding portion binds to the Ebola virus glycoprotein; or preferably wherein the monoclonal antibody or antigen-binding portion binds to at least one of Ebola virus, Sudan virus, Bundibugyo virus, Tai Forest virus or Reston virus.

7. The monoclonal antibody or antigen-binding portion of claim 1(b) or 1(c), wherein the monoclonal antibody or antigen-binding portion binds to the GP2 subunit of the Marburg virus glycoprotein.

8. A pharmaceutical composition comprising the monoclonal antibody or antigen-binding portion of any one of claims 1(a), 2-4 or 6 and at least one pharmaceutically acceptable adjuvant or at least one pharmaceutically acceptable carrier.

9. The pharmaceutical composition of claim 8, further comprising a second agent; preferably wherein the second agent is a monoclonal antibody or antigen-binding portion thereof; and/or
wherein the pharmaceutical composition comprises at least one Marburg virus-binding antibody or antigen-binding portion thereof, and at least one Ebola virus-binding antibody or antigen-binding protein thereof; and/or
wherein the pharmaceutical composition comprises at least two, at least three, or at least four Marburg virus-binding antibodies or antigen-binding portion thereof; and/or
wherein the pharmaceutical composition comprises at least two Marburg virus-binding antibodies or antigen-binding portion thereof, and wherein each Marburg virus-binding antibody or antigen-binding portion bind different epitopes of the Marburg virus glycoprotein.

10. A monoclonal antibody or antigen-binding portion of any one of claims 1(a), 2-4 or 6 for use in ameliorating, preventing or treating a filovirus infection in a subject in need thereof;
preferably wherein the filovirus infection is a Marburg virus infection or an Ebola virus infection.

11. A monoclonal antibody or antigen-binding portion of any one of claims 1(b), 1(c), 5 or 7 for use in ameliorating, preventing or treating a Marburg virus infection in a subject in need thereof.

12. An isolated nucleic acid molecule encoding the monoclonal antibody or antigen-binding portion of claim 1.

13. The isolated nucleic acid molecule of claim 12, wherein the nucleic acid molecule comprises one or more nucleotide sequence selected from the group consisting of SEQ ID NO:73, SEQ ID NO:75, SEQ ID NO:76, SEQ ID NO:77, SEQ ID NO:85, SEQ ID NO:87, SEQ ID NO:88, SEQ ID NO:89, SEQ ID NO:1, SEQ ID NO:13, SEQ ID NO: 202, SEQ ID NO:25, and SEQ ID NO:37.

14. An expression vector comprising a nucleic acid segment encoding the immunoglobulin light and heavy chain variable regions of the monoclonal antibody or antigen-binding portion of any one of claims 1(a), 2-4 or 6, wherein the nucleic acid segment is operatively linked to regulatory sequences suitable for expression of the nucleic acid segment in a host cell.

15. The expression vector according to claim 14, wherein the nucleic acid segment comprises one or more nucleotide sequences selected from the group consisting of SEQ ID NO:73, SEQ ID NO:75, SEQ ID NO:76, SEQ ID NO:77, SEQ ID NO:85, SEQ ID NO:87, SEQ ID NO:88, and SEQ ID NO:89.

16. A recombinant host cell comprising the expression vector of claim 14 or 15; preferably wherein the cell is a bacterial, eukaryotic or mammalian cell; more preferably wherein the cell is a COS-1, COS-7, HEK293, BHK21, CHO, BSC-1, HepG2, SP2/0, Hela, myeloma or lymphoma cell.

17. A method for producing a filovirus-binding monoclonal antibody or antigen-binding portion thereof, the method comprising
culturing a recombinant host cell comprising the expression vector of claim 14 or 15 under conditions whereby the nucleic acid segment is expressed, thereby producing the filovirus-binding monoclonal antibody or antigen-binding portion; preferably further comprising recovering the filovirus-binding monoclonal antibody or antigen-binding portion.

18. A method for detecting a filovirus glycoprotein in a sample, the method comprising contacting the sample with the antibody or antigen-binding portion of any one of claims 1(a), 2-4 or 6;
preferably wherein the sample is a cell, tissue, or biological fluid from a subject suspected of having or at risk of a filovirus infection; or preferably wherein the filovirus glycoprotein is a Marburg virus, Ebola virus, Sudan virus, Bundibugyo virus, Tai Forest virus or Reston virus glycoprotein.

## Patentansprüche

1. Monoklonaler Antikörper, oder Antigen-bindender Anteil davon, der spezifisch an ein Filovirus bindet, wobei der Antikörper oder Antigen-bindende Anteil Folgendes umfasst:
(a) eine variable Immunglobulin-Leichtkettenregion, umfassend LCDR1, LCDR2 und LCDR3, und eine variable Immunglobulin-Schwerkettenregion, umfassend HCDR1, HCDR2 und HCDR3, wobei die LCDR1, LCDR2 und LCDR3 die in SEQ ID NOs: 78, 79 bzw. 80 dargelegten Aminosäuresequenzen umfassen, und die HCDR1, HCDR2 und HCDR3 die in SEQ ID NOs: 90, 91 bzw. 92 dargelegten Aminosäuresequenzen umfassen; oder
(b) eine variable Immunglobulin-Leichtkettenregion, umfassend die in SEQ ID NO: 2 dargelegte Aminosäuresequenz und eine variable Immunglobulin-Schwerkettenregion, umfassend die in SEQ ID NO: 14 dargelegte Aminosäuresequenz; oder
(c) eine variable Immunglobulin-Leichtkettenregion, umfassend die in SEQ ID NO: 26 dargelegte Aminosäuresequenz und eine variable Immunglobulin-Schwerkettenregion, umfassend die in SEQ ID NO: 38 dargelegte Aminosäuresequenz.

2. Monoklonaler Antikörper oder Antigen-bindender Anteil nach Anspruch 1(a), wobei:
die variable Immunglobulin-Leichtkettenregion die in SEQ ID NO: 74 dargelegte Aminosäuresequenz umfasst; oder
die variable Immunglobulin-Schwerkettenregion die in SEQ ID NO: 86 dargelegte Aminosäuresequenz umfasst.

3. Monoklonaler Antikörper oder Antigen-bindender Anteil nach Anspruch 1(a) oder 2, wobei:
die variable Immunglobulin-Leichtkettenregion die in SEQ ID NO: 74 dargelegte Aminosäuresequenz umfasst und die variable Immunglobulin-Schwerkettenregion die in SEQ ID NO: 86 dargelegte Aminosäuresequenz umfasst.

4. Monoklonaler Antikörper oder Antigen-bindender Anteil nach einem der Ansprüche 1(a), 2 oder 3, wobei der monoklonale Antikörper oder Antigen-bindende Anteil aus der Gruppe ausgewählt ist, bestehend aus: (i) einem vollständigen Immunglobulinmolekül; (b) einem scFv; (c) einem Fab-Fragment; (d) einem F(ab')₂; und (e) einem Disulfid-verknüpften Fv; und/oder
wobei der Antikörper eine konstante Immunglobulinregion umfasst, ausgewählt aus der Gruppe, bestehend aus IgG1, IgG2, IgG3, IgG4, IgA1, IgA2, IgD, IgE und IgM.

5. Monoklonaler Antikörper oder Antigen-bindender Anteil nach einem der vorangehenden Ansprüche, wobei das Filovirus ein Mitglied der *Marburgvirus-*Gattung ist; bevorzugt wobei das Filovirus aus der Gruppe ausgewählt ist, bestehend aus den Marburgvirus-Stämmen Ravn, Angola, Musoke und Popp; und/oder
wobei der monoklonale Antikörper oder Antigen-bindende Anteil an das Marburgvirus-Glykoprotein bindet.

6. Monoklonaler Antikörper oder Antigen-bindender Anteil nach einem der Ansprüche 1(a) oder 2 bis 4:
wobei der monoklonale Antikörper oder Antigen-bindende Anteil an die GP1-Untereinheit des Marburgvirus-Glykoproteins bindet; und/oder
wobei der monoklonale Antikörper oder Antigen-bindende Anteil mit mindestens zwei Filoviren kreuzreaktiv ist; bevorzugt wobei der monoklonale Antikörper oder Antigen-bindende Anteil an ein Filovirus bindet, das ein Mitglied der *Ebolavirus-*Gattung ist; bevorzugt wobei der monoklonale Antikörper oder Antigen-bindende Anteil an das Ebolavirus-Glykoprotein bindet;
oder bevorzugt wobei der monoklonale Antikörper oder Antigen-bindende Anteil an mindestens eines vom Ebolavirus, Sudanvirus, Bundibugyovirus, Tai Forest-Virus oder Restonvirus bindet.

7. Monoklonaler Antikörper oder Antigen-bindender Anteil nach Anspruch 1(b) oder 1(c), wobei der monoklonale Antikörper oder Antigen-bindende Anteil an die GP2-Untereinheit des Marburgvirus-Glykoproteins bindet.

8. Pharmazeutische Zusammensetzung, umfassend den monoklonalen Antikörper oder Antigen-bindenden Anteil nach einem der Ansprüche 1(a), 2 bis 4 oder 6 und mindestens ein pharmazeutisch verträgliches Adjuvans oder mindestens einen pharmazeutisch verträglichen Träger.

9. Pharmazeutische Zusammensetzung nach Anspruch 8, weiter umfassend ein zweites Mittel;
bevorzugt wobei das zweite Mittel ein monoklonaler Antikörper oder Antigen-bindender Anteil davon ist; und/oder
wobei die pharmazeutische Zusammensetzung mindestens einen Marburgvirus-bindenden Antikörper oder Antigen-bindenden Anteil davon, und mindestens einen Ebolavirusbindenden Antikörper oder ein Antigen-bindendes Protein davon umfasst; und/oder
wobei die pharmazeutische Zusammensetzung mindestens zwei, mindestens drei oder mindestens vier Marburgvirus-bindende Antikörper oder Antigen-bindende Anteile davon umfasst; und/oder
wobei die pharmazeutische Zusammensetzung mindestens zwei Marburgvirus-bindende Antikörper oder Antigen-bindende Anteile davon umfasst, und wobei jeder Marburgvirus-bindende Antikörper oder Antigen-bindende Anteil verschiedene Epitope des Marburgvirus-Glykoproteins bindet.

10. Monoklonaler Antikörper oder Antigen-bindender Anteil nach einem der Ansprüche 1(a), 2 bis 4 oder 6 zur Verwendung bei der Besserung, Prävention oder Behandlung einer Filovirus-Infektion bei einem Patienten mit Bedarf daran;
bevorzugt wobei die Filovirus-Infektion eine Marburgvirus-Infektion oder eine Ebolavirus-Infektion ist.

11. Monoklonaler Antikörper oder Antigen-bindender Anteil nach einem der Ansprüche 1(b), 1(c), 5 oder 7 zur Verwendung bei der Besserung, Prävention oder Behandlung einer Marburgvirus-Infektion bei einem Patienten mit Bedarf daran.

12. Isoliertes Nukleinsäuremolekül, kodierend für den monoklonalen Antikörper oder Antigen-bindenden Anteil nach Anspruch 1.

13. Isoliertes Nukleinsäuremolekül nach Anspruch 12, wobei das Nukleinsäuremolekül eine oder mehr Nukleotidsequenz(en) umfasst, ausgewählt aus der Gruppe, bestehend aus SEQ ID NO: 73, SEQ ID NO: 75, SEQ ID NO: 76, SEQ ID NO: 77, SEQ ID NO: 85, SEQ ID NO: 87, SEQ ID NO: 88, SEQ ID NO: 89, SEQ ID NO: 1, SEQ ID NO: 13, SEQ ID NO: 202, SEQ ID NO: 25 und SEQ ID NO: 37.

14. Expressionsvektor, umfassend ein Nukleinsäuresegment, kodierend für die variablen Immunglobulin-Leichtkettenregionen des monoklonalen Antikörpers oder Antigen-bindenden Anteils nach einem der Ansprüche 1(a), 2 bis 4 oder 6, wobei das Nukleinsäuresegment mit den zur Expression des Nukleinsäuresegments in einer Wirtszelle geeigneten Regulationssequenzen operativ verknüpft ist.

15. Expressionsvektor nach Anspruch 14, wobei das Nukleinsäuresegment eine oder mehr Nukleotidsequenz(en) umfasst, ausgewählt aus der Gruppe, bestehend aus SEQ ID NO: 73, SEQ ID NO: 75, SEQ ID NO: 76, SEQ ID NO: 77, SEQ ID NO: 85, SEQ ID NO: 87, SEQ ID NO: 88 und SEQ ID NO: 89.

16. Rekombinante Wirtszelle, umfassend den Expressionsvektor nach Anspruch 14 oder 15;
bevorzugt wobei die Zelle eine Bakterien-, Eukaryoten- oder Säugerzelle ist; bevorzugter wobei die Zelle eine COS-1-, COS-7-, HEK293-, BHK21-, CHO-, BSC-1-, HepG2-, SP2/0-, Hela-, Myelom- oder Lymphomzelle ist.

17. Verfahren zur Herstellung eines Filovirus-bindenden monoklonalen Antikörpers oder Antigen-bindenden Anteils davon, wobei das Verfahren Folgendes umfasst: das Kultivieren einer rekombinanten Wirtszelle, umfassend den Expressionsvektor nach Anspruch 14 oder 15, unter Bedingungen, bei denen das Nukleinsäuresegment exprimiert wird, wodurch auf diese Weise der Filovirus-bindende monoklonale Antikörper oder Antigen-bindende Anteil hergestellt wird; bevorzugt weiter umfassend das Gewinnen des Filovirus-bindenden monoklonalen Antikörpers oder Antigen-bindenden Anteils.

18. Verfahren zum Nachweis eines Filovirus-Glykoproteins in einer Probe, wobei das Verfahren Folgendes umfasst: das Inkontaktbringen der Probe mit dem Antikörper oder Antigen-bindenden Anteil nach einem der Ansprüche 1(a), 2 bis 4 oder 6;
bevorzugt wobei die Probe eine Zelle, ein Gewebe oder eine biologische Flüssigkeit von einem Patienten mit Verdacht auf oder einem Risiko für eine Filovirus-Infektion ist; oder bevorzugt wobei das Filovirus-Glykoprotein ein Marburgvirus-, Ebolavirus-, Sudanvirus-, Bundibugyovirus-, Tai Forest-Virus- oder Restonvirus-Glykoprotein ist.

## Revendications

1. Anticorps monoclonal, ou portion de liaison à l'antigène de celui-ci, qui se lie spécifiquement à un filovirus, l'anticorps ou la portion de liaison à l'antigène comprenant :
(a) une région variable de chaîne légère d'immunoglobuline comprenant LCDR1, LCDR2, et LCDR3, et une région variable de chaîne lourde d'immunoglobuline comprenant HCDR1, HCDR2 et HCDR3, les régions LCDR1, LCDR2 et LCDR3 comprenant les séquences d'acides aminés énoncées dans les SÉQ. ID n^{os} 78, 79 et 80, respectivement, et les régions HCDR1, HCDR2 et HCDR3 comprenant les séquences d'acides aminés énoncées dans les SÉQ. ID n^{os} 90, 91 et 92, respectivement ; ou
(b) une région variable de chaîne légère d'immunoglobuline comprenant la séquence d'acides aminés énoncée dans la SÉQ. ID n° 2 et une région variable de chaîne lourde d'immunoglobuline comprenant la séquence d'acides aminés énoncée dans la SÉQ. ID n° 14 ; ou
(c) une région variable de chaîne légère d'immunoglobuline comprenant la séquence d'acides aminés énoncée dans la SÉQ. ID n° 26 et une région variable de chaîne lourde d'immunoglobuline comprenant la séquence d'acides aminés énoncée dans la SÉQ. ID n° 38.

2. Anticorps monoclonal ou portion de liaison à l'antigène selon la revendication 1 (a), dans lequel ou dans laquelle :
la région variable de chaîne légère d'immunoglobuline comprend la séquence d'acides aminés énoncée dans la SÉQ. ID n° 74 ; ou
la région variable de chaîne lourde d'immunoglobuline comprend la séquence d'acides aminés énoncée dans la SÉQ. ID n° 86.

3. Anticorps monoclonal ou portion de liaison à l'antigène selon la revendication 1 (a) ou 2, dans lequel ou dans laquelle :
la région variable de chaîne légère d'immunoglobuline comprend la séquence d'acides aminés énoncée dans la SÉQ. ID n° 74 et la région variable de chaîne lourde d'immunoglobuline comprend la séquence d'acides aminés énoncée dans la SÉQ. ID n° 86.

4. Anticorps monoclonal ou portion de liaison à l'antigène selon l'une quelconque des revendications 1 (a), 2 ou 3, l'anticorps monoclonal ou la portion de liaison à l'antigène étant sélectionné(e) dans le groupe constitué de : (i) une molécule d'immunoglobuline entière ; (b) un fragment scFv ; (c) un fragment Fab ; (d) un fragment F(ab')₂ ; et (e) un fragment Fv lié par une liaison disulfure ; et/ou
l'anticorps comprenant une région constante d'immunoglobuline sélectionnée dans le groupe constitué d'IgG1, IgG2, IgG3, IgG4, IgA1, IgA2, IgD, IgE et IgM.

5. Anticorps monoclonal ou portion de liaison à l'antigène selon l'une quelconque des revendications précédentes, le filovirus étant un membre du genre *Marburgvirus* ; le filovirus étant préférablement sélectionné dans le groupe constitué de souches Ravn, Angola, Musoke et Popp du virus Marburg ; et/ou
l'anticorps monoclonal ou la portion de liaison à l'antigène se liant à la glycoprotéine du virus Marburg.

6. Anticorps monoclonal ou portion de liaison à l'antigène selon l'une quelconque des revendications 1 (a) ou 2 à 4 :
l'anticorps monoclonal ou la portion de liaison à l'antigène se liant à la sous-unité GP1 de la glycoprotéine du virus Marburg ; et/ou
l'anticorps monoclonal ou la portion de liaison à l'antigène ayant une réactivité croisée avec au moins deux filovirus ; l'anticorps monoclonal ou la portion de liaison à l'antigène se liant préférablement à un filovirus qui est un membre du genre *Ebolavirus* ; l'anticorps monoclonal ou la portion de liaison à l'antigène se liant préférablement à la glycoprotéine du virus Ebola ;
ou l'anticorps monoclonal ou la portion de liaison à l'antigène se liant préférablement à au moins l'un du virus Ebola, du virus Soudan, du virus Bundibugyo, du virus Tai Forest ou du virus Reston.

7. Anticorps monoclonal ou portion de liaison à l'antigène selon la revendication 1(b) ou 1(c), l'anticorps monoclonal ou la portion de liaison à l'antigène se liant à la sous-unité GP2 de la glycoprotéine du virus Marburg.

8. Composition pharmaceutique comprenant l'anticorps monoclonal ou la portion de liaison à l'antigène selon l'une quelconque des revendications 1 (a), 2 à 4 ou 6, et au moins un adjuvant pharmaceutiquement acceptable ou au moins un support pharmaceutiquement acceptable.

9. Composition pharmaceutique selon la revendication 8, comprenant en outre un deuxième agent ;
préférablement dans laquelle le deuxième agent est un anticorps monoclonal ou une portion de liaison à l'antigène de celui-ci ; et/ou
la composition pharmaceutique comprenant au moins un anticorps se liant au virus Marburg ou une portion de liaison à l'antigène de celui-ci, et au moins un anticorps se liant au virus Ebola ou une protéine de liaison à l'antigène de celui-ci ; et/ou
la composition pharmaceutique comprenant au moins deux, au moins trois ou au moins quatre anticorps se liant au virus Marburg ou une portion de liaison à l'antigène de ceux-ci ; et/ou
la composition pharmaceutique comprenant au moins deux anticorps se liant au virus Marburg ou une portion de liaison à l'antigène de ceux-ci, et chaque anticorps se liant au virus Marburg ou portion de liaison à l'antigène se liant à différents épitopes de la glycoprotéine du virus Marburg.

10. Anticorps monoclonal ou portion de liaison à l'antigène selon l'une quelconque des revendications 1 (a), 2 à 4 ou 6, destiné(e) à une utilisation dans l'amélioration, la prévention ou le traitement d'une infection à filovirus chez un sujet qui en a besoin ;
l'infection à filovirus étant préférablement une infection par le virus Marburg ou une infection par le virus Ebola.

11. Anticorps monoclonal ou portion de liaison à l'antigène selon l'une quelconque des revendications 1 (b), 1 (c), 5 ou 7, destiné(e) à une utilisation dans l'amélioration, la prévention ou le traitement d'une infection par le virus Marburg chez un sujet qui en a besoin.

12. Molécule d'acide nucléique isolée codant l'anticorps monoclonal ou la portion de liaison à l'antigène selon la revendication 1.

13. Molécule d'acide nucléique isolée selon la revendication 12, la molécule d'acide nucléique comprenant une ou plusieurs séquences nucléotidiques sélectionnées dans le groupe constitué de la SÉQ. ID n° 73, de la SÉQ. ID n° 75, de la SÉQ. ID n° 76, de la SÉQ. ID n° 77, de la SÉQ. ID n° 85, de la SÉQ. ID n° 87, de la SÉQ. ID n° 88, de la SÉQ. ID n° 89, de la SÉQ. ID n° 1, de la SÉQ. ID n° 13, de la SÉQ. ID n° 202, de la SÉQ. ID n° 25, et de la SÉQ. ID n° 37.

14. Vecteur d'expression comprenant un segment d'acide nucléique codant les régions variables de chaîne légère et de chaîne lourde d'immunoglobuline de l'anticorps monoclonal ou de la portion de liaison à l'antigène selon l'une quelconque des revendications 1 (a), 2 à 4 ou 6, dans lequel le segment d'acide nucléique est fonctionnellement lié à des séquences régulatrices convenant pour l'expression du segment d'acide nucléique dans une cellule hôte.

15. Vecteur d'expression selon la revendication 14, dans lequel le segment d'acide nucléique comprend une ou plusieurs séquences nucléotidiques sélectionnées dans le groupe constitué de la SÉQ. ID n° 73, de la SÉQ. ID n° 75, de la SÉQ. ID n° 76, de la SÉQ. ID n° 77, de la SÉQ. ID n° 85, de la SÉQ. ID n° 87, de la SÉQ. ID n° 88, et de la SÉQ. ID n° 89.

16. Cellule hôte recombinante comprenant le vecteur d'expression selon la revendication 14 ou 15 ;
la cellule étant préférablement une cellule bactérienne, eucaryote ou mammifère ; la cellule étant plus préférablement une cellule COS-1, COS-7, HEK293, BHK21, CHO, BSC-1, HepG2, SP2/0, HeLa, de myélome ou de lymphome.

17. Procédé de production d'un anticorps monoclonal qui se lie à un filovirus ou d'une portion de liaison à l'antigène de celui-ci, le procédé comprenant
la mise en culture d'une cellule hôte recombinante comprenant le vecteur d'expression selon la revendication 14 ou 15 dans des conditions d'expression du segment d'acide nucléique, en produisant ainsi l'anticorps monoclonal qui se lie à un filovirus ou la portion de liaison à l'antigène ; comprenant préférablement en outre la récupération de l'anticorps monoclonal qui se lie à un filovirus ou de la portion de liaison à l'antigène.

18. Procédé de détection d'une glycoprotéine de filovirus dans un échantillon, le procédé comprenant la mise en contact de l'échantillon avec l'anticorps ou la portion de liaison à l'antigène selon l'une quelconque des revendications 1 (a), 2 à 4 ou 6 ;
préférablement dans lequel l'échantillon est une cellule, un tissu ou un liquide biologique provenant d'un sujet suspecté d'avoir, ou risquant, une infection à filovirus ; ou préférablement dans lequel la glycoprotéine de filovirus est une glycoprotéine du virus Marburg, du virus Ebola, du virus Soudan, du virus Bundibugyo, du virus Tai Forest ou du virus Reston.
